# EUROPEAN PATENT APPLICATION

(11) **EP 4 438 729 A2**
(43) Date of publication of application: **02.10.2024**
(21) Application number: 24183879.6
(22) Date of filing: 08.12.2020
(51) Int. Cl.: C12N 15/113

(54) **CRISPR ABASIC RESTRICTED NUCLEOTIDES AND CRISPR ACCURACY VIA ANALOGS**

(30) Priority: 09.12.2019 US 201962945788 P; 26.03.2020 US 202063000483 P
(62) Divisional of application: 20829483.5
(71) Applicant: Caribou Biosciences, Inc., Berkeley, CA 94710 (US)
(72) Inventor: DONOHOUE, Paul Daniel, Berkeley, California, 94710 (US); IRBY, Matthew John, Berkeley, California, 94710 (US)
(74) Representative: Grund, Martin

(57) **Abstract**

The present disclosure provides polynucleotide guides for use in CRISPR-Cas systems wherein such polynucleotides contain at least one CRISPR abasic restricted nucleotide (CABRNT) and/or at least one CRISPR accuracy via analogs (CAVA) nucleotide. CRISPR guides that contain at least one abasic site and/or at least one base analog, as well as nucleoprotein complexes of CRISPR-Cas proteins and CABRNT, CAVA, and CABRNT-CAVA guides are also described. Also disclosed are methods for making and using the CABRNT, CAVA, and CABRNT-CAVA polynucleotides and guides and the CABRNT/Cas, CAVA/Cas, and CABRNT-CAVA/Cas nucleoprotein complexes of the present invention.

## Description

### Cross-Reference to Related Applications

This application claims the benefit of U.S. Provisional Patent Application Serial No. 63/000,483, filed 26 March 2020, now pending, and U.S. Provisional Patent Application Serial No. 62/945,788, filed 09 December 2019, now pending, the contents of which are herein incorporated by reference in their entireties.

### Statement Regarding Federally Sponsored Research or Development

Not applicable.

### Sequence Listing

The present application contains a Sequence Listing that has been submitted electronically in ASCII format and is hereby incorporated by reference in its entirety. The ASCII copy, created on 07 December 2020 is named CBI037-30_ST25.txt and is 42 KB in size.

### Technical Field

The present disclosure relates generally to Clustered Regularly Interspaced Short Palindromic Repeats (CRISPR) systems. In particular, the disclosure relates to CRISPR polynucleotides and guides that are designed to include one or more abasic sites, base analogs, or combinations thereof; and to nucleoprotein complexes comprising such designed CRISPR guides comprising one or more abasic sites, base analogs, or combinations thereof and a CRISPR-Cas protein. The disclosure also relates to compositions and methods for making and using the polynucleotides and guides comprising one or more abasic sites, base analogs, or combinations thereof. Further still, the present disclosure relates to the production and therapeutic use of cells modified using the guide/nucleoprotein complexes of the present disclosure, and for instance, in the generation of chimeric antigen receptor (CAR)-expressing cells for the treatment of cancer.

### Background

The Clustered Regularly Interspaced Short Palindromic Repeats (CRISPR) and CRISPR-associated (Cas) protein systems are found in the genomes of many prokaryotes (bacteria and archaea). These systems provide adaptive immunity against foreign invaders (e.g., viruses, bacteriophages) in prokaryotes. In this way, the CRISPR system functions as a type of immune system to help defend prokaryotes against foreign invaders. *See, e.g.,* Barrangou et al. (Science, 2007, 315:1709-1712); Makarova et al. (Nature Reviews Microbiology, 2011, 9:467-477); Garneau et al. (Nature, 2010, 468:67-71); Sapranauskas et al. (Nucleic Acids Research, 2011, 39:9275-9282); Koonin et al. (Curr. Opin. Microbiol., 2017, 37:67-78); Shmakov et al. (Nat. Rev. Microbiol., 2017, 15(3): 169-182); and Makarova et al. (Nat. Rev. Microbiol., 2020, 18:67-83).

There are three main stages in CRISPR-Cas immune systems: (1) acquisition, (2) expression, and (3) interference. Acquisition involves cleaving the genome of invading viruses and plasmids and integrating segments (termed protospacers) of the genomic DNA into the CRISPR locus of the host organism. The segments that are integrated into the host genome are known as spacers, which mediate protection from subsequent attack by the same (or sufficiently related) virus or plasmid. Expression involves transcription of the CRISPR locus and subsequent enzymatic processing to produce short mature CRISPR RNAs, each containing a single spacer sequence. Interference is induced after the CRISPR RNAs associate with Cas proteins to form effector complexes, which are then targeted to complementary protospacers in foreign genetic elements to induce nucleic acid degradation.

Various CRISPR-Cas systems in their native hosts are capable of DNA targeting (Class 1 Type I; Class 2 Types II and V), RNA targeting (Class 2 Type VI), and joint DNA and RNA targeting (Class 1 Type III). *See, e.g.,* Makarova et al. (Nat. Rev. Microbiol., 2015, 13:722-736); Shmakov et al. (Nat. Rev. Microbiol., 2017, 15: 169-182); Abudayyeh et al. (Science, 2016, 353:1-17); and Makarova et al. (Nat. Rev. Microbiol., 2020, 18:67-83).

CRISPR-Cas systems provide powerful tools for site-directed genome editing by deleting, inserting, mutating, or substituting specific nucleic acid sequences. The alteration can be gene- or location-specific. Genome editing can use site-directed nucleases, such as Cas proteins and their cognate polynucleotides, to cut a target nucleic acid, thereby generating a site for alteration. In certain cases, the cleavage can introduce a double-strand break (DSB) in a target DNA sequence. DSBs can be repaired, e.g., by non-homologous end joining (NHEJ), microhomology-mediated end joining (MMEJ), or homology-directed repair (HDR). HDR relies on the presence of a template for repair. In some examples of genome editing, a donor polynucleotide or portion thereof can be inserted into the break.

For example, RNA-guided Cas endonucleases have been widely used for programmable genome editing in a variety of organisms and model systems. *See, e.g.,* Jinek et al. (Science, 2012, 337:816-821) and Jinek et al. (eLife, 2013, 2:e00471). Furthermore, Makarova et al. (Nat. Rev. Microbiol., 2020, 18:67-83) provides a summary of genes, homologs, effector protein domain organization, RNA components, effector complexes, and mechanisms of action for Class 1 and Class 2 CRISPR-Cas systems.

Although CRISPR-Cas systems have been used for genome editing, there remains a need to improve editing efficiency and editing fidelity of CRISPR-Cas systems.

### Summary of the Invention

The present invention is based on the discovery of new polynucleotides and guides for use in CRISPR-Cas systems, the polynucleotides and guides comprising one or more abasic sites, base analogs, or combinations thereof. In polynucleotides and guides of the present disclosure, an abasic site is referred to herein as a CRISPR abasic restricted nucleotide (CABRNT). In polynucleotides and guides of the present disclosure, a base analog is referred to herein as a CAVA (CRISPR accuracy via analogs). A polynucleotide or guide comprising an abasic site and one or more base analogs is referred to herein as a CABRNT-CAVA. The disclosed CABRNT, CAVA, and CABRNT-CAVA guides, when complexed with a CRISPR-Cas protein, are capable of robust on-target editing and reduced off-target editing, compared to previously known CRISPR guides.

This genome editing process is particularly useful for generating genetically modified cells useful in therapeutic applications. For instance, through this genome editing process, immune cells (such as T cells) can be genetically modified to express a CAR. Such CAR-expressing cells are useful, for instance, in adoptive immunotherapy - where CAR-expressing immune cells, such as T cells (CAR-T cells), can be infused into patients to target cells expressing a target antigen recognized by the CAR *(e.g.,* a foreign antigen, or a cancer-associated antigen).

Non-limiting embodiments of the disclosure include as follows below.
[1] A clustered regularly interspaced short palindromic repeats (CRISPR) polynucleotide comprising a spacer sequence comprising at least one nucleotide selected from the group consisting of a CRISPR abasic restricted nucleotide (CABRNT) and a CRISPR accuracy via analogs (CAVA) nucleotide.
[2] The CRISPR polynucleotide of [1], wherein the CRISPR polynucleotide comprises a CABRNT selected from an apurinic site or an apyrimidinic site.
[3] The CRISPR polynucleotide of [2], wherein the CABRNT comprises a ribose sugar.
[4] The CRISPR polynucleotide of [2], wherein the CABRNT comprises a deoxyribose sugar.
[5] The CRISPR polynucleotide of [1], wherein the CRISPR polynucleotide is capable of forming a nucleoprotein complex with a CRISPR-associated (Cas) protein.
[6] The CRISPR polynucleotide of [5], wherein, upon formation of the nucleoprotein complex, the nucleoprotein complex is capable of binding a target nucleic acid sequence complementary to the spacer sequence.
[7] The CRISPR polynucleotide of any one of [1]-[4], wherein the CRISPR polynucleotide is hybridized to a tracr polynucleotide.
[8] The CRISPR polynucleotide of [7], wherein the CRISPR polynucleotide and the tracr polynucleotide are located on separate molecules.
[9] The CRISPR polynucleotide of [7], wherein the CRISPR polynucleotide and the tracr polynucleotide are covalently linked to form a single molecule.
[10] The CRISPR polynucleotide of [7], wherein the CRISPR polynucleotide is capable of forming a nucleoprotein complex with a Cas protein.
[11] The CRISPR polynucleotide of [10], wherein, upon formation of the nucleoprotein complex, the nucleoprotein complex is capable of binding a target nucleic acid sequence complementary to the spacer sequence.
[12] A guide, comprising:
   a CRISPR polynucleotide comprising a spacer sequence comprising at least one nucleotide selected from the group consisting of a CABRNT and a CAVA;
   wherein the spacer sequence is capable of binding a target nucleic acid sequence; and wherein the guide is capable of forming a nucleoprotein complex with a Cas protein. sequence.
[13] The guide of [12], wherein, upon formation of the nucleoprotein complex, the nucleoprotein complex is capable of binding a target nucleic acid sequence complementary to the spacer sequence.
[14] The guide of [12], wherein the guide comprises ribonucleotide bases, deoxyribonucleotide bases, or ribonucleotide and deoxyribonucleotide bases.
[15] The guide of [12], wherein the guide comprises one or more nucleic acid analogs selected from the group consisting of inosine, deoxyinosine, deoxyuradine, xanthosine, C3 spacer, 5-methyl dC, 5-hydroxybutynl-2'-deoxyuridine, 5-nitroindole, 5-methyl iso-deoxycytosine, iso deoxyguanosine, deoxyuradine, and iso deoxycytidine.
[16] The guide of [14], wherein the guide comprises a CABRNT selected from an apurinic site or an apyrimidinic site.
[17] The guide of [16], wherein the CABRNT comprises a ribose sugar.
[18] The guide of [16], wherein the CABRNT comprises a deoxyribose sugar.
[19] The guide of [12], wherein the spacer sequence comprises more than one CABRNT.
[20] The guide of any one of [12]-[19], wherein the CRISPR polynucleotide is hybridized to a tracr polynucleotide.
[21] The guide of [20], wherein the CRISPR polynucleotide and the tracr polynucleotide are located on separate molecules.
[22] The guide of [20], wherein the CRISPR polynucleotide and the tracr polynucleotide are covalently linked to form a single molecule.
[23] A nucleic acid/protein composition, comprising:
   the guide of [12]; and
   a Cas protein.
[24] The nucleic acid/protein composition of [23], wherein the guide is in a complex with the Cas protein.
[25] The nucleic acid/protein composition of [23], wherein the guide comprises ribonucleotide bases, deoxyribonucleotide bases, or ribonucleotide and deoxyribonucleotide bases.
[26] The nucleic acid/protein composition of [23], wherein the guide comprises one or more nucleic acid analogs selected from the group consisting of inosine, deoxyinosine, deoxyuradine, xanthosine, C3 spacer, 5-methyl dC, 5-hydroxybutynl-2'-deoxyuridine, 5-nitroindole, 5-methyl iso-deoxycytosine, iso deoxyguanosine, deoxyuradine, and iso deoxycytidine.
[27] The nucleic acid/protein composition of [23], wherein the Cas protein comprises Cas9.
[28] The nucleic acid/protein composition of [23], wherein the Cas protein comprises Cas12.
[29] The nucleic acid/protein composition of [23], wherein the Cas protein comprises one or more Class 1 Type I Cascade subunit protein.
[30] The nucleic acid/protein composition of [29], wherein the one or more Class 1 Type I Cascade subunit protein is selected from the group consisting of Cas5, Cas6, Cas7, Cas8, and Cas11.
[31] A cell, comprising the guide of [12].
[32] The cell of [31], further comprising a Cas protein.
[33] The cell of [32], wherein the guide is in a complex with the Cas protein.
[34] The cell of [31], wherein the cell comprises a prokaryotic cell or a eukaryotic cell.
[35] The cell of [34], wherein the cell comprises a eukaryotic cell selected from the group consisting of a single-cell eukaryotic organism, a cell of a eukaryotic organism, a protozoal cell, a cell from a plant, an algal cell, a fungal cell, an animal cell, a cell from an invertebrate animal, a cell from a vertebrate animal, a cell from a mammal, a stem cell, and a progenitor cell.
[36] The cell of [32], further comprising a donor polynucleotide.
[37] A method of cleaving a target nucleic acid sequence, the method comprising:
   contacting a first target nucleic acid with a nucleoprotein complex comprising a catalytically active Cas protein and a guide of [12];
   wherein the spacer is capable of binding to the target nucleic acid, the guide is capable of hybridizing to the target nucleic acid sequence, and the nucleoprotein complex is capable of cleaving the target nucleic acid sequence.
[38] The method of [37], further comprising providing a donor polynucleotide.
[39] The method of [37], wherein the target nucleic acid is cleaved to provide a cleavage site, and the method further comprises modifying the target nucleic acid.
[40] The method of [39], wherein the modifying comprises inserting at least a portion of a donor polynucleotide at the cleavage site.
[41] The method of [39], wherein the modifying comprises deleting one or more nucleotides at the cleavage site.
[42] The method of [40], wherein the target nucleic acid sequence is in a eukaryotic cell, and the donor polynucleotide comprises a CAR expression vector.
[43] The method of [42], further comprising introducing the CAR expression vector into the cell using a viral vector.
[44] The method of [43], wherein said introducing comprises transduction.
[45] The cell of [35], wherein the cell comprises a lymphocyte, a chimeric antigen receptor (CAR) T cell, a T cell receptor (TCR) cell, a TCR-engineered CAR-T cell, a tumor infiltrating lymphocyte (TIL), a CAR TIL, a dendritic cell (DC), a CAR-DC, a macrophage, a CAR-macrophage (CAR-M), a natural killer (NK) cell, or a CAR-NK cell.
[46] The cell of [45], wherein the cell is a CAR-T cell.
[47] The method of [42], wherein the resulting cell comprises a lymphocyte, a chimeric antigen receptor (CAR) T cell, a T cell receptor (TCR) cell, a TCR-engineered CAR-T cell, a tumor infiltrating lymphocyte (TIL), a CAR TIL, a dendritic cell (DC), a CAR-DC, a macrophage, a CAR-macrophage (CAR-M), a natural killer (NK) cell, or a CAR-NK cell.
[48] The method of [42], wherein the first target nucleic acid sequence is within a target gene encoding a protein selected from the group consisting of a TRAC; a TRBV; a beta-2 microglobulin (B2M); a PD1; a PD-L1; a CTLA-4; a LAG-3; a TIGIT; a TIM3; a HLA-E; a HLA-A; a HLA-B; a HLA-C; a HLA-DRA; a ADAM17; a BTLA; a CD160; a SIGLEC10; a 2B4; a LAIR1; a CD52; a CD96; a VSIR; a VISTA; a KIR2DL1; a KIR2DL2; a KIR2DL3; a CEACAM1; a CBLB; a CISH; a IL-1R8; a AHR; a Adenosine 2A receptor; a GMCSF; a VISTA; a CII2A; and a NKG2A.
[49] The cell of [45], wherein the target nucleic acid sequence is within a target gene encoding a protein selected from the group consisting of a TRAC; a TRBV; a beta-2 microglobulin (B2M); a PD1; a PD-L1; a CTLA-4; a LAG-3; a TIGIT; a TIM3; a HLA-E; a HLA-A; a HLA-B; a HLA-C; a HLA-DRA; a ADAM17; a BTLA; a CD160; a SIGLEC10; a 2B4; a LAIR1; a CD52; a CD96; a VSIR; a VISTA; a KIR2DL1; a KIR2DL2; a KIR2DL3; a CEACAM1; a CBLB; a CISH; a IL-1R8; a AHR; a Adenosine 2A receptor; a GMCSF; a VISTA; a CII2A; and a NKG2A.
[50] The nucleic acid/protein composition of any one of [23]-[26], wherein the CRISPR polynucleotide is hybridized to a tracr polynucleotide.
[51] The cell of any one of [31]-[36], [45], [46], and [49], wherein the CRISPR polynucleotide is hybridized to a tracr polynucleotide.
[52] The method of any one of [37]-[44], [47], and [48], wherein the CRISPR polynucleotide is hybridized to a tracr polynucleotide.
[53] The CRISPR polynucleotide of [1], wherein the CRISPR polynucleotide comprises a CAVA.
[54] The CRISPR polynucleotide of [53], wherein the CAVA is selected from the group consisting of inosine, deoxyinosine, and deoxyuradine.
[55] The CRISPR polynucleotide of [53], wherein the CAVA comprises a ribose sugar.
[56] The CRISPR polynucleotide of [53], wherein the CAVA comprises a deoxyribose sugar.
[57] The guide of [14], wherein the guide comprises a CAVA.
[58] The guide of [57], wherein the CAVA is selected from the group consisting of inosine, deoxyinosine, and deoxyuradine.
[59] The guide of [57], wherein the CAVA comprises a ribose sugar.
[60] The guide of [57], wherein the CAVA comprises a deoxyribose sugar.
[61] The guide of [12], wherein the spacer sequence comprises more than one CAVA.
[62] A CRISPR polynucleotide comprising a spacer sequence comprising a CABRNT.
[63] The CRISPR polynucleotide of [62], wherein the CABRNT is selected from an apurinic site or an apyrimidinic site.
[64] The CRISPR polynucleotide of [63], wherein the CABRNT comprises a ribose sugar.
[65] The CRISPR polynucleotide of [63], wherein the CABRNT comprises a deoxyribose sugar.
[66] The CRISPR polynucleotide of [62], wherein the CRISPR polynucleotide is capable of forming a nucleoprotein complex with a Cas protein.
[67] The CRISPR polynucleotide of [66], wherein, upon formation of the nucleoprotein complex, the nucleoprotein complex is capable of binding a target nucleic acid sequence complementary to the spacer sequence.
[68] The CRISPR polynucleotide of any one of [62]-[65], wherein the CRISPR polynucleotide is hybridized to a tracr polynucleotide.
[69] The CRISPR polynucleotide of [68], wherein the CRISPR polynucleotide and tracr polynucleotide are located on separate molecules.
[70] The CRISPR polynucleotide of [68], wherein the CRISPR polynucleotide and tracr polynucleotide are covalently linked to form a single molecule.
[71] The CRISPR polynucleotide of [68], wherein the CRISPR polynucleotide is capable of forming a nucleoprotein complex with a Cas protein.
[72] The CRISPR polynucleotide of [71], wherein, upon formation of the nucleoprotein complex, the nucleoprotein complex is capable of binding a target nucleic acid sequence complementary to the spacer sequence.
[73] A CABRNT-containing guide, comprising:
   a CRISPR polynucleotide comprising a spacer sequence comprising at least one CABRNT;
   wherein the spacer sequence is capable of binding a target nucleic acid sequence, and the guide is capable of forming a nucleoprotein complex with a Cas protein.
[74] The CABRNT-containing guide of [73], wherein, upon formation of the nucleoprotein complex, the nucleoprotein complex is capable of binding a target nucleic acid sequence complementary to the spacer sequence.
[75] The CABRNT-containing guide of [73], wherein the guide comprises ribonucleotide bases, deoxyribonucleotide bases, or ribonucleotide and deoxyribonucleotide bases.
[76] The CABRNT-containing guide of [73], wherein the guide comprises one or more nucleic acid analogs selected from the group consisting of inosine, deoxyinosine, deoxyuradine, xanthosine, C3 spacer, 5-methyl dC, 5-hydroxybutynl-2'-deoxyuridine, 5-nitroindole, 5-methyl iso-deoxycytosine, iso deoxyguanosine, deoxyuradine, and iso deoxycytidine.
[77] The CABRNT-containing guide of [75], wherein the CABRNT is selected from an apurinic site or an apyrimidinic site.
[78] The CABRNT-containing guide of [77], wherein the CABRNT comprises a ribose sugar.
[79] The CABRNT-containing guide of [77], wherein the CABRNT comprises a deoxyribose sugar.
[80] The CABRNT-containing guide of [73], wherein the spacer sequence comprises more than one CABRNT.
[81] The CABRNT-containing guide of any one of [73]-[80], wherein the CRISPR polynucleotide is hybridized to a tracr polynucleotide.
[82] The CABRNT-containing guide of [81], wherein the CRISPR polynucleotide and tracr polynucleotide are located on separate molecules.
[83] The CABRNT-containing guide of [81], wherein the guide is a single molecule.
[84] A nucleic acid/protein composition, comprising:
   the CABRNT-containing guide of [73]; and
   a Cas protein.
[85] The nucleic acid/protein composition of [84], wherein the guide is in a complex with the Cas protein.
[86] The nucleic acid/protein composition of [84], wherein the guide comprises ribonucleotide bases, deoxyribonucleotide bases, or ribonucleotide and deoxyribonucleotide bases.
[87] The nucleic acid/protein composition of [84], wherein the guide comprises one or more nucleic acid analogs selected from the group consisting of inosine, deoxyinosine, deoxyuradine, xanthosine, C3 spacer, 5-methyl dC, 5-hydroxybutynl-2'-deoxyuridine, 5-nitroindole, 5-methyl iso-deoxycytosine, iso deoxyguanosine, deoxyuradine, and iso deoxycytidine.
[88] The nucleic acid/protein composition of [84], wherein the Cas protein comprises Cas9.
[89] The nucleic acid/protein composition of [84], wherein the Cas protein comprises Cas12.
[90] The nucleic acid/protein composition of [84], wherein the Cas protein comprises one or more Class 1 Type I Cascade subunit protein.
[91] The nucleic acid/protein composition of [90], wherein the one or more Class 1 Type I Cascade subunit protein is selected from the group consisting of Cas5, Cas6, Cas7, Cas8, and Cas11.
[92] A cell, comprising the CABRNT-containing guide of [73].
[93] The cell of [92], further comprising a Cas protein.
[94] The cell of [93], wherein the CABRNT-containing guide is in a complex with the Cas protein.
[95] The cell of [92], wherein the cell comprises a prokaryotic cell or a eukaryotic cell.
[96] The cell of [95], wherein the cell comprises a eukaryotic cell selected from the group consisting of a single-cell eukaryotic organism, a cell of a eukaryotic organism, a protozoal cell, a cell from a plant, an algal cell, a fungal cell, an animal cell, a cell from an invertebrate animal, a cell from a vertebrate animal, a cell from a mammal, a stem cell, and a progenitor cell.
[97] The cell of [93], further comprising a donor polynucleotide.
[98] A method of cleaving a target nucleic acid sequence, the method comprising:
   contacting a first target nucleic acid with a nucleoprotein complex comprising a catalytically active Cas protein and a CABRNT-containing guide of [73];
   wherein the spacer capable of binding to the target nucleic acid, the guide is capable of hybridizing to the target nucleic acid sequence, and the nucleoprotein complex is capable of cleaving the target nucleic acid sequence.
[99] The method of [98], further comprising providing a donor polynucleotide.
[100] The method of [98], wherein the target nucleic acid is cleaved to provide a cleavage site, and the method further comprises modifying the target nucleic acid.
[101] The method of [100], wherein the modifying comprises inserting at least a portion of a donor polynucleotide at the cleavage site.
[102] The method of [100], wherein the modifying comprises deleting one or more nucleotides at the cleavage site.
[103] The method of [101], wherein the target nucleic acid sequence is in a eukaryotic cell, and the donor polynucleotide comprises a CAR expression vector.
[104] The method of [103], further comprising introducing the CAR expression vector into the cell using a viral vector.
[105] The method of [104], wherein said introducing comprises transduction.
[106] The cell of [96], wherein the cell comprises a lymphocyte, a chimeric antigen receptor (CAR) T cell, a T cell receptor (TCR) cell, a TCR-engineered CAR-T cell, a tumor infiltrating lymphocyte (TIL), a CAR TIL, a dendritic cell (DC), a CAR-DC, a macrophage, a CAR-macrophage (CAR-M), a natural killer (NK) cell, or a CAR-NK cell.
[107] The cell of [106], wherein the cell is a CAR-T cell.
[108] The method of [103], wherein the resulting cell comprises a lymphocyte, a chimeric antigen receptor (CAR) T cell, a T cell receptor (TCR) cell, a TCR-engineered CAR-T cell, a tumor infiltrating lymphocyte (TIL), a CAR TIL, a dendritic cell (DC), a CAR-DC, a macrophage, a CAR-macrophage (CAR-M), a natural killer (NK) cell, or a CAR-NK cell.
[109] The method of [103], wherein the first target nucleic acid sequence is within a target gene encoding a protein selected from the group consisting of a TRAC; a TRBV; a beta-2 microglobulin (B2M); a PD1; a PD-L1; a CTLA-4; a LAG-3; a TIGIT; a TIM3; a HLA-E; a HLA-A; a HLA-B; a HLA-C; a HLA-DRA; a ADAM17; a BTLA; a CD160; a SIGLEC10; a 2B4; a LAIR1; a CD52; a CD96; a VSIR; a VISTA; a KIR2DL1; a KIR2DL2; a KIR2DL3; a CEACAM1; a CBLB; a CISH; a IL-1R8; a AHR; a Adenosine 2A receptor; a GMCSF; a VISTA; a CII2A; and a NKG2A.
[110] The cell of [106], wherein the target nucleic acid sequence is within a target gene encoding a protein selected from the group consisting of a TRAC; a TRBV; a beta-2 microglobulin (B2M); a PD1; a PD-L1; a CTLA-4; a LAG-3; a TIGIT; a TIM3; a HLA-E; a HLA-A; a HLA-B; a HLA-C; a HLA-DRA; a ADAM17; a BTLA; a CD160; a SIGLEC10; a 2B4; a LAIR1; a CD52; a CD96; a VSIR; a VISTA; a KIR2DL1; a KIR2DL2; a KIR2DL3; a CEACAM1; a CBLB; a CISH; a IL-1R8; a AHR; a Adenosine 2A receptor; a GMCSF; a VISTA; a CII2A; and a NKG2A.
[111] The nucleic acid/protein composition of any one of [84]-[87], wherein the CRISPR polynucleotide is hybridized to a tracr polynucleotide.
[112] The cell of any one of [92]-[97], [106], [107], and [110], wherein the CRISPR polynucleotide is hybridized to a tracr polynucleotide.
[113] The method of any one of [98]-[105], [108] and [109], wherein the CRISPR polynucleotide is hybridized to a tracr polynucleotide.
[114] A CRISPR polynucleotide comprising a spacer sequence comprising a CAVA nucleotide.
[115] The CRISPR polynucleotide of [114], wherein the CAVA is selected from the group consisting of inosine, deoxyinosine, and deoxyuradine.
[116] The CRISPR polynucleotide of [115], wherein the CAVA comprises a ribose sugar.
[117] The CRISPR polynucleotide of [115], wherein the CAVA comprises a deoxyribose sugar.
[118] The CRISPR polynucleotide of [114], wherein the CRISPR polynucleotide is capable of forming a nucleoprotein complex with a Cas protein.
[119] The CRISPR polynucleotide of [118], wherein, upon formation of the nucleoprotein complex, the nucleoprotein complex is capable of binding a target nucleic acid sequence complementary to the spacer sequence.
[120] The CRISPR polynucleotide of any one of [114]-[117], wherein the CRISPR polynucleotide is hybridized to a tracr polynucleotide.
[121] The CRISPR polynucleotide of [120], wherein the CRISPR polynucleotide and the tracr polynucleotide are located on separate molecules.
[122] The CRISPR polynucleotide of [120], wherein the CRISPR polynucleotide and the tracr polynucleotide are covalently linked to form a single molecule.
[123] The CRISPR polynucleotide of [120], wherein the CRISPR polynucleotide is capable of forming a nucleoprotein complex with a Cas protein.
[124] The CRISPR polynucleotide of [123], wherein, upon formation of the nucleoprotein complex, the nucleoprotein complex is capable of binding a target nucleic acid sequence complementary to the spacer sequence.
[125] A CAVA-containing guide, comprising:
   a CRISPR polynucleotide comprising a spacer sequence comprising at least one CAVA;
   wherein the spacer sequence is capable of binding a target nucleic acid sequence; and wherein the guide is capable of forming a nucleoprotein complex with a Cas protein.
[126] The CAVA-containing guide of [125], wherein, upon formation of the nucleoprotein complex, the nucleoprotein complex is capable of binding a target nucleic acid sequence complementary to the spacer sequence.
[127] The CAVA-containing guide of [125], wherein the guide comprises ribonucleotide bases, deoxyribonucleotide bases, or ribonucleotide and deoxyribonucleotide bases.
[128] The CAVA-containing guide of [125], wherein the guide comprises one or more nucleic acid analogs selected from the group consisting of inosine, deoxyinosine, deoxyuradine, xanthosine, C3 spacer, 5-methyl dC, 5-hydroxybutynl-2'-deoxyuridine, 5-nitroindole, 5-methyl iso-deoxycytosine, iso deoxyguanosine, deoxyuradine, and iso deoxycytidine.
[129] The CAVA-containing guide of [127], wherein the CAVA is selected from the group consisting of inosine, deoxyinosine, and deoxyuradine.
[130] The CAVA-containing guide of [129], wherein the CAVA comprises a ribose sugar.
[131] The CAVA-containing guide of [129], wherein the CAVA comprises a deoxyribose sugar.
[132] The CAVA-containing guide of [125], wherein the spacer sequence comprises more than one CAVA.
[133] The CAVA-containing guide of any one of [125]-[132], wherein the CRISPR polynucleotide is hybridized to a tracr polynucleotide.
[134] The CAVA-containing guide of [133], wherein the CRISPR polynucleotide and the tracr polynucleotide are located on separate molecules.
[135] The CAVA-containing guide of [133], wherein the CRISPR polynucleotide and the tracr polynucleotide are covalently linked to form a single molecule.
[136] A nucleic acid/protein composition, comprising:
   the CAVA-containing guide of [125]; and
   a Cas protein.
[137] The nucleic acid/protein composition of [136], wherein the guide is in a complex with the Cas protein.
[138] The nucleic acid/protein composition of [136], wherein the guide comprises ribonucleotide bases, deoxyribonucleotide bases, or ribonucleotide and deoxyribonucleotide bases.
[139] The nucleic acid/protein composition of [136], wherein the guide comprises one or more nucleic acid analogs selected from the group consisting of inosine, deoxyinosine, deoxyuradine, xanthosine, C3 spacer, 5-methyl dC, 5-hydroxybutynl-2'-deoxyuridine, 5-nitroindole, 5-methyl iso-deoxycytosine, iso deoxyguanosine, deoxyuradine, and iso deoxycytidine.
[140] The nucleic acid/protein composition of [136], wherein the Cas protein comprises Cas9.
[141] The nucleic acid/protein composition of [136], wherein the Cas protein comprises Cas12.
[142] The nucleic acid/protein composition of [136], wherein the Cas protein comprises one or more Class 1 Type I Cascade subunit protein.
[143] The nucleic acid/protein composition of [142], wherein the one or more Class 1 Type I Cascade subunit protein is selected from the group consisting of Cas5, Cas6, Cas7, Cas8, and Cas11.
[144] A cell, comprising the CAVA-containing guide of [125].
[145] The cell of [144], further comprising a Cas protein.
[146] The cell of [145], wherein the CAVA-containing guide is in a complex with the Cas protein.
[147] The cell of [144], wherein the cell comprises a prokaryotic cell or a eukaryotic cell.
[148] The cell of [147], wherein the cell comprises a eukaryotic cell selected from the group consisting of a single-cell eukaryotic organism, a cell of a eukaryotic organism, a protozoal cell, a cell from a plant, an algal cell, a fungal cell, an animal cell, a cell from an invertebrate animal, a cell from a vertebrate animal, a cell from a mammal, a stem cell, and a progenitor cell.
[149] The cell of [145], further comprising a donor polynucleotide.
[150] A method of cleaving a target nucleic acid sequence, the method comprising:
   contacting a first target nucleic acid with a nucleoprotein complex comprising a catalytically active Cas protein and a CAVA-containing guide of [125];
   wherein the spacer is capable of binding to the target nucleic acid, the guide is capable of hybridizing to the target nucleic acid sequence, and the nucleoprotein complex is capable of cleaving the target nucleic acid sequence.
[151] The method of [150], further comprising providing a donor polynucleotide.
[152] The method of [150], wherein the target nucleic acid is cleaved to provide a cleavage site, and the method further comprises modifying the target nucleic acid.
[153] The method of [152], wherein the modifying comprises inserting at least a portion of a donor polynucleotide at the cleavage site.
[154] The method of [152], wherein the modifying comprises deleting one or more nucleotides at the cleavage site.
[155] The method of [153], wherein the target nucleic acid sequence is in a eukaryotic cell, and the donor polynucleotide comprises a CAR expression vector.
[156] The method of [155], further comprising introducing the CAR expression vector into the cell using a viral vector.
[157] The method of [156], wherein said introducing comprises transduction.
[158] The cell of [148], wherein the cell comprises a lymphocyte, a chimeric antigen receptor (CAR) T cell, a T cell receptor (TCR) cell, a TCR-engineered CAR-T cell, a tumor infiltrating lymphocyte (TIL), a CAR TIL, a dendritic cell (DC), a CAR-DC, a macrophage, a CAR-macrophage (CAR-M), a natural killer (NK) cell, or a CAR-NK cell.
[159] The cell of [158], wherein the cell is a CAR-T cell.
[160] The method of [155], wherein the resulting cell comprises a lymphocyte, a chimeric antigen receptor (CAR) T cell, a T cell receptor (TCR) cell, a TCR-engineered CAR-T cell, a tumor infiltrating lymphocyte (TIL), a CAR TIL, a dendritic cell (DC), a CAR-DC, a macrophage, a CAR-macrophage (CAR-M), a natural killer (NK) cell, or a CAR-NK cell.
[161] The method of [155], wherein the first target nucleic acid sequence is within a target gene encoding a protein selected from the group consisting of a TRAC; a TRBV; a beta-2 microglobulin (B2M); a PD1; a PD-L1; a CTLA-4; a LAG-3; a TIGIT; a TIM3; a HLA-E; a HLA-A; a HLA-B; a HLA-C; a HLA-DRA; a ADAM17; a BTLA; a CD160; a SIGLEC10; a 2B4; a LAIR1; a CD52; a CD96; a VSIR; a VISTA; a KIR2DL1; a KIR2DL2; a KIR2DL3; a CEACAM1; a CBLB; a CISH; a IL-1R8; a AHR; a Adenosine 2A receptor; a GMCSF; a VISTA; a CII2A; and a NKG2A.
[162] The cell of [158], wherein the target nucleic acid sequence is within a target gene encoding a protein selected from the group consisting of a TRAC; a TRBV; a beta-2 microglobulin (B2M); a PD1; a PD-L1; a CTLA-4; a LAG-3; a TIGIT; a TIM3; a HLA-E; a HLA-A; a HLA-B; a HLA-C; a HLA-DRA; a ADAM17; a BTLA; a CD160; a SIGLEC10; a 2B4; a LAIR1; a CD52; a CD96; a VSIR; a VISTA; a KIR2DL1; a KIR2DL2; a KIR2DL3; a CEACAM1; a CBLB; a CISH; a IL-1R8; a AHR; a Adenosine 2A receptor; a GMCSF; a VISTA; a CII2A; and a NKG2A.
[163] The nucleic acid/protein composition of any one of [136]-[139], wherein the CRISPR polynucleotide is hybridized to a tracr polynucleotide.
[164] The cell of any one of [144]-[149], [158], [159], and [162], wherein the CRISPR polynucleotide is hybridized to a tracr polynucleotide.
[165] The method of any one of [150]-[157], [160] and [161], wherein the CRISPR polynucleotide is hybridized to a tracr polynucleotide.
[166] The method of any one of [42]-[44], [103]-[105], and [155]-[157], wherein the CAR expression vector encodes a CAR comprising an extracellular ligand-binding domain.
[167] The method of [166], wherein the CAR expression vector further encodes a hinge region, a transmembrane region, and one or more intracellular signaling regions.
[168] The method of [166] or [167], wherein the extracellular ligand-binding domain comprises an immunoglobulin single-chain variable fragment (scFv).
[169] The method of [168], wherein the scFv is capable of binding to a cellular target selected from the group consisting of a CD37, a CD38, a CD47, a CD73, a CD4, a CS1, a PD-L1, a NGFR, a ENPP3, a PSCA, a CD79B, a TACI, a VEGFR2, a B7-H3, a B7-H6, a B-cell maturation antigen (BCMA), a CD123, a CD138, a CD171/L1CAM, a CD19, a CD20, a CD22, a CD30, a CD33, a CD70, a CD371, a CEA, a Claudin 18.1, a Claudin 18.2, a CSPG4, a EFGRvIII, a EpCAM, a EphA2, a Epidermal growth factor receptor, a ErbB, a ErbB2 (HER2), a FAP, a FRα, a GD2, a GD3, a Glypican 3, a IL-11Rα, a IL-13Rα2, a IL13 receptor alpha, a LewisY/LeY, a Mesothelin, a MUC1, a MUC16, a NKG2D ligands, a PD1, a PSMA, a ROR-1, a SLAMF7, a TAG72, a ULBP and a MICA/B proteins, a VEGF2, and a WT1.
[170] The method of [169], wherein the scFv is capable of binding to a cellular target selected from the group consisting of a BCMA, a CD19, a CD20, a CD22, a CD47, a CD371, a ROR-1, a EphA2, a MUC16, a Glypican 3, a PSCA, and a Claudin 18.2.
[171] The method of [170], wherein the scFv is capable of binding to a BCMA.
[172] The method of [170], wherein the scFv is capable of binding to a CD371.
[173] The method of any one of [37], [98] and [150], wherein said method further comprises contacting a second target nucleic acid sequence in the cell with a nucleoprotein complex comprising a catalytically active Cas protein and a second guide, wherein the second guide comprises a guide of any one of [12], [73] and [125] that is capable of binding to a different target nucleic acid sequence than the first guide, wherein the targeting region of the second guide is capable of hybridizing to the second target nucleic acid sequence, and the nucleoprotein complex is capable of cleaving the second target nucleic acid sequence.
[174] The method of [173], wherein said first and second target nucleic acid sequences are each independently within a target gene encoding a protein selected from the group consisting of a TRAC; a TRBV protein; a beta-2 microglobulin (B2M); a PD1; a PD-L1; a CTLA-4; a LAG-3; a TIGIT; a TIM3; a HLA-E; a HLA-A; a HLA-B; a HLA-C; a HLA-DRA; an ADAM17; a BTLA; a CD160; a SIGLEC10; a 2B4; a LAIR1; a CD52; a CD96; a VSIR; a VISTA; a KIR2DL1; a KIR2DL2; a KIR2DL3; a CEACAM1; a CBLB; a CISH; an IL-1R8; an AHR; an Adenosine 2A receptor; a GMCSF; a VISTA; a CII2A; and a NKG2A.
[175] The method of [173] or [174], wherein the donor polynucleotide comprises a CAR expression vector, wherein the CAR comprises an extracellular ligand-binding domain, and wherein the extracellular ligand-binding domain comprises an scFv.
[176] The method of [175], wherein the scFv is capable of binding to a BCMA.
[177] The method of [175], wherein the scFv is capable of binding to a CD371.
[178] The method of [174], wherein said first target nucleic acid sequence is within a gene encoding a TRAC protein, and wherein said second target nucleic acid sequence is within a gene encoding a PD1 protein.
[179] The method of [174], wherein said first target nucleic acid sequence is within a gene encoding a TRAC protein, and wherein said second target nucleic acid sequence is within a gene encoding a B2M protein.
[180] The method of [179], further comprising providing a second donor polynucleotide comprising a B2M-HLA-E fusion construct to the cell, and wherein at least a portion of the second donor polynucleotide comprising the B2M-HLA-E fusion construct is inserted at the cleavage site of the second target nucleic acid sequence, wherein the B2M-HLA-E fusion construct encodes a fusion protein comprising, from the N- to C- terminus, a B2M secretion signal, a HLA-G peptide signal sequence, a first linker sequence, a B2M sequence, a second linker sequence, and a HLA-E sequence.
[181] The method of [171] or [176], wherein the anti-BCMA scFv comprises a heavy chain variable region (VH) comprising the amino acid sequence of SEQ ID NO: 93; and a light chain variable region (VL) comprising the amino acid sequence of SEQ ID NO: 94.
[182] The method of [181], wherein the scFv further comprises a linker between the VH and the VL.
[183] The method of [182], wherein the linker comprises the amino acid sequence of SEQ ID NO: 95.
[184] The method of [183], wherein said scFv comprises the amino acid sequence of SEQ ID NO: 96.
[185] The method of [166] or [175], wherein the CAR comprises: an scFv comprising a VH and a VL; a transmembrane domain; a co-stimulatory domain; and an activating domain.
[186] The method of [185], wherein the transmembrane domain is a transmembrane domain derived from a T cell receptor α chain, a T cell receptor β chain, a CD3ζ chain, a CD28, a CD3ε, a CD45, a CD4, a CD5, a CD8, a CD9, a CD16, a CD22, a CD33, a CD37, a CD64, a CD80, a CD86, a CD134, a CD137, an ICOS, a CD154, or a GITR.
[187] The method of [186], wherein the transmembrane domain comprises a transmembrane domain derived from a CD8.
[188] The method of [185], wherein the co-stimulatory domain is a co-stimulatory domain derived from a CD28, a 4-1BB, a GITR, an ICOS-1, a CD27, an OX-40, or a DAP10.
[189] The method of [188], wherein the co-stimulatory domain comprises a 4-1BB co-stimulatory domain.
[190] The method of [185], wherein the activating domain comprises a CD3ζ activating domain.
[191] The method of [185], wherein the transmembrane domain comprises a transmembrane domain derived from a CD8, the co-stimulatory domain comprises a 4-1BB co-stimulatory domain, and the activating domain comprises a CD3ζ activating domain.
[192] The method of [185], wherein the VH comprises the amino acid sequence of SEQ ID NO: 93, and the VL comprises the amino acid sequence of SEQ ID NO: 94.
[193] The method of any one of [42]-[44], [103]-[105], and [155]-[157], wherein the polynucleotide sequence encoding the CAR in said CAR expression vector has a leader sequence at the 5' end.
[194] The method of [193], wherein the leader sequence comprises the nucleic acid sequence of SEQ ID NO: 97.
[195] The method of [193], wherein the CAR expression vector comprises a promoter.
[196] The method of [195], wherein the promoter comprises an MND promoter.
[197] A cell produced by the method of any one of [42]-[44], [103]-[105], [155]-[157], [166]-[172], and [175]-[196].
[198] A CAR-T cell produced by the method of any one of [42]-[44], [103]-[105], [155]-[157], [166]-[172], and [175]-[196].
[199] The CAR-T cell of [198], wherein said CAR-T cell is an allogeneic CAR-T cell.
[200] The CAR-T cell of [198], wherein said CAR-T cell is an autologous CAR-T cell.
[201] A method of producing a CAR-T cell, comprising performing the method of any one of [42]-[44], [103]-[105], [155]-[157], [166]-[172], and [175]-[196] using a T-lymphocyte as the cell.
[202] A method of adoptive cell therapy, comprising administering to a subject in need thereof a cell produced by the method of any one of [42]-[44], [103]-[105], [155]-[157], [166]-[172], and [175]-[196].
[203] A method of adoptive cell therapy, comprising administering to a subject in need thereof a CAR-T cell produced by the method of any one of [42]-[44], [103]-[105], [155]-[157], [166]-[172], and [175]-[196].
[204] A method of killing BCMA-positive cancer cells, wherein said method comprises contacting BCMA-positive cancer cells with a CAR-T cell produced by the method of any one of [171], [176] and [181],
[205] The method of [204], wherein the BCMA-positive cancer cells comprise multiple myeloma cancer cells.
[206] The method of [205], wherein the multiple myeloma cancer cells comprise human cells.
[207] The method of [204], wherein the contacting is intra-tumoral.
[208] A method for producing a CAR-expressing cell, said method comprising: contacting a first target nucleic acid sequence in a cell with a nucleoprotein complex comprising a catalytically active Cas protein and a first guide, wherein the first guide comprises a guide of any one of [12], [73] and [125], wherein the targeting region of the first guide is capable of hybridizing to the first target nucleic acid sequence, and the nucleoprotein complex is capable of cleaving the first target nucleic acid sequence; contacting a second target nucleic acid sequence in the cell with a nucleoprotein complex comprising a catalytically active Cas protein and a second guide, wherein the second guide comprises a guide of any one of [12], [73] and [125] that is capable of binding to a different target nucleic acid sequence than the first guide, wherein the targeting region of the second guide is capable of hybridizing to the second target nucleic acid sequence, and the nucleoprotein complex is capable of cleaving the second target nucleic acid sequence; and providing a donor polynucleotide comprising a CAR expression vector to said cell, wherein at least a portion of the donor polynucleotide containing said CAR expression vector is capable of being inserted at the cleavage site in said first target nucleic acid sequence, and wherein the CAR comprises an extracellular ligand-binding domain.
[209] The method of [208], wherein the donor polynucleotide comprising the CAR expression vector is introduced into the cell using a viral vector.
[210] The method of [208], wherein the CAR expression vector further encodes a hinge region, a transmembrane region, and one or more intracellular signaling regions.
[211] The method of any one of [208]-[210], wherein said first target nucleic acid sequence is within a gene encoding a TRAC protein, and wherein said second target nucleic acid sequence is within a gene encoding a PD1 protein.
[212] The method of any one of [208]-[210], wherein said first target nucleic acid sequence is within a gene encoding a TRAC protein, and wherein said second target nucleic acid sequence is within a gene encoding a B2M protein.
[213] The method of any of [208]-[212], wherein the extracellular ligand-binding domain comprises an immunoglobulin single-chain variable fragment (scFv).
[214] The method of [213], wherein the scFv is capable of binding a BCMA.
[215] The method of [213], wherein the scFv is capable of binding a CD371.
[216] The method of [214], wherein the anti-BCMA scFv comprises a heavy chain variable region (VH) comprising the amino acid sequence of SEQ ID NO: 93, and a light chain variable region (VL) comprising the amino acid sequence of SEQ ID NO: 94.
[217] The method of [216], wherein the scFv further comprises a linker between the VH and the VL.
[218] The method of [217], wherein the linker comprises the amino acid sequence of SEQ ID NO: 95.
[219] The method of [214], wherein said scFv comprises the amino acid sequence of SEQ ID NO: 96.
[220] The method of any one of [208]-[219], further comprising providing a second donor polynucleotide comprising a B2M-HLA-E fusion construct to said cell, wherein at least a portion of the second donor polynucleotide comprising the B2M-HLA-E fusion construct is capable of being inserted at the cleavage site of the second target nucleic acid sequence, and wherein the B2M-HLA-E fusion construct encodes a fusion protein comprising, from the N- to C- terminus, a B2M secretion signal, a HLA-G peptide signal sequence, a first linker sequence, a B2M sequence, a second linker sequence, and a HLA-E sequence.
[221] The method of any one of [208]-[220], wherein the CAR comprises: an scFv comprising a VH and a VL; a transmembrane domain; a co-stimulatory domain; and an activating domain.
[222] The method of [221], wherein the transmembrane domain comprises a transmembrane domain derived from a T cell receptor α chain, a T cell receptor β chain, a CD3ζ chain, a CD28, a CD3ε, a CD45, a CD4, a CD5, a CD8, a CD9, a CD16, a CD22, a CD33, a CD37, a CD64, a CD80, a CD86, a CD134, a CD137, an ICOS, a CD154, or aGITR.
[223] The method of [222], wherein the transmembrane domain comprises a transmembrane domain derived from a CD8.
[224] The method of [221], wherein the co-stimulatory domain comprises a co-stimulatory domain derived from a CD28, a 4-1BB, a GITR, an ICOS-1, a CD27, a OX-40, or a DAP10.
[225] The method of [224] wherein the co-stimulatory domain comprises a 4-1BB co-stimulatory domain.
[226] The method of [221], wherein the activating domain comprises a CD3ζ activating domain.
[227] The method of [221], wherein the transmembrane domain comprises a transmembrane domain derived from a CD8, the co-stimulatory domain comprises a 4-1BB co-stimulatory domain, and the activating domain comprises a CD3ζ activating domain.
[228] The method of any one of [208]-[227], wherein the CAR-expressing cell is a CAR-T cell.
[229] The method of [228], wherein the CAR-T cell is an allogeneic CAR-T cell.
[230] The method of [228], wherein the CAR-T cell is an autologous CAR-T cell.
[231] The nucleic acid/protein composition of [27], wherein the CRISPR polynucleotide is hybridized to a tracr polynucleotide.
[232] The nucleic acid/protein composition of [231], wherein the CRISPR polynucleotide and the tracr polynucleotide are located on separate molecules.
[233] The nucleic acid/protein composition of [231], wherein the CRISPR polynucleotide and the tracr polynucleotide are covalently linked to form a single molecule.
[234] The nucleic acid/protein composition of [88], wherein the CRISPR polynucleotide is hybridized to a tracr polynucleotide.
[235] The nucleic acid/protein composition of [234], wherein the CRISPR polynucleotide and the tracr polynucleotide are located on separate molecules.
[236] The nucleic acid/protein composition of [234], wherein the CRISPR polynucleotide and the tracr polynucleotide are covalently linked to form a single molecule.
[237] The nucleic acid/protein composition of [140], wherein the CRISPR polynucleotide is hybridized to a tracr polynucleotide.
[238] The nucleic acid/protein composition of [237], wherein the CRISPR polynucleotide and the tracr polynucleotide are located on separate molecules.
[239] The nucleic acid/protein composition of [237], wherein the CRISPR polynucleotide and the tracr polynucleotide are covalently linked to form a single molecule.
[240] The nucleic acid/protein composition of [28], wherein the CRISPR polynucleotide comprises a stem-loop duplex.
[241] The nucleic acid/protein composition of [89], wherein the CRISPR polynucleotide comprises a stem-loop duplex.
[242] The nucleic acid/protein composition of [141], wherein the CRISPR polynucleotide comprises a stem-loop duplex.

### Incorporation by Reference

All patents, publications, and patent applications cited in the present specification are herein incorporated by reference as if each individual patent, publication, or patent application was specifically and individually indicated to be incorporated by reference in its entirety for all purposes.

### Brief Description of the Figures

The features of the invention are set forth with particularity in the appended claims. A better understanding of the features and advantages of the present invention will be obtained by reference to the following detailed description that sets forth illustrative embodiments, in which the principles of the invention are utilized, and the accompanying figures. The figures are not proportionally rendered, nor are they to scale. The locations of indicators are approximate.
**FIG. 1A** and **FIG. 1B** illustrate examples of dual-guide Type II CRISPR-Cas9 guide RNAs.
**FIG. 2** illustrates an example of a single-guide Type II CRISPR-Cas9 RNA.
**FIG. 3A, FIG. 3B,** and **FIG. 3C** illustrate RNA nucleotide, abasic ribose, and abasic deoxyribose molecules, respectively.
**FIG. 4A** illustrates hydrogen base pair formation between a CRISPR spacer and target polynucleotide.
**FIG. 4B** illustrates hydrogen base pair formation between a CABRNT spacer and target polynucleotide.
**FIG. 5** illustrates a Cas9 nucleoprotein complex, wherein the double-stranded nucleic acid comprising the target sequence is unwound, and the target binding sequence of the Cas9/guide complex is connected via hydrogen bonds to the target sequence.
**FIG. 6** illustrates an embodiment of a nucleoprotein complex that comprises a Cas9 protein and a CABRNT spacer. The double-stranded nucleic acid comprising the target sequence is unwound, and the target binding sequence of the Cas9/CABRNT guide complex is connected via hydrogen bonds to the target sequence.
**FIG. 7** illustrates hydrogen base pair formation between an inosine base and the four canonical nucleotide bases.
**FIG. 8** illustrates an embodiment of a nucleoprotein complex that comprises a Cas9 protein and a CAVA guide. The double-stranded nucleic acid comprising the target sequence is unwound, and the target binding sequence of the Cas9/CAVA guide complex is connected via hydrogen bonds to the target sequence.
**FIG. 9A, FIG. 9B, and FIG. 9C** illustrate examples of CAVA nucleotides.
**FIG. 10A, FIG. 10B, and FIG. 10C** illustrate examples of Type V CRISPR-Cas12a guide RNAs.
**FIG. 11** illustrates an embodiment of a nucleoprotein complex that comprises a Cas12a protein and a CAVA spacer. The double-stranded nucleic acid comprising the target sequence is unwound, and the target binding sequence of the Cas12a/CAVA guide complex is connected via hydrogen bonds to the target sequence.

### Detailed Description of the Invention

It is to be understood that the terminology used herein is for the purpose of describing particular embodiments only and is not intended to be limiting. As used in the present specification and the appended claims, the singular forms "a," "an," and "the" include plural referents unless the context clearly dictates otherwise. Thus, for example, reference to "a polynucleotide" includes one or more polynucleotides, and reference to "a vector" includes one or more vectors.

Unless defined otherwise, all technical and scientific terms used herein have the same meaning as commonly understood by one of ordinary skill in the art to which the invention pertains. Although other methods and materials similar, or equivalent, to those described herein can be useful in the present invention, preferred materials and methods are described herein.

In view of the teachings of the present specification, one of ordinary skill in the art can apply conventional techniques of immunology, biochemistry, chemistry, molecular biology, microbiology, cell biology, genomics, and recombinant polynucleotides, as taught, for example, by the following standard texts: Abbas et al. (Cellular and Molecular Immunology, 2017, 9th Edition, Elsevier, ISBN 978-0323479783); Butterfield et al. (Cancer Immunotherapy Principles and Practice, 2017, 1st Edition, Demos Medical, ISBN 978-1620700976); Kenneth Murphy (Janeway's Immunobiology, 2016, 9th Edition, Garland Science, ISBN 978-0815345053); Stevens et al. (Clinical Immunology and Serology: A Laboratory Perspective, 2016, 4th Edition, Davis Company, ISBN 978-0803644663); E.A. Greenfield (Antibodies: A Laboratory Manual, 2014, Second edition, Cold Spring Harbor Laboratory Press, ISBN 978-1-936113-81-1); R.I. Freshney (Culture of Animal Cells: A Manual of Basic Technique and Specialized Applications, 2016, 7th Edition, Wiley-Blackwell, ISBN 978-1118873656); C.A. Pinkert (Transgenic Animal Technology, Third Edition: A Laboratory Handbook, 2014, Elsevier, ISBN 978-0124104907); H. Hedrich (The Laboratory Mouse, 2012, Second Edition, Academic Press, ISBN 978-0123820082); Behringer et al. (Manipulating the Mouse Embryo: A Laboratory Manual, 2013, Fourth Edition, Cold Spring Harbor Laboratory Press, ISBN 978-1936113019); McPherson et al. (PCR 2: A Practical Approach, 1995, IRL Press, ISBN 978-0199634248); J.M. Walker (Methods in Molecular Biology (Series), Humana Press, ISSN 1064-3745); Rio et al. (RNA: A Laboratory Manual, 2010, Cold Spring Harbor Laboratory Press, ISBN 978-0879698911); Methods in Enzymology (Series), Academic Press; Green et al. (Molecular Cloning: A Laboratory Manual, 2012, Fourth Edition, Cold Spring Harbor Laboratory Press, ISBN 978-1605500560); and G.T. Hermanson (Bioconjugate Techniques, 2013, Third Edition, Academic Press, ISBN 978-0123822390).

Clustered regularly interspaced short palindromic repeats (CRISPR) and related CRISPR-associated proteins (Cas proteins) constitute CRISPR-Cas systems. As used herein, a "CRISPR-Cas system" refers to any of the various CRISPR-Cas classes, types, and subtypes. The classification of CRISPR-Cas systems has had many iterations. Makarova et al. (Nat. Rev. Microbiol., 2020, 18:67-83) proposed a classification system that takes into consideration the signature *cas* genes specific for individual types and subtypes of CRISPR-Cas systems. The classification also considered sequence similarity between multiple shared Cas proteins, the phylogeny of the best conserved Cas protein, gene organization, and the structure of the CRISPR array. This approach provided a classification scheme that divides CRISPR-Cas systems into two distinct classes: Class 1 and Class 2.

Class 1 systems comprise a multiprotein effector complex and are divided into three types: Type I (CRISPR-associated complex for antiviral defense, Cascade, effector complex), Type III (Cmr/Csm effector complex), and Type IV systems. Type I is the most common and diverse, Type III is more common in archaea than bacteria, and Type IV is the least common. Type I systems comprise the signature Cas3 protein. The Cas3 protein has helicase and DNase domains responsible for DNA target sequence cleavage. To date, seven subtypes of the Type I system have been identified *(i.e.,* Type I-A, I-B, I-C, I-D, I-E, I-F) and variants for I-F *(e.g.,* I-Fv1, I-Fv2), and I-U that have a variable number of cas genes. Examples of organisms having Type I systems are as follows: I-A, *Archaeoglobus fulgidus*; I-B, *Clostridium kluyveri;* I-C, *Bacillus halodurans*; I-U, *Geobacter sulfurreducens*; I-D, *Cyanothece* spp. 8802; I-E, *Escherichia coli* K12; I-F, *Yersinia pseudo-tuberculosis;* I-F variant, *Shewanella putrefaciens* CN-32. *See, e.g.,* Koonin etal. (Curr. Opin. Microbiol., 2017, 37:67-78). Type I systems typically encode proteins that combine with a CRISPR RNA (crRNA) to form a Cascade complex. Subunit proteins of the Cascade complex comprise Cas5, Cas6, Cas7, Cas8, and Cas11 (formerly known as Cse2).

To date, it appears that all Type III systems possess a *cas10* gene, which encodes a multidomain protein containing a Palm domain (a variant of the RNA recognition motif (RRM)) that is homologous to the core domain of numerous nucleic acid polymerases and cyclases and that is the largest subunit of Type III crRNA--effector complexes. All Type III loci also encode the small subunit protein, one Cas5 protein and typically several Cas7 proteins. Type III is also further divided into several subtypes.

Type IV systems encode a minimal multisubunit crRNA-effector complex comprising a partially degraded large subunit, Csf1, Cas5, Cas7, and in some cases, a putative small subunit. Type IV systems do not have subtypes, but there are two distinct variants. One Type IV variant has a DinG family helicase, whereas a second Type IV variant lacks a DinG family helicase, but has a gene encoding a small a-helical protein. An example of an organism with a Type IV system is *Acidithiobacillus ferrooxidans.*

Class 2 systems comprise a single effector protein (Type II (Cas9)), Type V (Cas12a, previously referred to as Cpf1), and Type VI (Cas13, previously referred to as C2c2)). This single effector protein complexes with a guide to form an effector complex.

In Type II systems, nucleic acid target sequence binding and cleavage involve a Cas9 protein, a crRNA, and a trans-activating CRISPR RNA (tracrRNA). In Type II systems, the RuvC-like nuclease (RNase H fold) domain and the HNH (McrA-like) nuclease domain of the Cas9 protein each cleave one of the strands of the double-stranded nucleic acid target sequence. The Cas9 protein cleavage activity of Type II systems also requires hybridization of the crRNA to the tracrRNA to form a duplex that facilitates the crRNA and nucleic acid target sequence binding by the Cas9 protein.

Cas9 is an endonuclease that can be programmed by crRNA/tracrRNA to cleave, in a site-specific manner, a target DNA sequence using two distinct endonuclease domains (HNH and RuvC/RNase H-like domains). *See, e.g.,* Jinek et al. (Science, 2012, 337:816-821). Many Cas9 protein orthologs are known in the art as well as their associated polynucleotide components (crRNA and tracrRNA). *See, e.g.,* Fonfara et al. (Nucleic Acids Research, 2014, 42(4):2577-2590, including all Supplemental Data); and Chylinski et al. (Nucleic Acids Research, 2014, 42(10):6091-6105, including all Supplemental Data). In addition, Cas9-like synthetic proteins are known in the art.

As used herein, "Cas9 protein" refers to Cas9 wild-type proteins derived from Type II CRISPR-Cas9 systems, modifications of Cas9 proteins, variants of Cas9 proteins, Cas9 orthologs, and combinations thereof. Cas9 proteins include, but are not limited to, Cas9 from *Streptococcus pyogenes* (UniProtKB - Q99ZW2 (CAS9_STRP1)), *Streptococcus thermophilus* (UniProtKB - G3ECR1 (CAS9_STRTR)), *Staphylococcus aureus* (UniProtKB - J7RUA5 (CAS9_STAAU), *Campylobacter jejuni* (UniProtKB - Q0P897 (CAS9_CAMJE)), *Campylobacter lari* (UniProtKB - A0A0A8HTA3 (A0A0A8HTA3_CAMLA), and *Helicobacter canadensis* (UniProtKB - C5ZYI3 (C5ZYI3_9HELI)). Cas9 homologs can be identified using sequence similarity search methods known to one skilled in the art. Typically, for use in embodiments of the present invention, a Cas9 protein is capable of interacting with one or more cognate polynucleotides to form a nucleoprotein complex capable of binding to a target nucleic acid sequence.

"nCas9" as used herein refers to variants of a Cas9 protein that are nuclease deficient proteins, also termed "nicking Cas9" or "Cas9-nickase." Such molecules lack a portion of the endonuclease activity and therefore can only nick one strand of the target nucleic acid. *See, e.g.,* Jinek etal. (Science, 2012, 337:816-821). This is accomplished by introducing mutations in only one of the nuclease domains, such as D10A in the RuvC domain or H840A in the HNH domain (numbered relative to *S. pyogenes* Cas9), that inactivate the double-strand cleavage activity of a Cas9. It is understood that mutations of other catalytic residues to reduce activity of either of the nuclease domains can also be carried out by one skilled in the art. The resultant nCas9 is unable to cleave double-stranded DNA but retains the ability to complex with a guide nucleic acid and bind a target DNA sequence and nick only one strand of the target DNA. Targeting specificity is determined by Cas9 protein binding to the PAM sequence and by complementary base pairing of guide RNA (typically, a single-guide RNA) to the genomic locus.

"dCas9" as used herein refers to variants of a Cas9 protein that are nuclease-deactivated Cas9 proteins, also termed "catalytically inactive Cas9 protein," "enzymatically inactive Cas9," "catalytically dead Cas9," or "dead Cas9." Such molecules lack all of the endonuclease activity and can therefore be used to regulate genes in a RNA-guided manner. *See, e.g.,* Jinek etal. (Science, 2012, 337:816-821). This is accomplished by introducing mutations into both catalytic domains, such as D10A in the RuvC domain and H840A in the HNH domain (numbered relative to *S. pyogenes* Cas9), that inactivate Cas9 nuclease function. It is understood that mutations of other catalytic residues to reduce activity of both of the nuclease domains can also be carried out by one skilled in the art. The resultant dCas9 is unable to cleave double-stranded DNA but retains the ability to complex with a guide nucleic acid and bind a target DNA sequence. The Cas9 double mutant with changes at amino acid positions D10A and H840A (numbered relative to *S. pyogenes* Cas9) inactivates both the nuclease and nickase activities of the Cas9 protein. Targeting specificity is determined by Cas9 protein binding to the PAM sequence and by complementary base pairing of guide RNA (typically, a single-guide RNA) to the genomic locus.

Typically, each wild-type CRISPR-Cas9 system includes a crRNA and a tracrRNA. The crRNA has a region of complementarity to a potential target DNA sequence and a second region that forms base-pair hydrogen bonds with the tracrRNA to form a secondary structure, typically to form at least one stem structure. The region of complementarity to the target DNA sequence is the spacer. The tracrRNA and the crRNA interact through a number of base-pair hydrogen bonds to form secondary RNA structures. Complex formation between crRNA/tracrRNA and a Cas9 protein results in conformational change of the Cas9 protein that facilitates binding to DNA, endonuclease activities of the Cas9 protein, and crRNA-guided site-specific DNA cleavage by the Cas9 endonuclease. For a Cas9 protein/crRNA/tracrRNA complex to cleave a double-stranded target DNA sequence, the target DNA sequence is adjacent to a cognate PAM. By engineering a crRNA to have an appropriate spacer sequence, the complex can be targeted to cleave at a locus of interest, e.g., a locus at which sequence modification is desired.

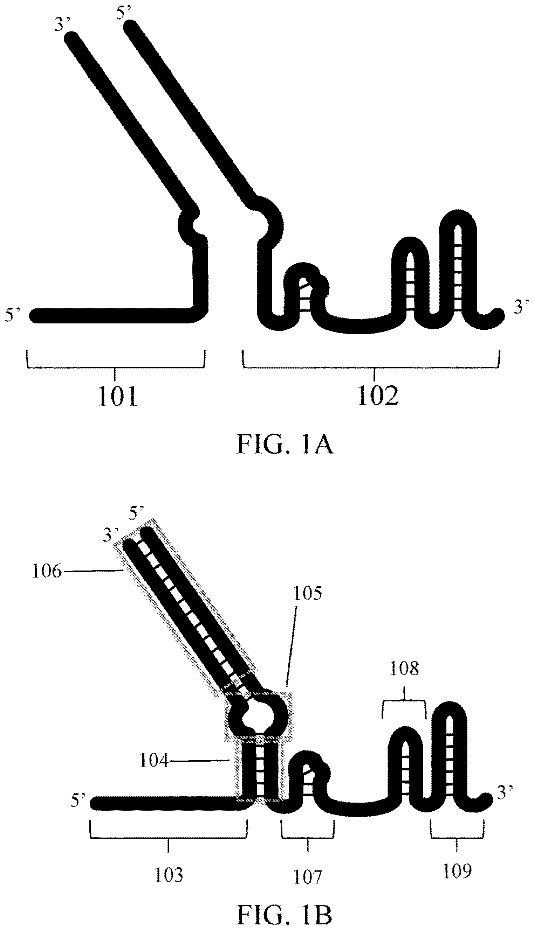

A variety of Type II CRISPR-Cas system crRNA and tracrRNA sequences, as well as predicted secondary structures, are known in the art. *See, e.g.,* Ran et al. (Nature, 2015, 520(7546): 186-191, including all Supplemental Data, in particular Extended Data Figure 1); and Fonfara et al. (Nucleic Acids Research, 2014, 42(4):2577-2590, including all Supplemental Data, in particular Supplemental Figure S11). Predicted tracrRNA secondary structures were based on the Constraint Generation RNA folding model. *See* Zuker et al. (Nucleic Acids Research, 2003, 31:3406-3415). RNA duplex secondary structures were predicted using RNAcofold of the Vienna RNA package. *See, e.g.,* Bernhart et al. (Algorithms for Molecular Biology, 2006, 1(1):3 ); Hofacker et al. (Journal of Molecular Biology, 2002, 319:1059-1066) and RNAhybrid (bibiserv.techfak.uni-bielefeld.de/rnahybrid/). The structure predictions were visualized using VARNA. *See* Darty et al. (Bioinformatics, 2009, 25:1974-1975). Fonfara *et al.* show that the crRNA/tracrRNA complex for *Campylobacter jejuni* does not have the bulge region; however, the complex retains a stem structure located 3' of the spacer that is followed in the 3' direction with another stem structure.

The spacer of Class 2 CRISPR-Cas systems can hybridize to a nucleic acid target sequence that is located 5' or 3' of a PAM, depending upon the Cas protein to be used. A PAM can vary depending upon the Cas protein to be used. For example, if a Cas9 protein from *S*. *pyogenes* is used, the PAM can be a sequence in the nucleic acid target sequence that comprises the sequence 5'-NRR-3', wherein R can be either A or G, N is any nucleotide, and N is immediately 3' of the nucleic acid target sequence targeted by the nucleic acid target binding sequence. A Cas protein may be modified such that a PAM may be different compared with a PAM for an unmodified Cas protein. For example, if a Cas9 protein from *S. pyogenes* is used, the Cas9 protein may be modified such that the PAM no longer comprises the sequence 5'-NRR-3', but instead comprises the sequence 5'-NNR-3', wherein R can be either A or G, N is any nucleotide, and N is immediately 3' of the nucleic acid target sequence targeted by the nucleic acid target sequence.

In Class 2, Type V, systems, the crRNA and target binding involves Cas12, as does the target nucleic acid cleavage. The RuvC-like nuclease domain of Cas12a, for instance, cleaves both strands of the target nucleic acid in a staggered configuration, producing 5' overhangs, which is in contrast to the blunt ends generated by Cas9 cleavage. These 5' overhangs may facilitate insertion of DNA through homologous recombination methods.

Other proteins associated with Type V crRNA and target binding and cleavage include Cas12b (formerly C2c1) and Cas12c (formerly C2c3). Cas12b and Cas12c proteins are similar in length to CRISPR Class 2 Type II Cas9 and CRISPR Class 2 Type V Cas12a proteins, ranging from approximately 1,100 amino acids to approximately 1,500 amino acids. C2c1 and C2c3 proteins also contain RuvC-like nuclease domains and have an architecture similar to Cas12a. C2c1 proteins are similar to Cas9 proteins in requiring a crRNA and a tracrRNA for target binding and cleavage, but have an optimal cleavage temperature of 50 °C. C2c1 proteins target an AT-rich PAM, which similar to Cas12a, is 5' of the target sequence. *See, e.g.,* Shmakov et al. (Molecular Cell, 2015, 60(3):385-397).

The CRISPR Type V subtypes include the Cast2 proteins, and demonstrate a broad sequence and diversity in size; however, Cas12 subtypes share a common evolutionary origin from TnpB nucleases encoded by IS605-like transposons. Owing to the low sequence similarity, and likely evolution through multiple independent recombination events of Cas12 proteins, classification of Cas12 proteins into their respective subtypes has resulted in multiple naming conventions. Table 1 presents the classification and names for the Type V Cas12 proteins as well as their approximate size, guide requirements, preferred target polynucleotide, and a representative organism of origin.

| **Table 1** | | | | | | |
|---|---|---|---|---|---|---|
| **Classification of Type V Subtypes** | | | | | | |
| **Type V** | **Cas nomenclature** | **Other names** | **Effector size** | **Guide** | **Target polynucleotide** | **Representative organism** |
| V-A | Cas12a | MAD7 | >1000aa | crRNA | dsDNA | *Francisella cf. novicida* |
| V-B1 | Cas12b1 | c2c1 | >1000aa | crRNA, tracrRNA | dsDNA | *Alicyclobacillus acidoterrestris* |
| V-B2 | Cas12b2 | - | | | | *Planctomycetes bacterium* RBG_13_46_10 |
| V-C | Cas12c | c2c3 | >1000aa | crRNA, tracrRNA | dsDNA | *Oleiphilus spp.* |
| V-D | Cas12d | CasY | >1000aa | crRNA | dsDNA | *Bacterium* CG09_39_24 |
| V-E | Cas12e | CasX | ~1000aa | crRNA, tracrRNA | dsDNA | *Deltaproteobacteria bacterium* |
| V-F1 | Cas12f1 | Cas 14a, c2c 10, V-U3 | 400-800aa | crRNA, tracrRNA | dsDNA | *Uncultured archaeon* |
| V-F2 | Cas12f2 | Cas14b | | crRNA | | *Bacillus thuringiensis* HD-771 |
| V-F3 | Cas12f3 | Cas 14c | | crRNA | | *Candidatus Micrarchaeota archaeon* |
| V-G | Cas12g | - | 700-800aa | crRNA, tracrRNA | ssRNA | Hot springs metagenome |
| V-H | Cas12h | - | ∼1000aa | crRNA | ssDNA, dsDNA | Hypersaline lake sediment metagenome |
| V-I | Cas12i | - | ∼1000aa | crRNA | ssDNA, dsDNA | Freshwater metagenome |
| V-J | Cas12j | Casϕ (Cas-phi) | 700-800aa | crRNA | dsDNA | Biggiephage |
| V-K | Cas12K | c2c5 | >700aa | crRNA, tracrRNA | no nuclease activity | *Cyanothece spp.* PCC 8801 |
| V-U | - | c2c4, c2c8, c2c9 | n.d. | n.d. | n.d. | *Gordonia otitidis* |

Cas12 homologs can be identified using sequence similarity search methods known to those skilled in the art. Typically, a Cas12 protein is capable of interacting with a cognate Cas12 guide to form a Cas12 guide/nucleoprotein complex capable of binding to a target nucleic acid sequence. In some embodiments of the present disclosure, the Cas12 protein or homolog thereof is a Cas12a protein or homolog thereof.

Cas12a proteins include, but are not limited to, Cas12a from *Parcubacteria bacterium* GWC2011_GWC2_44_17 (PbCpf1), *Lachnospiraceae bacterium* MC2017 (Lb3 Cpf1), *Butyrivibrio proteoclasticus* (BpCpf1), *Peregrinibacteria bacterium* GW2011_GWA_33_10 (PeCpf1), *Acidaminococcus spp.* BV3L6 (AsCpf1), *Porphyromonas macacae* (PmCpf1), *Lachnospiraceae bacterium* ND2006 (LbCpf1), *Porphyromonas crevioricanis* (PcCpf1), *Prevotella disiens* (PdCpf1), *Moraxella bovoculi* 237 (MbCpf1), *Smithella sp.* SC_K08D17 (SsCpf1), *Leptospira inadai* (LiCpf1), *Lachnospiraceae bacterium* MA2020 (Lb2Cpf1), *Franciscella novicida* U112 (FnCpf1), *Candidatus methanoplasma termitum* (CMtCpf1), and *Eubacterium eligens* (EeCpf1).

In Type V systems, nucleic acid target sequence binding typically involves a Cas12 protein and a crRNA, as does the nucleic acid target sequence cleavage. In Type V systems, the RuvC-like nuclease domain of Cast 2 protein cleaves both strands of the nucleic acid target sequence in a sequential fashion, *see* Swarts et al. (Mol. Cell, 2017, 66:221-233), producing 5' overhangs, which contrasts with the blunt ends generated by Cas9 protein cleavage.

The Cas12 protein cleavage activity of Type V systems can be independent of a tracrRNA (e.g., Type V-A); and some Type V systems require only a single crRNA that has a stem-loop structure forming an internal duplex. Cast2 protein binds the crRNA in a sequence- and structure-specific manner by recognizing the stem loop and sequences adjacent to the stem loop, most notably the nucleotides 5' of the spacer sequence, which hybridize to the nucleic acid target sequence. This stem-loop structure is typically in the range of 15 to 22 nucleotides in length. Substitutions that disrupt this stem-loop duplex abolish cleavage activity, whereas other substitutions that do not disrupt the stem-loop duplex do not abolish cleavage activity. Certain Type V systems require the hybridization between a crRNA and tracrRNA, such as the Type V-F1, V-G, V-C, V-E (CasX), V-K, and V-B. *See, e.g.,* Yan et. al. (Science, 2019, 363(6422):88-91).

Type VI systems include the Cas13 protein (also known as Class 2 candidate 2 protein, or C2c2), which does not share sequence similarity with other CRISPR effector proteins. *See, e.g.,* Abudayyeh et al. (Science, 2016, 353:aaf5573). Cas13 proteins have two HEPN domains and possess single-stranded RNA (ssRNA) cleavage activity. Cast3 proteins are similar to Cas12 proteins in requiring a crRNA for nucleic acid target sequence binding and cleavage, but not requiring tracrRNA. Also, similar to Cas12 protein, the crRNA for Cas13 proteins forms a stable hairpin, or stem-loop structure, that aids in association with the Cas13a protein. Type VI systems have a single polypeptide RNA endonuclease that utilizes a single crRNA to direct RNA cleavage in a target-dependent fashion. Additionally, after hybridizing to the target RNA complementary to the spacer, Cas13 protein becomes a promiscuous RNA endonuclease exhibiting non-specific endonuclease activity toward any single-stranded RNA in a sequence independent manner. *See, e.g.,* O'Connell etal. (J. Mol. Biol., 2019, 431:66-87).

In the present invention, Class 1 Type I, Class 2 Type II, and Class 2 Type V CRISPR-Cas systems are preferred.

As used herein, "Cas protein" refers to CRISPR-associated proteins (Cas) including, but not limited to Class 1 Type I CRISPR-associated proteins, Class 1 Type III CRISPR-associated proteins, Class 1 Type IV CRISPR-associated proteins, Class 2 Type II CRISPR-associated proteins, Class 2 Type V CRISPR-associated proteins, and Class 2 Type VI CRISPR-associated proteins. Typically, for use in embodiments of the present invention, a Cas protein is capable of interacting with one or more cognate guides to form a nucleoprotein complex. In some embodiments, the Cas protein is catalytically active, and in some embodiments the Cas protein is catalytically inactive.

"Guide" and "guide polynucleotide" as used herein refers to one or more polynucleotides that form a nucleoprotein complex with a Cas protein, wherein the nucleoprotein complex preferentially binds a nucleic acid target sequence in a polynucleotide (relative to a polynucleotide that does not comprise the nucleic acid target sequence). Such guides can comprise ribonucleotide bases *(e.g.,* RNA), deoxyribonucleotide bases *(e.g.,* DNA), combinations of ribonucleotide bases and deoxyribonucleotide bases (e.g., RNA/DNA), nucleotide analogs, modified nucleotides, and the like, as well as synthetic, naturally occurring, and non-naturally occurring modified backbone residues or linkages. Many such guides are known, such as but not limited to single-guide RNA (including miniature and truncated single-guide RNAs), crRNA, dual-guide RNA, including but not limited to, crRNA/tracrRNA molecules, and the like, the use of which depends on the particular Cas protein. For example, "Type II CRISPR-Cas9-associated guide" is a guide that specifically associates with a cognate Cas9 protein to form a nucleoprotein complex.

With reference to a guide, a "spacer," "spacer sequence," or "spacer element" as used herein refers to a polynucleotide sequence that can specifically hybridize to a target nucleic acid sequence. The spacer element interacts with the target nucleic acid sequence through hydrogen bonding between complementary base pairs *(i.e.,* paired bases). A spacer element binds to a selected nucleic acid target sequence. Accordingly, the spacer element is the nucleic acid target-binding sequence. The spacer element determines the location of a Cas protein's site-specific binding and nucleolytic cleavage. Variability of the functional length for a spacer element is known in the art.

As used herein, the terms "abasic," "abasic site," "abasic nucleotide," "apurinic/apyrimidinic site," and "AP site" are used interchangeably and refer to a site in a nucleotide sequence that lacks the purine or a pyrimidine base. In certain embodiments, abasic sites comprise a deoxyribose site. In other embodiments, abasic sites comprise a ribose site. In yet further embodiments, abasic sites comprise a modified backbone, such as a pentose ring with a 1' hydroxyl group. An abasic site cannot form hydrogen base pair bonding with a complementary nitrogen base of a DNA or RNA nucleotide, because it does not contain a nitrogen base.

As used herein, the term "CRISPR accuracy via analogs" or "CAVA" refers to one or more base analogs present in a CRISPR polynucleotide or guide. As used herein, the term "base analog" refers to a compound having structural similarity to a canonical purine or pyrimidine base occurring in DNA or RNA. The base analog may contain a modified sugar and/or a modified nucleobase, as compared to a purine or pyrimidine base occurring naturally in DNA or RNA. In some embodiments, the base analog is inosine or deoxyinosine, such as 2'-deoxyinosine. In other embodiments, the base analog is a 2'-deoxyribonucleoside, 2'-ribonucleoside, 2'-deoxyribonucleotide or a 2'-ribonucleotide, wherein the nucleobase includes a modified base (such as, for example, xanthine, uridine, oxanine (oxanosine), 7-methlguanosine, dihydrouridine, 5-methylcytidine, C3 spacer, 5-methyl dC, 5-hydroxybutynl-2'-deoxyuridine, 5-nitroindole, 5-methyl iso-deoxycytosine, iso deoxyguanosine, deoxyuradine, iso deoxycytidine, other 0-1 purine analogs, N-6-hydroxylaminopurine, nebularine, 7-deaza hypoxanthine, other 7-deazapurines, and 2-methyl purines). In some embodiments, the base analog may be selected from the group consisting of 7-deaza-2'-deoxyinosine, 2'-aza-2'-deoxyinosine, PNA-inosine, morpholino-inosine, LNA-inosine, phosphoramidate-inosine, 2'-O-methoxyethyl-inosine, and 2'-OMe-inosine. The term "base analog" also includes, for example, 2'-deoxyribonucleosides, 2'-ribonucleosides, 2'-deoxyribonucleotides or 2'-ribonucleotides, wherein the nucleobase is a substituted hypoxanthine. For instance, the substituted hypoxanthine may be substituted with a halogen, such as fluorine or chlorine. In some embodiments, the base analog may be a fluoroinosine or a chloroinosine, such as 2-chloroinosine, 6-chloroinosine, 8-chloroinosine, 2-fluoroinosine, 6-fluoroinosine, or 8-fluoroinosine. In other embodiments, the base analog is deoxyuradine. In other embodiments the base analog is a nucleic acid mimic (such as, for example, artificial nucleic acids and xeno nucleic acids (XNA).

As used herein, a polynucleotide for use in a CRISPR-Cas system includes a CRISPR (cr) polynucleotide containing a spacer sequence (e.g., crRNAs and chRDNAs), optionally a trans-activating CRISPR polynucleotide (tracr polynucleotide) (e.g., tracrRNAs and ch-acrs), or both a CRISPR polynucleotide containing a spacer sequence and a tracr polynucleotide (e.g., crRNA:tracrRNA, chRDNA:ch-acr, single-guide RNA, or single-guide chRDNA).

As used herein, a "CRISPR abasic restricted nucleotide" or "CABRNT" is an abasic site within a sequence of nucleotides for use in a CRISPR-Cas system.

As used herein, a "CRISPR polynucleotide" is a polynucleotide sequence comprising a portion of a guide molecule *(e.g.,* a portion of a single guide or a dual guide). In some embodiments, the CRISPR polynucleotide includes a spacer sequence (e.g., crRNAs and chRDNAs). In some embodiments, the CRISPR polynucleotide further comprises a nucleotide sequence that is configured to hybridize to, or is hybridized to, a tracr polynucleotide.

As used herein, a "CABRNT polynucleotide" refers to a CRISPR polynucleotide that includes one or more abasic sites designed into the polynucleotide. The one or more abasic sites can be designed at any position in the polynucleotide and is preferably designed in the spacer sequence. In other embodiments, the one or more abasic sites can be designed in the tracr polynucleotide.

As used herein, a "CABRNT guide" refers to a guide comprising a spacer sequence and further comprising RNA with one or more abasic sites designed into the polynucleotide. The abasic site can be designed at any position in the guide and is preferably designed in the spacer sequence. The CABRNT guide can include a tracr polynucleotide, either hybridized to the CRISPR polynucleotide component so as to remain as separate molecules in a dual-guide system (e.g., Type II systems), or covalently linked to the CRISPR polynucleotide component to form a single polynucleotide in a single-guide system. In CABRNT guides that include a tracr polynucleotide, the one or more abasic sites can be designed in the tracr polynucleotide. In an alternative embodiment, the CABRNT guide does not include a tracr polynucleotide (*e.g*., a Type V system or a Type VI system). In any of these embodiments, the CABRNT guide is capable of forming a nucleoprotein complex with a Cas protein and binding a target nucleic acid sequence complementary to the spacer sequence.

As used herein, the term "CRISPR hybrid RNA/DNA guide" (chRDNA) refers to a guide comprising a spacer, wherein the polynucleotide comprises RNA with DNA designed into the polynucleotide. In some embodiments, the chRDNA corresponds to a crRNA component of a Cas9 dual guide, wherein DNA has been designed into the polynucleotide sequence. chRDNA/DNAs comprise combinations of ribonucleotide bases and deoxyribonucleotide bases (e.g., RNA/DNA). In other embodiments, the chRDNA corresponds to a crRNA component of a Cas12 guide. As used herein, the term "chimeric-activating CRISPR" (ch-acr) refers to a polynucleotide comprising a nexus and at least one 3' hairpin, wherein the polynucleotide comprises RNA with DNA designed into the polynucleotide. In some embodiments, the ch-acr corresponds to a tracr polynucleotide component of a Cas9 dual-guide RNA, wherein DNA has been designed into the polynucleotide sequence. *See, e.g.,* U.S. Patent Nos. 9,650,617; 9,580,701; 9,688,972; 9,771,601; and 9,868,962.

As used herein, a "CAVA polynucleotide" refers to a CRISPR polynucleotide that includes one or more base analogs designed into the polynucleotide. The one or more base analogs can be designed at any position in the polynucleotide and is preferably designed in the spacer sequence. In other embodiments, the one or more base analogs can be designed in the tracr polynucleotide.

As used herein, a "CAVA guide" refers to a guide comprising a spacer sequence and further comprising RNA with one or more base analogs designed into the polynucleotide. The one or more base analogs can be designed at any position in the polynucleotide and is preferably designed in the spacer sequence. The CAVA guide can include a tracr polynucleotide, either hybridized to the CRISPR polynucleotide component so as to remain as separate molecules in a dual-guide system (*e.g*., Type II systems), or covalently linked to the CRISPR polynucleotide component to form a single polynucleotide in a single-guide system. In CAVA guides that include a tracr polynucleotide, the one or more base analogs can be designed in the tracr polynucleotide. In an alternative embodiment, the CAVA guide does not include a tracr polynucleotide (*e.g*., a Type V system or a Type VI system). In any of these embodiments, the CAVA guide is capable of forming a nucleoprotein complex with a Cas protein and binding a target nucleic acid sequence complementary to the spacer sequence.

As used herein, a "CABRNT-CAVA polynucleotide" refers to a CRISPR polynucleotide that includes one or more abasic sites and one or more base analogs designed into the polynucleotide. The one or more abasic sites and the one or more base analogs can be designed at any position in the polynucleotide, preferably designed in the spacer sequence. In other embodiments, the one or more abasic sites and the one or more base analogs can be designed in the tracr polynucleotide.

As used herein, a "CABRNT-CAVA guide" refers to a guide comprising a spacer sequence and further comprising RNA with one or more abasic sites and one or more base analogs designed into the polynucleotide. The one or more abasic sites and the one or more base analogs can be designed at any position in the polynucleotide, preferably designed in the spacer sequence. The CABRNT-CAVA guide can include a tracr polynucleotide, either hybridized to the CRISPR polynucleotide component so as to remain as separate molecules in a dual-guide system (*e.g*., Type II systems) or covalently linked to the CRISPR polynucleotide component to form a single polynucleotide in a single-guide system. In CABRNT-CAVA guides that include a tracr polynucleotide, the one or more abasic sites and the one or more base analogs can be designed in the tracr polynucleotide. In an alternative embodiment, the CABRNT-CAVA guide lacks a tracr polynucleotide (e.g., a Type V system or a Type VI system). In any of these embodiments, the CABRNT-CAVA guide is capable of forming a nucleoprotein complex with a Cas protein and binding a target nucleic acid sequence complementary to the spacer sequence.

It will be understood that, because a CABRNT-CAVA polynucleotide or guide contains at least one abasic site, a CABRNT-CAVA polynucleotide or guide also falls within the scope of a CABRNT-polynucleotide or guide, respectively. It will also be understood that, because a CABRNT-CAVA polynucleotide or guide contains at least one base analog, a CABRNT-CAVA polynucleotide or guide also falls within the scope of a CAVA-polynucleotide or guide, respectively.

As used herein, a "stem element" or "stem structure" refers to two strands of nucleic acids that form a double-stranded region (the "stem element"). A "stem-loop element" or "stem-loop structure" refers to a stem structure wherein 3'-end sequences of one strand are covalently bonded to 5'-end sequences of the second strand by a nucleotide sequence of typically single-stranded nucleotides ("a stem-loop element nucleotide sequence"). In some embodiments, the loop element comprises a loop element nucleotide sequence of between about 3 and about 20 nucleotides in length, preferably between about 4 and about 10 nucleotides in length. In some embodiments, a loop element nucleotide sequence is a single-stranded nucleotide sequence of unpaired nucleic acid bases that do not interact through hydrogen bond formation to create a stem element within the loop element nucleotide sequence. The term "hairpin element" is also used herein to refer to stem-loop structures. Such structures are well known in the art. The base pairing may be exact; however, as is known in the art, a stem element does not require exact base pairing. Thus, the stem element may include one or more base mismatches or non-paired bases.

A "linker element nucleotide sequence," "linker nucleotide sequence," "linker sequence," and "linker polynucleotide" are used interchangeably herein and refer to a sequence of one or more nucleotides covalently attached to a first nucleic acid sequence (5'-linker nucleotide sequence-first nucleic acid sequence-3'). In some embodiments, a linker nucleotide sequence connects two separate nucleic acid sequences to form a single polynucleotide (e.g., 5'-first nucleic acid sequence-linker nucleotide sequence-second nucleic acid sequence-3'). Other examples of linker sequences include, but are not limited to, 5'-first nucleic acid sequence-linker nucleotide sequence-3', and 5'-linker nucleotide sequence-first first nucleic acid sequence-linker nucleotide sequence-3'. In some embodiments, the linker element nucleotide sequence can be a single-stranded nucleotide sequence of unpaired nucleic acid bases that do not interact with each other through hydrogen bond formation to create a secondary structure (e.g., a stem-loop structure) within the linker element nucleotide sequence. In some embodiments, two single-stranded linker element nucleotide sequences can interact with each other through hydrogen bonding between the two linker element nucleotide sequences. In some embodiments, a linker element nucleotide sequence can be between about 1 and about 50 nucleotides in length, preferably between about 1 and about 15 nucleotides in length.

As used herein, a "dual guide" refers to a two-component guide capable of associating with a cognate Cas protein, such as a Type II CRISPR-Cas9 protein. FIG. 1A illustrates an example of an *S. pyogenes* dual guide comprising a crRNA (FIG. 1A, 101) and a tracrRNA (FIG. 1A, 102). FIG. 1B presents an overview of and nomenclature for secondary structural elements of the crRNA and tracrRNA *of S. pyogenes,* including the following: a spacer (FIG. 1B, 103) (also referred to herein as a spacer sequence or nucleic acid target binding sequence); a first stem element (FIG. 1B, 104, 105, 106) comprising a lower stem element (FIG. 1B, 104), a bulge element comprising unpaired nucleotides (FIG. 1B, 105), and an upper stem element (FIG. 1B, 106); a nexus element comprising a second stem element (FIG. 1B, 107; also referred to herein as a nexus or nexus stem-loop element,see, *e.g.,* Briner et al. (Mol. Cell., 2014, 23;56(2):333-339); Nowak etal. (Nucleic Acids Res., 2016, 44 (20):9555-9564); and Wright et al. (Proceedings of the National Academy of Sciences of the United States of America, 2015, 112(10):2984-2989)); a first 3' hairpin element (FIG. 1B, 108) comprising a third stem element; and a second 3' hairpin element (FIG. 1B, 109) comprising a fourth stem element. In some Type II CRISPR-Cas9 systems, the first stem element does not have a bulge element *(e.g., C. jejuni).* A CRISPR dual guide is capable of forming a nucleoprotein complex with a cognate Cas protein, such as a Cas9 protein, wherein the complex is capable of targeting a nucleic acid target sequence complementary to the spacer sequence. A CRISPR dual guide typically comprises two polynucleotides: a first polynucleotide comprising a spacer *(e.g.,* a crRNA) and a second polynucleotide comprises a nexus and a 3' hairpin *(e.g.,* a tracrRNA).

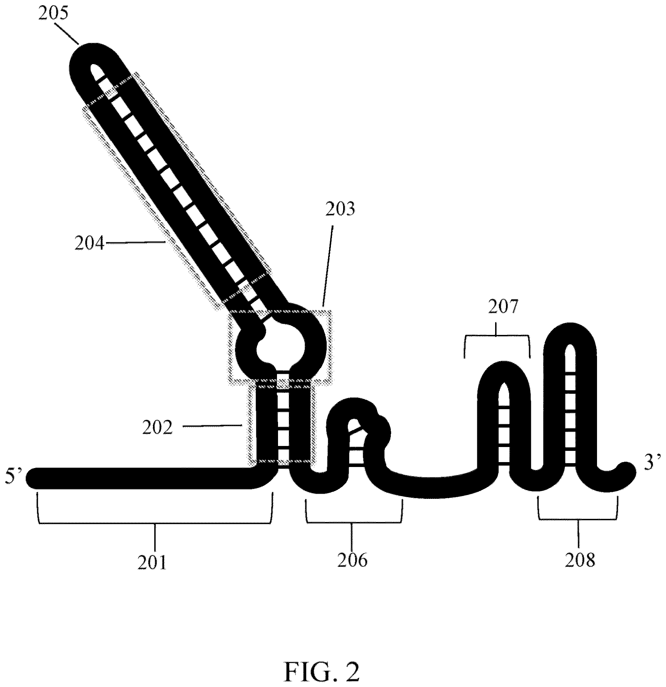

As used herein, "single guide" or "sg" refers to a one-component guide capable of associating with a cognate Cas protein, such as a Type II CRISPR-Cas9 protein, to form a nucleoprotein complex. The secondary structural elements of a Cas-sgRNA correspond to the secondary structural elements of a CRISPR dual-guide RNA. FIG. 2 presents an overview of and nomenclature for secondary structural elements of a Cas9-sgRNA for *S. pyogenes* including the following: a spacer element (FIG. 2A, 201); a first stem-loop element (FIG. 2, 202, 203, 204, 205) comprising a first stem element that comprises a lower stem element (FIG. 2, 202), a bulge element comprising unpaired nucleotides (FIG. 2A, 203), and an upper stem element (FIG. 2, 204), and a loop element (or linker polynucleotide) (FIG. 2, 205) comprising unpaired nucleotides; a nexus element (FIG. 2, 206) comprising a second stem element; a first 3' hairpin element (FIG. 2, 207) comprising a third stem element; and a second 3' hairpin element comprising a fourth stem element (FIG. 2, 208). As used herein, the term single-guide chRDNA (sg-chRDNA) comprises a chRDNA, crRNA, or modification thereof, and a ch-acr, tracrRNA, or modification thereof, covalently linked via a polynucleotide (e.g., a tetra-loop linker polynucleotide sequence), wherein a linker polynucleotide connects the 3' end of the chRDNA, crRNA, or modification thereof, to the 5' end of the ch-acr, tracrRNA, or modification thereof. As used herein, the term single-guide CABRNT (sg-CABRNT) comprises a CABRNT polynucleotide, crRNA, or modification thereof, and a ch-acr, tracrRNA, or modification thereof, covalently linked via a polynucleotide (*e.g*., a tetra-loop linker polynucleotide sequence), wherein a linker polynucleotide connects the 3' end of the CABRNT polynucleotide, crRNA, or modification thereof, to the 5' end of the ch-acr, tracrRNA, or modification thereof. As used herein, the term single-guide CAVA (sg-CAVA) comprises a CAVA polynucleotide, crRNA, or modification thereof, and a ch-acr, a tracrRNA, or modification thereof, covalently linked via a polynucleotide (e.g., a tetra-loop linker polynucleotide sequence), wherein a linker polynucleotide connects the 3' end of the CAVA polynucleotide, crRNA, or modification thereof, to the 5' end of the ch-acr, tracrRNA, or modification thereof. As used herein, the term single-guide CABRNT-CAVA (sg-CABRNT-CAVA) comprises a CABRNT-CAVA polynucleotide, crRNA, or modification thereof, and a ch-acr, tracrRNA, or modification thereof, covalently linked via a polynucleotide (e.g., a tetra-loop linker polynucleotide sequence), wherein a linker polynucleotide connects the 3' end of the CABRNT-CAVA polynucleotide, crRNA, or modification thereof, to the 5' end of the ch-acr, tracrRNA, or modification thereof.

As used herein, the term "cognate" typically refers to a Cas protein (e.g., Cas9) and one or more guides (e.g., Type II CRISPR-Cas9-associated guides) that are capable of forming a nucleoprotein complex capable of site-directed binding to a nucleic acid target sequence complementary to the nucleic acid target binding sequence present in one of the one or more guides.

The terms "wild-type," "naturally occurring," and "unmodified" are used herein to mean the typical (or most common) form, appearance, phenotype, or strain existing in nature; for example, the typical form of cells, organisms, polynucleotides, proteins, macromolecular complexes, genes, RNAs, DNAs, or genomes as they occur in, and can be isolated from, a source in nature. The wild-type form, appearance, phenotype, or strain serve as the original parent before an intentional modification. Thus, mutant, variant, engineered, recombinant, and modified forms are not wild-type forms.

By "isolated" is meant, when referring to a polypeptide, that the indicated molecule is separate and discrete from the whole organism with which the molecule is found in nature or is present in the substantial absence of other biological macromolecules of the same type. The term "isolated" with respect to a polynucleotide is a nucleic acid molecule devoid, in whole or part, of sequences normally associated with it in nature; or a sequence, as it exists in nature, but having heterologous sequences in association therewith; or a molecule disassociated from the chromosome.

The term "purified" as used herein preferably means at least 75% by weight, more preferably at least 85% by weight, more preferably still at least 95% by weight, and most preferably at least 98% by weight, of the same molecule is present.

The terms "engineered," "genetically engineered," "genetically modified," "recombinant," "modified," "non-naturally occurring," and "non-native" indicate intentional human manipulation of the genome of an organism or cell. The terms encompass methods of genomic modification that include genomic editing, as defined herein, as well as techniques that alter gene expression or inactivation, enzyme engineering, directed evolution, knowledge-based design, random mutagenesis methods, gene shuffling, codon optimization, and the like. Methods for genetic engineering are known in the art.

"Covalent bond," "covalently attached," "covalently bound," "covalently linked," "covalently connected," and "molecular bond" are used interchangeably herein and refer to a chemical bond that involves the sharing of electron pairs between atoms. Examples of covalent bonds include, but are not limited to, phosphodiester bonds and phosphorothioate bonds.

"Non-covalent bond," "non-covalently attached," "non-covalently bound," "non-covalently linked," "non-covalent interaction," and "non-covalently connected" are used interchangeably herein, and refer to any relatively weak chemical bond that does not involve sharing of a pair of electrons. Multiple non-covalent bonds often stabilize the conformation of macromolecules and mediate specific interactions between molecules. Examples of non-covalent bonds include, but are not limited to hydrogen bonding, ionic interactions (e.g., Na⁺Cl⁻), van der Waals interactions, and hydrophobic bonds.

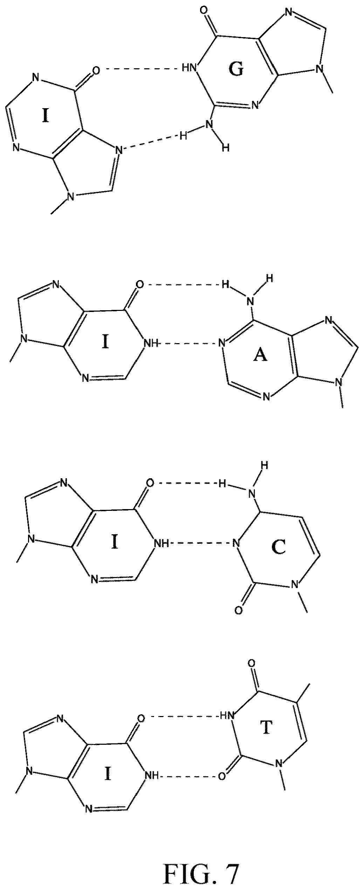

As used herein, "hydrogen bonding," "hydrogen-base pairing," and "hydrogen bonded" are used interchangeably and refer to canonical hydrogen bonding and non-canonical hydrogen bonding including, but not limited to, "Watson-Crick-hydrogen-bonded base pairs" (W-C-hydrogen-bonded base pairs or W-C hydrogen bonding); "Hoogsteen-hydrogen-bonded base pairs" (Hoogsteen hydrogen bonding); and "wobble-hydrogen-bonded base pairs" (wobble hydrogen bonding). W-C hydrogen bonding, including reverse W-C hydrogen bonding, refers to purine-pyrimidine base pairing, that is, adenine:thymine, guanine: cytosine, and uracil: adenine. Hoogsteen hydrogen bonding, including reverse Hoogsteen hydrogen bonding, refers to a variation of base pairing in nucleic acids wherein two nucleobases, one on each strand, are held together by hydrogen bonds in the major groove. This non-W-C hydrogen bonding can allow a third strand to wind around a duplex and form triple-stranded helices. Wobble hydrogen bonding, including reverse wobble hydrogen bonding, refers to a pairing between two nucleotides in RNA molecules that does not follow Watson-Crick base pair rules. There are four major wobble base pairs: guanine:uracil, inosine (hypoxanthine):uracil, inosine:adenine, and inosine: cytosine. Wobble base interaction are also known to occur between inosine: thymine and inosine: guanine. Inosine bases and deoxy inosine bases can be referred to as "universal pairing bases" as they are capable of hydrogen bonding with the canonical DNA and RNA bases *(see, e.g.,* FIG. 7). *See also* Watkins et al. (Nucleic Acid Research, 2005, 33(19):6258-67). Rules for canonical hydrogen bonding and non-canonical hydrogen bonding are known to those of ordinary skill in the art. *See, e.g.,* R. F. Gesteland (The RNA World, Third Edition (Cold Spring Harbor Monograph Series), 2005, Cold Spring Harbor Laboratory Press, ISBN 978-0879697396); R. F. Gesteland (The RNA World, Second Edition (Cold Spring Harbor Monograph Series), 1999, Cold Spring Harbor Laboratory Press, ISBN 978-0879695613); R. F. Gesteland (The RNA World, First Edition (Cold Spring Harbor Monograph Series), 1993, Cold Spring Harbor Laboratory Press, 978-0879694562) *(see, e.g.,* Appendix 1: Structures of Base Pairs Involving at Least Two Hydrogen Bonds, I. Tinoco); W. Saenger (Principles of Nucleic Acid Structure, 1988, Springer International Publishing AG, ISBN 978-0-387-90761-1); S. Neidle (Principles of Nucleic Acid Structure, 2007, First Edition, Academic Press, ISBN 978-01236950791).

"Connect," "connected," and "connecting" are used interchangeably herein, and refer to a covalent bond or a non-covalent bond between two macromolecules (e.g., polynucleotides, proteins, and the like).

As used herein, the terms "nucleic acid sequence," "nucleotide sequence," and "oligonucleotide" are interchangeable and refer to a polymeric form of nucleotides. As used herein, the term "polynucleotide" refers to a polymeric form of nucleotides that has one 5' end and one 3' end and can comprise one or more nucleic acid sequences. The nucleotides may be deoxyribonucleotides (DNA), ribonucleotides (RNA), analogs thereof, or combinations thereof, and may be of any length. Polynucleotides may perform any function and may have various secondary and tertiary structures. The terms encompass known analogs of natural nucleotides and nucleotides that are modified in the base, sugar, and/or phosphate moieties. Analogs of a particular nucleotide have the same base-pairing specificity (e.g., an analog of A base pairs with T). A polynucleotide may comprise one modified nucleotide or multiple modified nucleotides. Examples of modified nucleotides include fluorinated nucleotides, methylated nucleotides, and nucleotide analogs. Nucleotide structure may be modified before or after a polymer is assembled. Following polymerization, polynucleotides may be additionally modified via, for example, conjugation with a labeling component or target binding component. A nucleotide sequence may incorporate non-nucleotide components. The terms also encompass nucleic acids comprising modified backbone residues or linkages, that are synthetic, naturally occurring, and/or non-naturally occurring, and have similar binding properties as a reference polynucleotide (e.g., DNA or RNA). Examples of such analogs include, but are not limited to, phosphorothioates, phosphoramidates, methyl phosphonates, chiral-methyl phosphonates, 2-O-methyl ribonucleotides, peptide-nucleic acids (PNAs), Locked Nucleic Acid (LNA^{™}) (Exiqon, Woburn, MA) nucleosides, glycol nucleic acid, bridged nucleic acids, and morpholino structures.

Peptide-nucleic acids (PNAs) are synthetic homologs of nucleic acids wherein the polynucleotide phosphate-sugar backbone is replaced by a flexible pseudo-peptide polymer. Nucleobases are linked to the polymer. PNAs have the capacity to hybridize with high affinity and specificity to complementary sequences of RNA and DNA.

In phosphorothioate nucleic acids, the phosphorothioate (PS) bond substitutes a sulfur atom for a non-bridging oxygen in the polynucleotide phosphate backbone. This modification makes the internucleotide linkage resistant to nuclease degradation. In some embodiments, phosphorothioate bonds are introduced between the last 3 to 5 nucleotides at the 5'-end or 3'-end sequences of a polynucleotide sequence to inhibit exonuclease degradation. Placement of phosphorothioate bonds throughout an entire oligonucleotide helps reduce degradation by endonucleases as well.

Threose nucleic acid (TNA) is an artificial genetic polymer. The backbone structure of TNA comprises repeating threose sugars linked by phosphodiester bonds. TNA polymers are resistant to nuclease degradation. TNA can self-assemble by base-pair hydrogen bonding into duplex structures.

Linkage inversions can be introduced into polynucleotides through use of "reversed phosphoramidites" *(see, e.g.,* www.ucalgary.ca/dnalab/synthesis/- modifications/linkages). A 3'-3' linkage at a terminus of a polynucleotide stabilizes the polynucleotide to exonuclease degradation by creating an oligonucleotide having two 5'-OH termini but lacking a 3'-OH terminus. Typically, such polynucleotides have phosphoramidite groups on the 5'-OH position and a dimethoxytrityl (DMT) protecting group on the 3'-OH position. Normally, the DMT protecting group is on the 5'-OH and the phosphoramidite is on the 3'-OH.

Polynucleotide sequences are displayed herein in the conventional 5' to 3' orientation unless otherwise indicated.

As used herein, "sequence identity" generally refers to the percent identity of nucleotide bases or amino acids comparing a first polynucleotide or polypeptide to a second polynucleotide or polypeptide using algorithms having various weighting parameters. Sequence identity between two polynucleotides or two polypeptides can be determined using sequence alignment by various methods and computer programs (e.g., BLAST, CS-BLAST, FASTA, HMMER, L-ALIGN, and the like) available through the worldwide web at sites including, but not limited to, GENBANK (www.ncbi.nlm.nih.gov/genbank/) and EMBL-EBI (www.ebi.ac.uk.). Sequence identity between two polynucleotides or two polypeptide sequences is generally calculated using the standard default parameters of the various methods or computer programs. A high degree of sequence identity between two polynucleotides or two polypeptides is typically between about 90% identity and 100% identity over the length of the reference polypeptide, for example, about 90% identity or higher, preferably about 95% identity or higher, more preferably about 98% identity or higher. A moderate degree of sequence identity between two polynucleotides or two polypeptides is typically between about 80% identity to about 85% identity, for example, about 80% identity or higher, preferably about 85% identity over the length of the reference polypeptide. A low degree of sequence identity between two polynucleotides or two polypeptides is typically between about 50% identity and 75% identity, for example, about 50% identity, preferably about 60% identity, more preferably about 75% identity over the length of the reference polypeptide. For example, a Cas protein *(e.g.,* a Cas9 comprising amino acid substitutions) can have a low degree of sequence identity, a moderate degree of sequence identity, or a high degree of sequence identity, over its length to a reference Cas protein *(e.g.,* a wild-type Cas9) over its length. As another example, a guide can have a low degree of sequence identity, a moderate degree of sequence identity, or a high degree of sequence identity, over its length compared to a reference wild-type guide over its length that complexes with the reference Cas protein *(e.g.,* an sgRNA that forms a complex with Cas9).

As used herein, "hybridization," "hybridize," or "hybridizing" is the process of combining two complementary single-stranded DNA or RNA molecules so as to form a single double-stranded molecule (DNA/DNA, DNA/RNA, RNA/RNA) through hydrogen base pairing. Hybridization stringency is typically determined by the hybridization temperature and the salt concentration of the hybridization buffer; *e.g.,* high temperature and low salt provide high stringency hybridization conditions. Examples of salt concentration ranges and temperature ranges for different hybridization conditions are as follows: high stringency, approximately 0.01M to approximately 0.05M salt, hybridization temperature 5°C to 10°C below Tₘ; moderate stringency, approximately 0.16M to approximately 0.33M salt, hybridization temperature 20°C to 29°C below Tₘ; and low stringency, approximately 0.33M to approximately 0.82M salt, hybridization temperature 40 °C to 48 °C below Tₘ. Tₘ of duplex nucleic acid sequences is calculated by standard methods well-known in the art. *See, e.g.,* Maniatis et al. (Molecular Cloning: A Laboratory Manual, 1982, Cold Spring Harbor Laboratory Press: New York); Casey et al. (Nucleic Acids Research, 1977, 4: 1539-1552); Bodkin et al. (Journal of Virological Methods, 1985, 10(1):45-52); and Wallace et al. (Nucleic Acids Research, 1981, 9(4):879-894). Algorithm prediction tools to estimate Tₘ are also widely available. High stringency conditions for hybridization typically refer to conditions under which a polynucleotide complementary to a target sequence predominantly hybridizes with the target sequence, and substantially does not hybridize to non-target sequences. Typically, hybridization conditions are of moderate stringency, preferably high stringency.

As used herein, "complementarity" refers to the ability of a nucleic acid sequence to form hydrogen bonds with another nucleic acid sequence (*e.g*., through canonical Watson-Crick base pairing). A percent complementarity indicates the percentage of residues in a nucleic acid sequence that can form hydrogen bonds with a second nucleic acid sequence. If two nucleic acid sequences have 100% complementarity, the two sequences are perfectly complementary, *i.e.,* all of the contiguous residues of a first polynucleotide hydrogen bond with the same number of contiguous residues in a second polynucleotide.

As used herein, "binding" refers to a non-covalent interaction between macromolecules (*e.g*., between a protein and a polynucleotide, between a polynucleotide and a polynucleotide, or between a protein and a protein, and the like). Such non-covalent interaction is also referred to as "associating" or "interacting" *(e.g.,* if a first macromolecule interacts with a second macromolecule, the first macromolecule binds to second macromolecule in a non-covalent manner). Some portions of a binding interaction may be sequence-specific (the terms "sequence-specific binding," "sequence-specifically bind," "site-specific binding," and "site specifically binds" are used interchangeably herein). Sequence-specific binding typically refers to one or more guides capable of forming a complex with a protein (*e.g*., Cas9) to cause the protein to bind a nucleic acid sequence (*e.g*., a DNA sequence) comprising a nucleic acid target sequence (*e.g*., a target DNA sequence) preferentially relative to a second nucleic acid sequence *(e.g.,* a second DNA sequence) without the nucleic acid target binding sequence *(e.g.,* the DNA target binding sequence). All components of a binding interaction do not need to be sequence-specific, such as contacts of a protein with phosphate residues in a DNA backbone. Binding interactions can be characterized by a dissociation constant (Kd). "Binding affinity" refers to the strength of the binding interaction. An increased binding affinity is correlated with a lower Kd.

As used herein, a Cas protein is said to "target" a polynucleotide if a Cas protein/guide nucleoprotein complex binds or cleaves a polynucleotide at the nucleic acid target sequence within the polynucleotide.

A "protospacer adjacent motif' or "PAM" as used herein refers to double-stranded nucleic acid sequences comprising a Cas protein-binding recognition sequence, wherein amino acids of the Cas protein directly interact with the recognition sequence (e.g., Cas9 protein interacts with the PAM 5'-NGG-3' and Cas12a protein interacts with the PAM 5'-TTTN-3'). The PAM sequence is on the non-target strand and can be 5' or 3' of a target complement sequence (*e.g.*, in CRISPR-Cas9 systems the PAM 5'-NGG-3 sequence is on the non-target strand and is 3' of the target complement sequence; in CRISPR-Cas12a systems the PAM 5'-TTTN-3' sequence is on the non-target strand and is 5' of the target-complement sequence). PAMs are recognized by the Cas effector proteins *(e.g.,* a Cas9 protein or a Cas12a protein) prior to target sequence unwinding and hydrogen base-pair bonding between the target sequence and the nucleic acid target binding sequence.

"Target," "target sequence," "nucleic acid target sequence," "target nucleic acid sequence," and "on-target sequence" are used interchangeably herein to refer to a nucleic acid sequence that is wholly, or in part, complementary to a nucleic acid target binding sequence of a Cas polynucleotide (*e.g*., the spacer of a crRNA). Typically, the nucleic acid target binding sequence is selected to be 100% complementary to a nucleic acid target sequence to which binding of a Cas nucleoprotein complex is being directed; however, to attenuate binding to a nucleic acid target sequence, lower percent complementarity can be used.

When the nucleic acid target binding sequence is 100% complementary to the target sequence, excluding abasic sites contained in the nucleic acid target binding sequence, the target sequence is referred to as an "on-target." On-target sequence binding refers to binding of the Cas nucleoprotein complex to a nucleic acid sequence having 100% complementarity to the non-abasic site portion of the nucleic acid target binding sequence (spacer). When the nucleic acid target binding sequence (spacer) has less than 100% complementary to the target sequence, excluding abasic sites contained in the nucleic acid target binding sequence, the target sequence can be referred to as an "off-target." Off-target sequence binding refers to binding of the Cas nucleoprotein complex to nucleic acid sequences having less than 100% complementarity to the non-abasic site portion of the nucleic acid target binding sequence (spacer). The nucleic acid target sequence can be a double-stranded or a single-stranded DNA molecule. The target sequence can be a double-stranded or single-stranded RNA molecule. The target sequence can be a RNA:DNA hybrid molecule. The target sequence can be present on the opposite strand of a PAM sequence.

As used herein, "double-strand break" (DSB) refers to both strands of a double-stranded segment of DNA being severed. In some instances, if such a break occurs, one strand can be said to have a "sticky end" wherein nucleotides are exposed and not hydrogen bonded to nucleotides on the other strand. In other instances, a "blunt end" can occur wherein both strands remain fully base paired with each other.

"Donor polynucleotide," "donor oligonucleotide," "donor template," "non-viral donor," and "non-viral template" are used interchangeably herein and can be a double-stranded polynucleotide *(e.g.,* DNA), a single-stranded polynucleotide *(e.g.,* DNA or RNA), or a combination thereof. Donor polynucleotides can comprise homology arms flanking the insertion sequence (e.g., DSBs in the DNA). The homology arms on each side can vary in length. Parameters for the design and construction of donor polynucleotides are well-known in the art. *See, e.g.,* Ran et al. (Nature Protocols, 2013, 8(11):2281-2308); Smithies et al. (Nature, 1985, 317:230-234); Thomas et al. (Cell, 1986, 44:419-428); Wu et al. (Nature Protocols, 2008, 3:1056-1076); Singer et al. (Cell, 1982, 31:25-33); Shen et al. (Genetics, 1986, 112:441-457); Watt et al. (PNAS, 1985, 82:4768-4772); Sugawara et al. (Journal of Molecular Cell Biology, 1992, 12(2):563-575); Rubnitz et al. (Journal of Molecular Cell Biology, 1984, 4(11):2253-2258); Ayares et al. (PNAS, 1986, 83(14):5199-5203); and Liskay et al. (Genetics, 1987, 115(1): 161-167). In some embodiments, a donor polynucleotide comprises a chimeric antigen receptor (CAR).

As used herein, "homology-directed repair" (HDR) refers to DNA repair that takes place in cells, for example, during repair of a DSB in DNA. HDR requires nucleotide sequence homology and uses a donor polynucleotide to repair the sequence wherein the DSB (e.g., within a target DNA sequence) occurred. The donor polynucleotide generally has the requisite sequence homology with the sequence flanking the DSB so that the donor polynucleotide can serve as a suitable template for repair. HDR results in the transfer of genetic information from, for example, the donor polynucleotide to the target DNA sequence. HDR may result in alteration of the target DNA sequence (e.g., insertion, deletion, or mutation) if the donor polynucleotide sequence differs from the target DNA sequence and part or all of the donor polynucleotide is incorporated into the target DNA sequence. In some embodiments, an entire donor polynucleotide, a portion of the donor polynucleotide, or a copy of the donor polynucleotide is integrated at the site of the target DNA sequence. For example, a donor polynucleotide can be used for repair of the break in the target DNA sequence, wherein the repair results in the transfer of genetic information (e.g., polynucleotide sequences) from the donor polynucleotide at the site or in close proximity of the break in the DNA. Accordingly, new genetic information (e.g., polynucleotide sequences) may be inserted or copied at a target DNA sequence.

A "genomic region" is a segment of a chromosome in the genome of a host cell that is present on either side of the nucleic acid target sequence site or, alternatively, also includes a portion of the nucleic acid target sequence site. The homology arms of the donor polynucleotide have sufficient homology to undergo homologous recombination with the corresponding genomic regions. In some embodiments, the homology arms of the donor polynucleotide share significant sequence homology to the genomic region immediately flanking the nucleic acid target sequence site; it is recognized that the homology arms can be designed to have sufficient homology to genomic regions farther from the nucleic acid target sequence site.

As used herein, "non-homologous end joining" (NHEJ) refers to the repair of a DSB in DNA by direct ligation of one terminus of the break to the other terminus of the break without a requirement for a donor polynucleotide. NHEJ is a DNA repair pathway available to cells to repair DNA without the use of a repair template. NHEJ in the absence of a donor polynucleotide often results in nucleotides being randomly inserted or deleted at the site of the DSB.

"Microhomology-mediated end joining" (MMEJ) is pathway for repairing a DSB in DNA. MMEJ involves deletions flanking a DSB and alignment of microhomologous sequences internal to the break site before joining. MMEJ is genetically defined and requires the activity of, for example, CtIP, Poly(ADP-Ribose) Polymerase 1 (PARP1), DNA polymerase theta (Pol 0), DNA Ligase 1 (Lig 1), or DNA Ligase 3 (Lig 3). Additional genetic components are known in the art. *See, e.g.,* Sfeir et al. (Trends in Biochemical Sciences, 2015, 40:701-714).

As used herein, "DNA repair" encompasses any process whereby cellular machinery repairs damage to a DNA molecule contained in the cell. The damage repaired can include single-strand breaks or double-strand breaks (DSBs). At least three mechanisms exist to repair DSBs: HDR, NHEJ, and MMEJ. "DNA repair" is also used herein to refer to DNA repair resulting from human manipulation, wherein a target locus is modified, *e.g.,* by inserting, deleting, or substituting nucleotides, all of which represent forms of genome editing.

As used herein, "recombination" refers to a process of exchange of genetic information between two polynucleotides.

As used herein, the terms "regulatory sequences," "regulatory elements," and "control elements" are interchangeable and refer to polynucleotide sequences that are upstream (5' non-coding sequences), within, or downstream (3' non-translated sequences) of a polynucleotide target to be expressed. Regulatory sequences influence, for example, the timing of transcription, amount or level of transcription, RNA processing or stability, and/or translation of the related structural nucleotide sequence. Regulatory sequences may include activator binding sequences, enhancers, introns, polyadenylation recognition sequences, promoters, transcription start sites, repressor binding sequences, stem-loop structures, translational initiation sequences, internal ribosome entry sites (IRES), translation leader sequences, transcription termination sequences (e.g., polyadenylation signals and poly-U sequences), translation termination sequences, primer binding sites, and the like.

Regulatory elements include those that direct constitutive, inducible, and repressible expression of a nucleotide sequence in many types of host cells and those that direct expression of the nucleotide sequence only in certain host cells (e.g., tissue-specific regulatory sequences). In some embodiments, a vector comprises one or more pol III promoters, one or more pol II promoters, one or more pol I promoters, or combinations thereof. Examples of pol III promoters include, but are not limited to, U6 and H1 promoters. Examples of pol II promoters include, but are not limited to, the retroviral Rous sarcoma virus (RSV) LTR promoter (optionally with the RSV enhancer), the cytomegalovirus (CMV) promoter (optionally with the CMV enhancer; see, *e.g.,* Boshart et al. (Cell, 1985, 41:521-530)), the SV40 promoter, the dihydrofolate reductase promoter, the β-actin promoter, the phosphoglycerol kinase (PGK) promoter, and the EF1α promoter. It will be appreciated by those skilled in the art that the design of an expression vector may depend on such factors as the choice of the host cell to be transformed, the level of expression desired, and the like. A vector can be introduced into host cells to thereby produce transcripts, proteins, or peptides, including fusion proteins or peptides, encoded by nucleic acid sequences as described herein.

"Gene" as used herein refers to a polynucleotide sequence comprising exons and related regulatory sequences. A gene may further comprise introns and/or untranslated regions (UTRs).

As used herein, the term "operably linked" refers to polynucleotide sequences or amino acid sequences placed into a functional relationship with one another. For example, regulatory sequences (e.g., a promoter or enhancer) are "operably linked" to a polynucleotide encoding a gene product if the regulatory sequences regulate or contribute to the modulation of the transcription of the polynucleotide. Operably linked regulatory elements are typically contiguous with the coding sequence. However, enhancers can function if separated from a promoter by up to several kilobases or more. Accordingly, some regulatory elements may be operably linked to a polynucleotide sequence but not contiguous with the polynucleotide sequence. Similarly, translational regulatory elements contribute to the modulation of protein expression from a polynucleotide.

As used herein, "expression" refers to transcription of a polynucleotide from a DNA template, resulting in, for example, a messenger RNA (mRNA) or other RNA transcript (e.g., non-coding, such as structural or scaffolding RNAs). The term further refers to the process through which transcribed mRNA is translated into peptides, polypeptides, or proteins. Transcripts and encoded polypeptides may be referred to collectively as "gene products." Expression may include splicing the mRNA in a eukaryotic cell, if the polynucleotide is derived from genomic DNA.

A "coding sequence" or a sequence that "encodes" a selected polypeptide, is a nucleic acid molecule that is transcribed (in the case of DNA) and translated (in the case of mRNA) into a polypeptide *in vitro* or *in vivo* when placed under the control of appropriate regulatory sequences. The boundaries of the coding sequence are determined by a start codon at the 5' terminus and a translation stop codon at the 3' terminus. A transcription termination sequence may be located 3' to the coding sequence.

As used herein, the term "modulate" refers to a change in the quantity, degree or amount of a function. For example, a Cas9-guide/Cas9 protein nucleoprotein complex, as disclosed herein, may modulate the activity of a promoter sequence by binding to a nucleic acid target sequence at or near the promoter. Depending on the action occurring after binding, the Cas9-guide/Cas9 protein nucleoprotein complex can induce, enhance, suppress, or inhibit transcription of a gene operatively linked to the promoter sequence. Thus, "modulation" of gene expression includes both gene activation and gene repression.

Modulation can be assayed by determining any characteristic directly or indirectly affected by the expression of the target gene. Such characteristics include, for example, changes in RNA or protein levels, protein activity, product levels, expression of the gene, or activity level of reporter genes. Accordingly, the terms "modulating expression," "inhibiting expression," and "activating expression" of a gene can refer to the ability of a Cas9-guide/Cas9 protein nucleoprotein complex to change, activate, or inhibit transcription of a gene.

"Vector" and "plasmid" as used herein refer to a polynucleotide vehicle to introduce genetic material into a cell. Vectors can be linear or circular. Vectors can contain a replication sequence capable of effecting replication of the vector in a suitable host cell (*e.g.,* an origin of replication). Upon transformation of a suitable host, the vector can replicate and function independently of the host genome or integrate into the host genome. Vector design depends, among other things, on the intended use and host cell for the vector, and the design of a vector of the invention for a particular use and host cell is within the level of skill in the art. The four major types of vectors are plasmids, viral vectors, cosmids, and artificial chromosomes. Typically, vectors comprise an origin of replication, a multicloning site, and/or a selectable marker. An expression vector typically comprises an expression cassette. By "recombinant virus" is meant a virus that has been genetically altered, *e.g.,* by the addition or insertion of a heterologous nucleic acid construct into a viral genome or portion thereof.

As used herein, "expression cassette" refers to a polynucleotide construct generated using recombinant methods or by synthetic means and comprising regulatory sequences operably linked to a selected polynucleotide to facilitate expression of the selected polynucleotide in a host cell. For example, the regulatory sequences can facilitate transcription of the selected polynucleotide in a host cell, or transcription and translation of the selected polynucleotide in a host cell. An expression cassette can, for example, be integrated in the genome of a host cell or be present in a vector to form an expression vector.

As used herein, a "targeting vector" is a recombinant DNA construct typically comprising tailored DNA arms, homologous to genomic DNA, that flank elements of a target gene or nucleic acid target sequence (*e.g*., a DSB). A targeting vector comprises a donor polynucleotide. Elements of the target gene can be modified in a number of ways including deletions and/or insertions. A defective target gene can be replaced by a functional target gene, or in the alternative a functional gene can be knocked out. Optionally, the donor polynucleotide of a targeting vector comprises a selection cassette comprising a selectable marker that is introduced into the target gene. Targeting regions adjacent or within a target gene can be used to affect regulation of gene expression.

As used herein, the term "between" is inclusive of end values in a given range (*e.g*., between about 1 and about 50 nucleotides in length includes 1 nucleotide and 50 nucleotides.

As used herein, the term "amino acid" refers to natural and synthetic (unnatural) amino acids, including amino acid analogs, modified amino acids, peptidomimetics, glycine, and D or L optical isomers.

As used herein, the terms "peptide," "polypeptide," and "protein" are interchangeable and refer to polymers of amino acids. A polypeptide may be of any length. It may be branched or linear, it may be interrupted by non-amino acids, and it may comprise modified amino acids. The terms also refer to an amino acid polymer that has been modified through, for example, acetylation, disulfide bond formation, glycosylation, lipidation, phosphorylation, pegylation, biotinylation, cross-linking, and/or conjugation (*e.g*., with a labeling component or ligand). Polypeptide sequences are displayed herein in the conventional N-terminal to C-terminal orientation, unless otherwise indicated. Polypeptides and polynucleotides can be made using routine techniques in the field of molecular biology. Furthermore, essentially any polypeptide or polynucleotide is available from commercial sources.

The terms "fusion protein" and "chimeric protein" as used herein refer to a single protein created by joining two or more proteins, protein domains, or protein fragments that do not naturally occur together in a single protein. For example, a fusion protein can contain a first domain from a Cas9 protein and a second domain from a Csy4 protein. The modification to include such domains in fusion proteins may confer additional activity on the modified Cas proteins. Such activities can include nuclease activity, methyltransferase activity, demethylase activity, DNA repair activity, DNA damage activity, deamination activity, dismutase activity, alkylation activity, depurination activity, oxidation activity, pyrimidine dimer forming activity, integrase activity, transposase activity, recombinase activity, polymerase activity, ligase activity, helicase activity, photolyase activity, glycosylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, and/or myristoylation activity or demyristoylation activity that modifies a polypeptide associated with nucleic acid target sequence (*e.g*., a histone).

A fusion protein can also comprise epitope tags (*e.g*., histidine tags, FLAG^{®} (Sigma Aldrich, St. Louis, MO) tags, Myc tags), reporter protein sequences (*e.g*., glutathione-S-transferase, beta-galactosidase, luciferase, green fluorescent protein, cyan fluorescent protein, yellow fluorescent protein), and/or nucleic acid sequence binding domains (*e.g*., a DNA binding domain or a RNA binding domain). A fusion protein can comprise at least one nuclear localization sequence (NLS), such as a simian virus 40 (SV40) NLS or a nucleoplamin NLS. A fusion protein can also comprise activator domains (*e.g*., heat shock transcription factors, NFKB activators) or repressor domains (*e.g.,* a KRAB domain). As described by Lupo et al. (Current Genomics, 2013, 14(4):268-278), the KRAB domain is a potent transcriptional repression module and is located in the amino-terminal sequence of most C2H2 zinc finger proteins. *See, e.g.,* Margolin et al. (PNAS, 1994, 91:4509-4513); and Witzgall et al. (PNAS, 1994, 91:4514-4518 (1994). The KRAB domain typically binds to co-repressor proteins and/or transcription factors via protein-protein interactions, causing transcriptional repression of genes to which KRAB zinc finger proteins (KRAB-ZFPs) bind. See, *e.g.,* Friedman et al. (Genes & Development, 1996, 10:2067-2678). In some embodiments, linker nucleic acid sequences are used to join the two or more proteins, protein domains, or protein fragments.

A "moiety" as used herein refers to a portion of a molecule. A moiety can be a functional group or describe a portion of a molecule with multiple functional groups (*e.g*., that share common structural aspects). The terms "moiety" and "functional group" are typically used interchangeably; however, a "functional group" can more specifically refer to a portion of a molecule that comprises some common chemical behavior. "Moiety" is often used as a structural description. In some embodiments, a 5' terminus, a 3' terminus, or a 5' terminus and a 3' terminus (*e.g*., a non-native 5' terminus and/or a non-native 3' terminus in a first stem element) can comprise one or more moieties.

The term "affinity tag" as used herein typically refers to one or more moieties that increases the binding affinity of a guide to a Cas protein, for example, to facilitate formation of a Cas9-guide/Cas9 protein nucleoprotein complex. In some embodiments, an affinity tag can be used to increase the binding affinity of a first polynucleotide of a Cas9-guide for a second polynucleotide of a Cas9-guide. Some embodiments of the present invention use an "affinity sequence," which is a polynucleotide sequence comprising one or more affinity tags. Some embodiments of the present invention introduce one or more affinity tags to the N-terminal of a Cas protein sequence (*e.g*., a Cas9 protein sequence), to the C-terminal of a Cas protein sequence, to a position located between the N-terminal and C-terminal of a Cas protein sequence, or to combinations thereof.

The terms "modified protein," "mutated protein," "protein variant," and "engineering protein" as used herein typically refers to a protein that has been modified such that it comprises a non-native sequence (*i.e.,* the modified protein has a unique sequence compared to an unmodified protein).

The terms "gene therapy" and "cell therapy" as used herein refer to the treatment of a disease utilizing cells that are genetically modified. Genetic modifications can be introduced into the cell using, for example, viral vectors, nucleofection, gene gun, sonoporation, cell squeezing, lipofection, or chemicals (*e.g*., cell penetrating peptides).

The terms "subject," "individual," or "patient" are used interchangeably herein and refer to any member of the phylum Chordata, including, without limitation, humans and other primates, including non-human primates, such as rhesus macaques, chimpanzees, and other monkey and ape species; farm animals, such as cattle, sheep, pigs, goats, and horses; domestic mammals, such as dogs and cats; laboratory animals, including rabbits, mice, rats, and guinea pigs; birds, including domestic, wild, and game birds, such as chickens, turkeys, and other gallinaceous birds, ducks, and geese; and the like. The term does not denote a particular age or gender. Thus, the term includes adult, young, and newborn individuals as well as males and females. In some embodiments, a host cell is derived from a subject (for example, lymphocytes, stem cells, progenitor cells, or tissue-specific cells). In some embodiments, the subject is a non-human subject.

The terms "effective amount" or "therapeutically effective amount" of a composition or agent, such as a genetically engineered adoptive cell as provided herein, refer to a sufficient amount of the composition or agent to provide the desired response. Preferably, the effective amount will prevent, avoid, or eliminate one or more harmful side-effects. Such responses will depend on the particular disease in question. For example, in a patient being treated for cancer using an adoptive cell therapy, a desired response includes, but is not limited to, treatment or prevention of the effects of graft versus host disease (GvHD), host versus graft rejection, cytokine release syndrome (CRS), cytokine storm, and the reduction of oncogenic transformations of administered genetically modified cells. The exact treatment amount required will vary from subject to subject, depending on the species, age, and general condition of the subject, the severity of the condition being treated, and the particular modified lymphocyte used, mode of administration, and the like. An appropriate "effective" amount in any individual case may be determined by one of ordinary skill in the art using routine experimentation.

"Treatment" or "treating" a particular disease, such as cancerous condition or GvHD includes: (1) preventing the disease, for example, preventing the development of the disease or causing the disease to occur with less intensity in a subject that may be predisposed to the disease, but does not yet experience or display symptoms of the disease; (2) inhibiting the disease, for example, reducing the rate of development, arresting the development or reversing the disease state; and/or (3) relieving symptoms of the disease, for example, decreasing the number of symptoms experienced by the subject.

By "gene editing" or "genome editing" as used herein is meant a type of genetic engineering that results in a genetic modification, such as an insertion, deletion, or replacement of a nucleotide sequence, or even a single base, at a specific site in a cell genome. The terms include, without limitation, heterologous gene expression, gene or promoter insertion or deletion, nucleic acid mutation, and a disruptive genetic modification, as defined herein.

"Transformation" as used herein refers to the insertion of an exogenous polynucleotide into a host cell, irrespective of the method used for insertion. For example, transformation can be by direct uptake, transfection, infection, and the like. The exogenous polynucleotide may be maintained as a nonintegrated vector, for example, an episome, or, alternatively, may be integrated into the host genome.

A "host cell" is a cell that has been transformed, or is capable of transformation, by an exogenous DNA sequence. A host cell can originate from any organism having one or more cells. Examples of host cells include, but are not limited to, a prokaryotic cell, a eukaryotic cell, a bacterial cell, an archaeal cell, a cell of a single-cell eukaryotic organism, a protozoa cell, a cell from a plant, an algal cell, a fungal cell (*e.g.,* a yeast cell, a cell from a mushroom), an animal cell, a cell from an invertebrate animal, a cell from a vertebrate animal, such as a cell from a mammal (*e.g.,* a pig, a cow, a goat, a sheep, a rodent, a rat, a mouse, a non-human primate, a human, *etc*.). Furthermore, a host cell can be a stem cell or progenitor cell, or a cell of the immune system, such as any of the cells of the immune system described herein. The host cell can be a human cell. For example, host cells can be lymphocytes or stem cells, such as hematopoietic stem cells. Lymphocytes include T cells for cell-mediated, cytotoxic adaptive immunity, such as CD4+ and/or CD8+ cytotoxic T cells; natural killer (NK) cells that function in cell-mediated, cytotoxic innate immunity; and B cells for humoral, antibody-driven adaptive immunity. Also included are hematopoietic stem cells that give rise to lymphoid cells. Additionally, CAR-T cells, T-cell receptor (TCR) cells, including TCR-engineered CAR-T cells, tumor infiltrating lymphocytes (TILs), CAR TILs, CAR-NK cells, and the like, can be modified using the techniques herein. In some embodiments, the human cell is outside of the human body. In some embodiments, cells of a body of a living organism (*e.g.,* a human body) are manipulated *ex vivo* (*i.e.,* outside of the living body). *Ex vivo* often refers to a medical procedure in which an organ, cells, or tissue are taken from a living body (*e.g.,* a human body) for a treatment or procedure, and then returned to the living body. *In vivo* often refers to a medical procedure in which an organ, cells, or tissue within a living body (*e.g.,* a human body) are subject to a treatment or procedure.

### CABRNTs

Embodiments of the disclosed CABRNT polynucleotides and guides include at least one abasic site that is designed into the polynucleotide. The abasic site or nucleotide is an apurinic/apyrimidinic site that does not comprise a nitrogenous base. In certain embodiments the abasic site is a ribose site that has a 2' hydroxyl group. In other embodiments, the abasic site is a deoxyribose site that does not include a 2' hydroxyl group. In yet further embodiments, the abasic site is a modified nucleotide backbone, such as a pentose sugar with a 1' hydroxyl group. Examples of available abasic nucleotides can be found at idtdna.com/pages/education/decoded/article/insert-an-abasic-site-into-your-sequence.

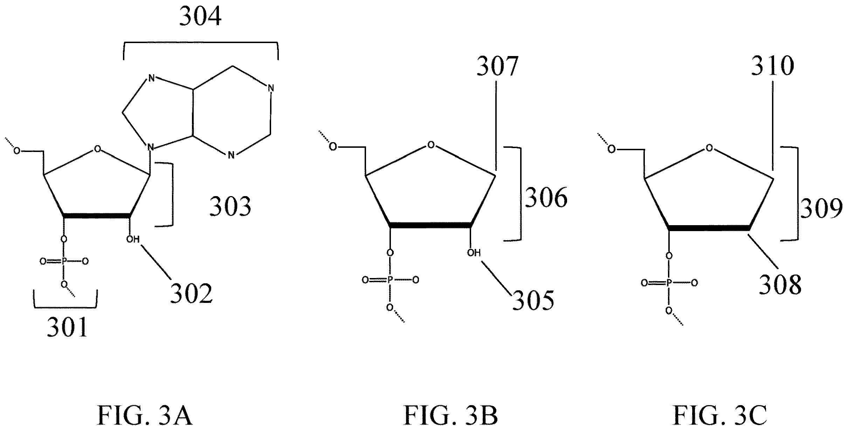

FIG. 3A illustrates a RNA nucleotide, wherein the nucleotide comprises a 3' phosphate group (FIG. 3A, 301), which is connected to the 5' carbon of the next nucleotide in a polynucleotide chain. The RNA nucleotide in FIG. 3A comprises a 2' hydroxyl group (FIG. 3A, 302), a pentose sugar (FIG. 3A, 303), and a nitrogen base (FIG. 3A, 304). The nitrogen base (FIG. 3A, 304) is capable of forming hydrogen base pair bonding with a complementary nitrogen base of a DNA or RNA nucleotide. FIG. 3A shows a RNA nucleotide that could be a RNA nucleotide of a CRISPR polynucleotide.

FIG. 3B depicts an abasic ribose site that could be an abasic site of a CABRNT guide. The abasic ribose site comprises a 2' hydroxyl group (FIG. 3B, 305) and a pentose sugar (FIG. 3B, 306). The abasic ribose site does not comprise a nitrogen base (FIG. 3B, 307). The abasic ribose site shown in FIG. 3B cannot form hydrogen base pair bonding with a complementary nitrogen base of a DNA or RNA nucleotide, because it does not comprise a nitrogen base (FIG. 3B, 307).

FIG. 3C depicts an abasic deoxyribose site that could be an abasic site of a CABRNT guide. The abasic deoxyribose site comprises a pentose sugar (FIG. 3C, 309). The abasic deoxyribose site does not comprise 2' hydroxyl (FIG. 3C, 308) or a nitrogen base (FIG. 3C, 310). The abasic deoxyribose site shown in FIG. 3C cannot form hydrogen base pair bonding with a complementary nitrogen base of a DNA or RNA nucleotide, because it does not comprise a nitrogen base (FIG. 3C, 310).

### CAVAs

Embodiments of the disclosed CAVA polynucleotides and guides include at least one base analog designed into the polynucleotide. In certain embodiments, the base analog is selected from the group consisting of inosine, deoxyinosine, and deoxyuradine.

### CABRNT-CAVAs

Embodiments of the disclosed CABRNT-CAVA polynucleotides and guides include at least one abasic site and at least one base analog designed into the polynucleotide. The abasic site or nucleotide is an apurinic/apyrimidinic site that does not comprise a nitrogenous base. In certain embodiments the abasic site is a ribose site that has a 2' hydroxyl group. In other embodiments, the abasic site is a deoxyribose site that does not include a 2' hydroxyl group. In yet further embodiments, the abasic site is a modified nucleotide backbone, such as a pentose sugar with a 1' hydroxyl group. Examples of available abasic nucleotides can be found at idtdna.com/pages/education/decoded/article/insert-an-abasic-site-into-your-sequence. In certain embodiments, the base analog is selected from the group consisting of inosine, deoxyinosine and deoxyuradine.

The disclosed CABRNT, CAVA, and CABRNT-CAVA polynucleotides and guides are capable for use in any of the CRISPR-Cas systems described herein, but are preferably used in Class 1 Type I, Class 2 Type II, and Class 2 Type V CRISPR-Cas systems. The CABRNT, CAVA, and CABRNT-CAVA polynucleotides and guides of the present disclosure cannot be made *in vivo* using expression vectors, but can be synthesized *in vitro,* such as by chemical synthesis. *See, e.g.,* Example 2.

The disclosed CABRNT polynucleotides and guides comprise a combination of nucleotide bases and one or more abasic sites. In some embodiments, the CABRNT polynucleotide or guide includes a CRISPR polynucleotide containing a spacer sequence, wherein one or more abasic sites have been designed into the polynucleotide sequence. In other embodiments, the one or more abasic sites have been designed into the spacer sequence. In other embodiments, the CABRNT polynucleotide or guide includes a tracr polynucleotide, either as a single guide or dual guide, and one or more abasic sites may be designed into the tracr polynucleotide.

Embodiments of the disclosure also relate to CABRNT guides that comprise one or more abasic sites. The CABRNT guides are capable of forming a nucleoprotein complex with a Cas protein and, upon formation of the nucleoprotein complex, the nucleoprotein complex is capable of binding a target nucleic acid sequence complementary to the guide spacer sequence.

The disclosed CABRNT guides correspond to a single molecule polynucleotide or a dual molecule polynucleotide, preferably single guide or dual guide, wherein one or more abasic sites has been designed into the guide. The abasic sites can be designed into an internal site of the guide or at the 5' or 3' end of the guide. The abasic site is preferably located in the spacer sequence of the guide.

The disclosed CABRNT polynucleotides and guides can comprise RNA nucleotides, DNA nucleotides, or a mixture thereof, where the CABRNT abasic site is designed into a desired location, preferably the spacer sequence. In some embodiments, the CABRNT polynucleotide or guide comprises a chRDNA (a polynucleotide comprising a spacer, wherein the polynucleotide comprises RNA with DNA designed into the polynucleotide) and/or a ch-acr (a polynucleotide comprising a nexus and a 3' hairpin, wherein the polynucleotide comprises RNA with DNA designed into the polynucleotide). *See, e.g.,* Example 8, illustrating the design and use of guides comprising RNA and DNA (chRDNAs) and an abasic site (CABRNT).

The disclosed CAVA polynucleotides and guides comprise a combination of nucleotide bases and one or more base analogs. In some embodiments, the CAVA polynucleotide or guide includes a CRISPR polynucleotide containing a spacer sequence, wherein one or more base analogs have been designed into the polynucleotide sequence. In other embodiments, the one or more base analogs have been designed into the spacer sequence. In other embodiments, the CAVA polynucleotide or guide includes a tracr polynucleotide, either as a single guide or dual guide, and one or more base analogs may be designed into the tracr polynucleotide.

Embodiments of the disclosure also relate to CAVA guides that comprise one or more base analogs. The CAVA guides are capable of forming a nucleoprotein complex with a Cas protein and, upon formation of the nucleoprotein complex, the nucleoprotein complex is capable of binding a target nucleic acid sequence complementary to the guide spacer sequence.

The disclosed CAVA guides correspond to a single molecule polynucleotide or a dual molecule polynucleotide, preferably single guide or dual guide, wherein one or more base analogs have been designed into the guide. The one or more base analogs can be designed into an internal site of the guide or at the 5' or 3' end of the guide. The one or more base analogs are preferably located in the spacer sequence of the guide.

The disclosed CAVA polynucleotides and guides can comprise RNA nucleotides, DNA nucleotides, or a mixture thereof, where the one or more base analogs are designed into a desired location, preferably the spacer sequence. In some embodiments, the CAVA polynucleotide or guide comprises a chRDNA (a polynucleotide comprising a spacer, wherein the polynucleotide comprises RNA with DNA designed into the polynucleotide) and/or a ch-acr (a polynucleotide comprising a nexus and a 3' hairpin, wherein the polynucleotide comprises RNA with DNA designed into the polynucleotide).

The disclosed CABRNT-CAVA polynucleotides and guides can comprise a combination of nucleotide bases, one or more abasic sites, and one or more base analogs. In some embodiments, the CABRNT-CAVA polynucleotide or guide includes a CRISPR polynucleotide containing a spacer sequence, wherein one or more abasic sites and one or more base analogs have been designed into the polynucleotide sequence. In other embodiments, the one or more abasic sites and the one or more base analogs have been designed into the spacer sequence. In other embodiments, the CABRNT-CAVA polynucleotide or guide includes a tracr polynucleotide, either as a single guide or dual guide, and one or more abasic sites and one or more base analogs may be designed into the tracr polynucleotide.

Embodiments of the disclosure also relate to CABRNT-CAVA guides that comprise one or more abasic sites and one or more base analogs. The CABRNT-CAVA guides are capable of forming a nucleoprotein complex with a Cas protein and, upon formation of the nucleoprotein complex, the nucleoprotein complex is capable of binding a target nucleic acid sequence complementary to the guide spacer sequence.

The disclosed CABRNT-CAVA guides correspond to a single molecule polynucleotide or a dual molecule polynucleotide, preferably single guide or dual guide, wherein one or more abasic sites and one or more base analogs have been designed into the guide. The one or more abasic sites and the one or more base analogs can be designed into an internal site of the guide or at the 5' or 3' end of the guide. The one or more abasic sites and the one or more base analogs are preferably located in the spacer sequence of the guide.

The disclosed CABRNT-CAVA polynucleotides and guides can comprise RNA nucleotides, DNA nucleotides, or a mixture thereof, where the one or more abasic sites and the one or more base analogs are designed into a desired location, preferably the spacer sequence. In some embodiments, the CABRNT-CAVA polynucleotide or guide comprises a chRDNA (a polynucleotide comprising a spacer, wherein the polynucleotide comprises RNA with DNA designed into the polynucleotide) and/or a ch-acr (a polynucleotide comprising a nexus and a 3' hairpin, wherein the polynucleotide comprises RNA with DNA designed into the polynucleotide).

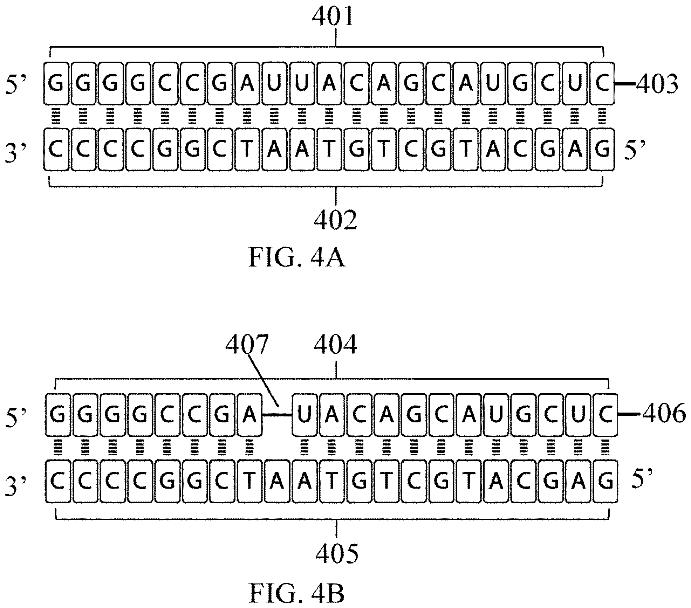

FIG. 4A illustrates hydrogen base pair bonding between a CRISPR spacer (FIG. 4A, 401) and target polynucleotide (FIG. 4A, 402), wherein all the nucleotides of the CRISPR spacer (FIG. 4A, 401) form hydrogen base bonds (dashed lines) with the nucleotides of the target polynucleotide (FIG. 4A, 402). The guide backbone (FIG. 4A, 403) can be upstream (*e.g*., covalently connected to the 5' end) of the spacer or downstream (*e.g*., covalently connected to the 3' end) of the spacer, depending on the CRISPR class and type.

FIG. 4B illustrates hydrogen base pair bonding between a CABRNT spacer (FIG. 4B, 404) with an abasic site (FIG. 4B, 407) and a target polynucleotide (FIG. 4B, 405), wherein the abasic site (FIG. 4B, 407) is not capable of forming a hydrogen base pair bond (dashed lines) with the nucleotide of the target polynucleotide (FIG. 4B, 405). The guide backbone (FIG. 4B, 406) can be upstream (*e.g*., covalently connected to the 5' end) of the spacer or downstream (*e.g*., covalently connected to the 3' end) of the spacer, depending on the CRISPR class and type. FIG. 4B depicts one embodiment of a CABRNT guide that includes a single internal abasic site; however, the disclosed CABRNT guides can include an abasic site at any position in the polynucleotide sequence, such as any location in the spacer sequence, and can include more than one abasic site.

The CABRNT, CAVA, and CABRNT-CAVA guides of the present invention can be used with any of the Cas proteins and CRISPR-Cas systems described herein by designing guides having one or more abasic sites, and/or one or more base analogs, for use with the particular system.

One or more abasic sites, and/or one or more base analogs, can be designed into any portion of a guide. In some examples, the spacer sequence includes one or more abasic sites. As demonstrated in Examples 6 and 7, the type of abasic site and the location of the abasic site can impact functionality of a nucleoprotein complex comprising a CABRNT guide and a Cas protein. Example 6 illustrates the on-target editing efficiency of CABRNT guides with a ribose abasic site designed into the spacer sequence and CABRNT guides with a deoxyribose abasic site designed into the spacer sequence. Example 7 illustrates differences in on-target editing and off-target editing between CABRNT guides with abasic sites at different positions of a spacer sequence.

Methods of designing particular guides into which abasic sites, and/or base analogs, can be designed are known. *See, e.g.,* Briner et al. ("Guide RNA Functional Modules Direct Cas9 Activity and Orthogonality," Molecular Cell, 2014, 56:333-339). To do so, the genomic sequence for the gene to be targeted is first identified. The exact region of the selected gene to target will depend on the specific application. For example, in order to activate or repress a target gene using, for example, CRISPR activation or CRISPR inhibition, cognate guide/Cas protein complexes can be targeted to the promoter driving expression of the gene of interest. For genetic knockouts, guides are commonly designed to target 5' constitutively expressed exons, which reduces the chances or removal of the targeted region from mRNA due to alternative splicing. Exons near the N-terminus can be targeted because frameshift mutations here will increase the likelihood of the production of a nonfunctional protein product. Alternatively, cognate guides can be designed to target exons that code for known essential protein domains. In this regard, non-frameshift mutations such as insertions or deletions are more likely to alter protein function when they occur in protein domains that are essential for protein function. For gene editing using HDR, the target sequence should be close to the location of the desired edit. In this case, the location where the edit is desired is identified and a target sequence is selected nearby. Example 4 describes designing CABRNT guides to target the programmed cell death 1 (PDCD1) gene in human T cells.

The present invention found that the location, number, and identity of the abasic sites, or of the base analogs, in a guide can unexpectedly impact on-target editing and off-target editing of a target sequence. The Examples herein provide non-limiting examples of CABRNT guides containing abasic sites that demonstrate these effects. In these non-limiting examples, the CABRNT guides contain differing abasic sites in varying locations of their spacer sequence. The location of the abasic sites in the spacer sequence can impact both on-target editing and/or off-target editing. Example 6 demonstrates that varying abasic ribose sites and abasic deoxyribose sites in CABRNT/Cas9 nucleoprotein complexes can impact on-target editing. Example 7 demonstrates that the position of the abasic site in the CABRNT guide can impact on-target editing and off-target editing. Example 8 demonstrates that hybrid CABRNT guides, containing both DNA and RNA in combination with an abasic site, can impact both on-target editing and off-target editing. Following the guidance of the present specification, the design and validation of CABRNT guides can be practiced by one of ordinary skill in the art.

The Examples herein also provide non-limiting examples of CAVA guides containing base analogs that demonstrate these effects. In these non-limiting examples, the CAVA guides contain differing base analogs at varying locations in their spacer sequence. The location of the base analogs in the spacer sequence can impact off-target editing. Example 10 demonstrates that guides comprising inosine, deoxyinosine and deoxyuradine in CAVA/Cas9 nucleoprotein complexes can impact off-target editing. Following the guidance of the present specification, the design and validation of CAVA guides can be practiced by one of ordinary skill in the art.

In some embodiments, the disclosed CABRNT, CAVA, and CABRNT-CAVA guides are capable of interacting with a Cas protein (*e.g*., Cas9), thereby forming a nucleoprotein complex. The CABRNT, CAVA, or CABRNT-CAVA guide can guide the Cas protein to a target nucleic acid. The binding specificity is determined jointly by the complementary region on the CABRNT, CAVA, or CABRNT-CAVA guide and a short DNA motif (protospacer adjacent motif or PAM) juxtaposed to the complementary region. The spacer present in the CABRNT, CAVA, or CABRNT-CAVA guide specifically hybridizes to a target nucleic acid sequence and determines the location of a Cas protein's site-specific binding and nucleolytic cleavage.

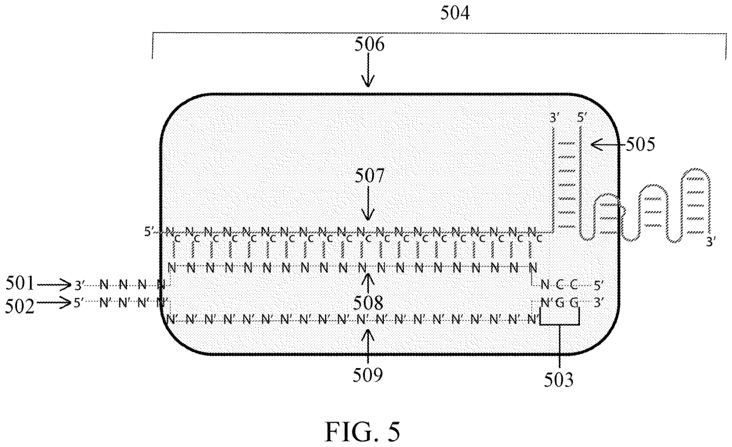

FIG. 5 illustrates a CRISPR/Cas9 nucleoprotein complex (FIG. 5, 504), wherein the double-stranded nucleic acid comprising the target sequence is unwound, and the target binding sequence of the Cas9 guide (FIG. 5, 507) is connected via hydrogen bonds (FIG. 5, indicated by a vertical line between polynucleotides) to the target sequence (FIG. 5, 508). In FIG. 5, nucleotides of the non-target strand (FIG. 5, 502) comprising the target complement sequence (FIG. 5, 509) and the PAM sequence (5'-NGG-3'; FIG. 5, 503) are designated as N', nucleotides of the target strand (FIG. 5, 501) comprising the target sequence (FIG. 5, 508) are designated as N, and nucleotides of the guide comprising the target binding sequence (FIG. 5, 507) are designated as Nc. In FIG. 5, the Cas9 nucleoprotein complex (FIG. 5, 504) comprises the Cas9 guide (FIG. 5, 505) and the Cas9 protein (FIG. 5, 506).

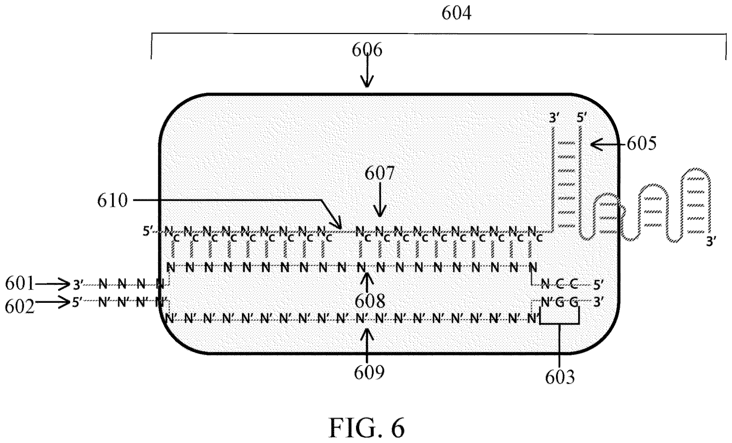

FIG. 6 illustrates a CABRNT/Cas9 nucleoprotein complex (FIG. 6, 604), wherein the double-stranded nucleic acid comprising the target sequence is unwound, and the target binding sequence (FIG. 6, 607) of the Cas9 guide comprises an abasic site (FIG. 6, 610) and is connected via hydrogen bonds (FIG. 6, indicated by a vertical line between polynucleotides) to the target sequence (FIG. 6, 608). In FIG. 6, nucleotides of the non-target strand (FIG. 6, 602) comprising the target complement sequence (FIG. 6, 609) and the PAM sequence (5'-NGG-3'; FIG. 6, 603) are designated as N', nucleotides of the target strand (FIG. 6, 601) comprising the target sequence (FIG. 6, 608) are designated as N, and nucleotides of the guide polynucleotide comprising the target binding sequence (FIG. 6, 607) are designated as N_{c}. In FIG. 6, the abasic site (FIG. 6, 610) on the target binding sequence (FIG. 6, 607) does not form hydrogen base pair bonds with the target sequence (FIG. 6, 608). The Cas9 nucleoprotein complex (FIG. 6, 604) comprises the CABRNT guide (FIG. 6, 605) and the Cas9 protein (FIG. 6, 606).

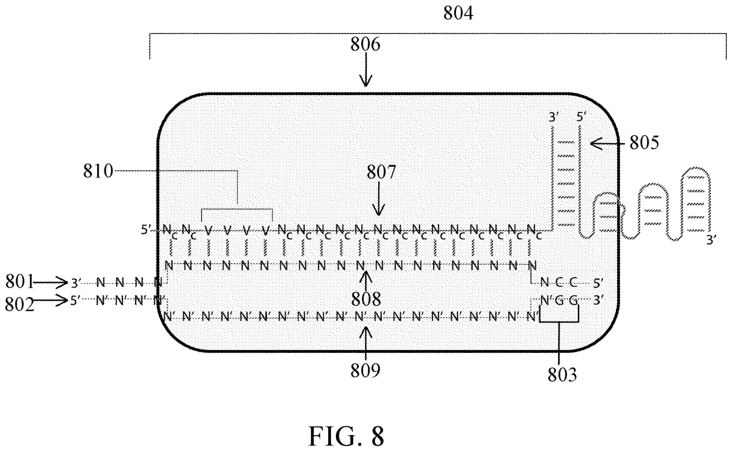

FIG. 8 illustrates a CAVA/Cas9 nucleoprotein complex (FIG. 8, 804), wherein the double-stranded nucleic acid comprising the target sequence is unwound, and the target binding sequence (FIG. 8, 807) of the Cas9 guide comprises one of more analogs (FIG. 8, 810) and is connected via hydrogen bonds (FIG. 8, indicated by a vertical line between polynucleotides) to the target sequence (FIG. 8, 808). In FIG. 8, nucleotides of the non-target strand (FIG. 8, 802) comprising the target complement sequence (FIG. 8, 809) and the PAM sequence (5'-NGG-3'; FIG. 8, 803) are designated as N', nucleotides of the target strand (FIG. 8, 801) comprising the target sequence (FIG. 8, 808) are designated as N, and nucleotides of the guide polynucleotide comprising the target binding sequence (FIG. 8, 807) are designated as N_{c}. In FIG. 8, the nucleic acid analogs (FIG. 8, 810), designated as V, on the target binding sequence (FIG. 8, 807) is capable of forming hydrogen base pair bonds with the target sequence (FIG. 8, 808). The Cas9 nucleoprotein complex (FIG. 8, 804) comprises the CAVA guide (FIG. 8, 805) and the Cas8 protein (FIG. 8, 806).

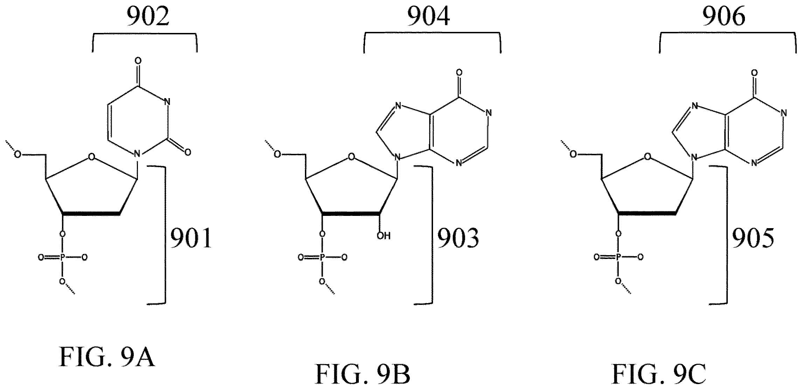

FIG. 9A illustrates a CAVA nucleotide, wherein the CAVA nucleotide comprises a deoxyribose sugar (FIG. 9A, 901) and a uracil base (FIG. 9A, 902). The uracil base (FIG. 9A, 902) is capable of forming hydrogen base pair bonding with a complementary nitrogen base of a DNA or RNA nucleotide.

FIG. 9B illustrates a CAVA nucleotide, wherein the CAVA nucleotide comprises a ribose sugar (FIG. 9B, 902) and an inosine base (FIG. 9B, 904). The inosine base (FIG. 9B, 902) is capable of forming hydrogen base pair bonding with the nitrogen base of a DNA or RNA nucleotide.

FIG. 9C illustrates a CAVA nucleotide, wherein the CAVA nucleotide comprises a deoxyribose sugar (FIG. 9C, 905) and an inosine base (FIG. 9C, 906). The inosine base (FIG. 9C, 906) is capable of forming hydrogen base pair bonding with the nitrogen base of a DNA or RNA nucleotide.

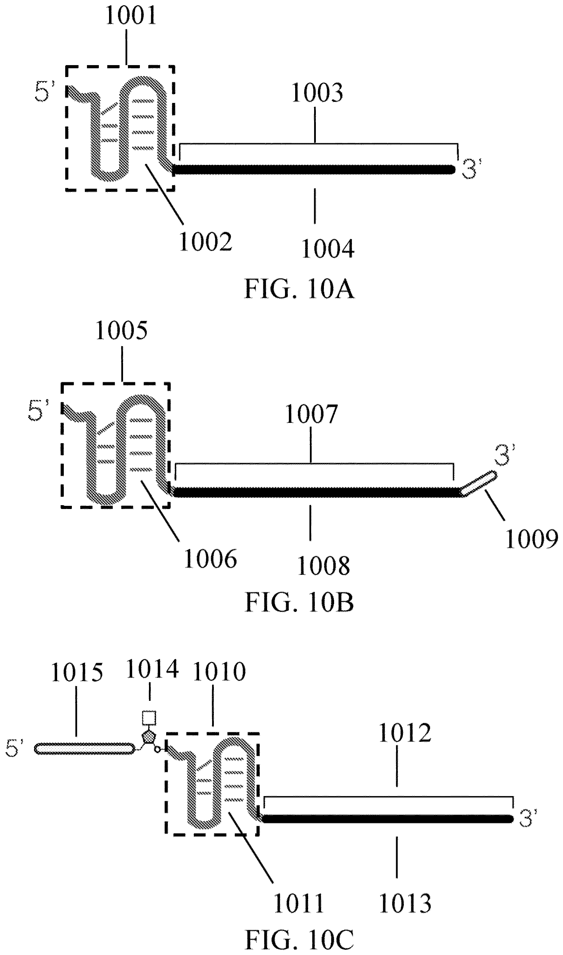

FIG. 10A illustrates an example of an *Acidaminococcus spp.* BV316 Cas12a guide molecule comprising the following: an activating region (FIG. 10A, 1001), comprising a stem-loop duplex (FIG. 10A, 1002); and a spacer sequence (FIG. 10A, 1003), comprising a target binding sequence (FIG. 10A, 1004). FIG. 10B illustrates an alternative Cas12a guide molecule comprising the following: an activating region (FIG. 10B, 1005), comprising a stem-loop duplex (FIG. 10B, 1006); and a spacer sequence (FIG. 10B, 1007), comprising a target binding sequence (FIG. 10B, 1008) and a 3' extension (FIG. 10B, 1009). The 3' extension (FIG. 10B, 1009) can be connected to the spacer sequence (FIG. 10B, 1007) via a linker sequence. FIG. 10C illustrates an alternative Cas12a guide molecule comprising the following: an activating region (FIG. 10C, 1010), comprising a stem-loop duplex (FIG. 10C, 1011) and a linker nucleotide (FIG. 10C, 1014) and a 5' extension (FIG. 10C, 1015); and a spacer sequence (FIG. 10C, 1012), comprising a target binding sequence (FIG. 10C, 1013).

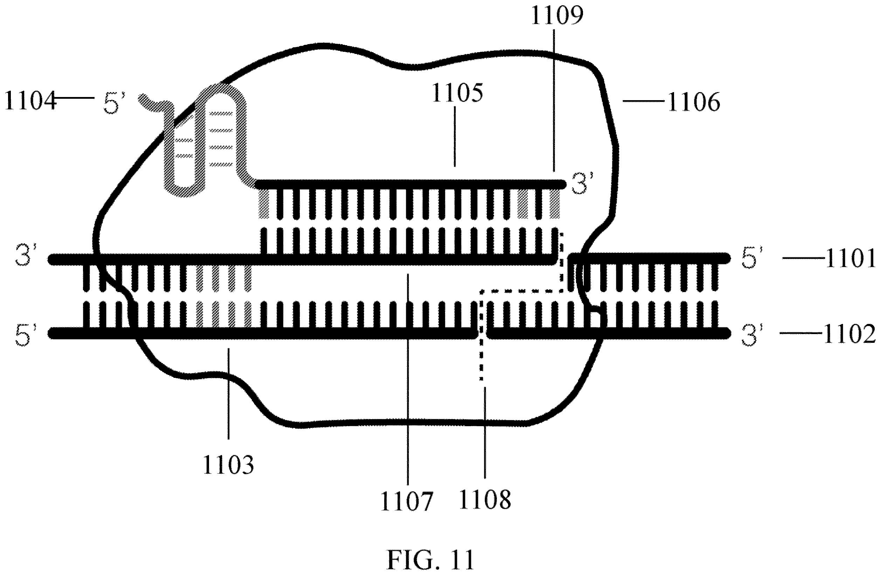

FIG. 11 illustrates a Cas12a protein (FIG. 11, 1106) bound to a cognate CAVA guide molecule (FIG. 11, 1104) comprising a target binding sequence (FIG. 11, 1105), wherein the target binding sequence (FIG. 11, 1105) comprises a base analog (FIG. 11, 1109). The Cas12a chRDNA guide/nucleoprotein complex unwinds a target polynucleotide comprising the target sequence, and the target binding sequence of the Cas12 chRDNA guide molecule (FIG. 11, 1105) is connected via hydrogen bonds (FIG. 11, indicated by a vertical line between polynucleotides) to the target sequence (FIG. 11, 1107). In FIG. 11, the target polynucleotide comprises a target strand (FIG. 11, 1101) comprising the target sequence (FIG. 11, 1107), and a non-target strand (FIG. 11, 1102) comprising a PAM sequence (FIG. 11, 1103). The PAM sequence (FIG. 11, 1103) typically occurs upstream (*i.e.,* in a 5' direction) of the target sequence (FIG. 11, 1107) on the non-target strand (FIG. 11, 1102). Formation of hydrogen bonds between the target binding sequence of the CAVA guide molecule (FIG. 11, 1105) and the target sequence (FIG. 11, 1107) result in the staggered cleavage (FIG. 11, 1108) of the target strand (FIG. 11, 1101) and the non-target strand (FIG. 11, 1102).

The CABRNT, CAVA, and CABRNT-CAVA guides can be designed so as to interact and form a nucleoprotein complex with any Cas protein. In some embodiments, the CABRNT, CAVA, and CABRNT-CAVA guides are capable of forming nucleoprotein complexes with a Cas9 protein. In other embodiments, the CABRNT, CAVA, and CABRNT-CAVA guides are capable of forming nucleoprotein complexes with a Cas 12 protein. In other embodiments, the CABRNT, CAVA, and CABRNT-CAVA guides are capable of forming nucleoprotein complexes with one or more subunit protein from a Cascade effector complex (*e.g.,* Cas5, Cas6, Cas7, Cas8, Cas11).

In some embodiments, a CABRNT, CAVA, or CABRNT-CAVA guide can be designed such that the nucleoprotein complex can bind outside of the cleavage site of the Cas protein. In this case, the target nucleic acid may not interact with the complex and the target nucleic acid can be excised (*e.g*., free from the complex). In some embodiments, a CABRNT, CAVA, or CABRNT-CAVA guide can be designed such that the complex can bind inside of the cleavage site of the Cas protein. In this case, the target nucleic acid can interact with the complex and the target nucleic acid can be bound (*e.g*., bound to the complex).

The CABRNT, CAVA, or CABRNT-CAVA guide can be designed in such a way that the complex can hybridize to a plurality of locations within a nucleic acid sample. A plurality of complexes can be contacted to a nucleic acid sample. The plurality of complexes can comprise CABRNT, CAVA, and/or CABRNT-CAVA guides designed to hybridize to the same sequence. The plurality of complexes can comprise CABRNT, CAVA, and/or CABRNT-CAVA guides designed to hybridize to the different sequences.

The sequences can be at different locations within a target nucleic acid. The locations can comprise the same, or similar, target nucleic acid sequences. The locations can comprise different target nucleic acid sequences. The locations can be a defined distance from each other. The locations can be less than 10 kilobases (Kb) apart, less than 8 Kb apart, less than 6 Kb apart, less than 4 Kb apart, less than 2 Kb apart, less than 1 Kb apart, less than 900 nucleotides apart, less than 800 nucleotides apart, less than 700 nucleotides apart, less than 600 nucleotides apart, less than 500 nucleotides apart, less than 400 nucleotides apart, less than 300 nucleotides apart, less than 200 nucleotides apart, less than 100 nucleotides apart.

The complexes can cleave the target nucleic acid, which can result in an excised target nucleic acid that can be less than 10 kilobases (Kb) long, less than 8 Kb long, less than 6 Kb long, less than 4 Kb long, less than 2 Kb long, less than 1 Kb long, less than 900 nucleotides long, less than 800 nucleotides long, less than 700 nucleotides long, less than 600 nucleotides long, less than 500 nucleotides long, less than 400 nucleotides long, less than 300 nucleotides long, less than 200 nucleotides long, less than 100 nucleotides long.

The complexes can be bound to a fragmented target nucleic acid that can be less than 10 kilobases (Kb) long, less than 8 Kb long, less than 6 Kb long, less than 4 Kb long, less than 2 Kb long, less than 1 Kb long, less than 900 nucleotides long, less than 800 nucleotides long, less than 700 nucleotides long, less than 600 nucleotides long, less than 500 nucleotides long, less than 400 nucleotides long, less than 300 nucleotides long, less than 200 nucleotides long, less than 100 nucleotides long.

The CABRNT, CAVA, and CABRNT-CAVA guides of the present disclosure comprising one or more abasic sites, and/or one or more base analogs, on the same strand can be synthesized *in vitro* by known methods, such as chemically in solution or on a solid support. Cognate CABRNT, CAVA, or CABRNT-CAVA guide/Cas protein complexes also can be produced using methods well known in the art. Cas protein components can be recombinantly produced and then the guides and Cas proteins can be complexed together using methods known in the art. *See, e.g.,* Example 2, which provides a non-limiting example of a method of assembling nucleoprotein complexes comprising a guide/Cas9 protein.

### Cas Proteins

Additionally, cell lines constitutively expressing Cas proteins can be developed and can be transfected with the guide components, and complexes can be purified from the cells using standard purification techniques, such as but not limited to affinity, ion exchange and size exclusion chromatography. *See, e.g.,* Jinek et al. ("A programmable dual-RNA-guided DNA endonuclease in adaptive bacterial immunity," Science, 2012, 337:816-821).

According to known methods, Cas proteins can be produced using expression cassettes encoding a Cas protein. Expression cassettes typically comprise regulatory sequences functional in host cells into which they are introduced. Regulatory sequences are involved in one or more of the following: regulation of transcription, post-transcriptional regulation, and regulation of translation. Expression cassettes can be present in expression vectors and introduced into a wide variety of host cells, including bacterial cells, yeast cells, plant cells, and mammalian cells.

In one embodiment, the Cas proteins are produced in vectors, including expression vectors, comprising polynucleotides encoding the Cas proteins. Vectors useful for producing components for use in the present methods include plasmids, viruses (including phage), and integratable nucleic acid fragments (*i.e.,* fragments integratable into the host genome by homologous recombination). A vector replicates and functions independently of the host genome, or may, in some instances, integrate into the genome itself. Suitable replicating vectors will contain a replicon and control sequences derived from species compatible with the intended expression host cell. In some embodiments, polynucleotides encoding one or more of the various components are operably linked to an inducible promoter, a repressible promoter, or a constitutive promoter. Expression vectors can also include polynucleotides encoding protein tags (*e.g*., poly-His tags, hemagglutinin tags, fluorescent protein tags, bioluminescent tags, nuclear localization tags). The coding sequences for such protein tags can be fused to the coding sequences or can be included in an expression cassette, for example, in a targeting vector.

General methods for construction of expression vectors are known in the art. Expression vectors for most host cells are commercially available. There are several commercial software products designed to facilitate selection of appropriate vectors and construction thereof, such as insect cell vectors for insect cell transformation and gene expression in insect cells, bacterial plasmids for bacterial transformation and gene expression in bacterial cells, yeast plasmids for cell transformation and gene expression in yeast and other fungi, mammalian vectors for mammalian cell transformation and gene expression in mammalian cells or mammals, viral vectors (including retroviral, lentiviral, and adenoviral vectors) for cell transformation and gene expression and methods to easily enable cloning of such polynucleotides. SnapGene.TM. (GSL Biotech LLC, Chicago, Ill.; snapgene.com/resources/plasmid _files/your_time_is_valuable/), for example, provides an extensive list of vectors, individual vector sequences, and vector maps, as well as commercial sources for many of the vectors.

For example, the various components can be incorporated into mammalian vectors for use in mammalian cells. A large number of mammalian vectors suitable for use with the systems of the present invention are commercially available (*e.g*., from Life Technologies, Grand Island, NY; NeoBiolab, Cambridge, MA; Promega, Madison, WI; DNA2.0, Menlo Park, CA; Addgene, Cambridge, MA).

Vectors derived from mammalian viruses can also be used for expressing the Cas protein components of the present methods in mammalian cells. These include vectors derived from viruses such as adenovirus, adeno-associated virus, papovirus, herpesvirus, polyomavirus, cytomegalovirus, lentivirus, retrovirus, vaccinia and Simian Virus 40 (SV40). *See, e.g.,* Kaufman et al. (Molec. Biotech., 2000, 16: 151-160); and Cooray et al. (Methods Enzymol., 2012, 507:29-57). Regulatory sequences operably linked to the components can include activator binding sequences, enhancers, introns, polyadenylation recognition sequences, promoters, repressor binding sequences, stem-loop structures, translational initiation sequences, translation leader sequences, transcription termination sequences, translation termination sequences, primer binding sites, and the like. Commonly used promoters are constitutive mammalian promoters CMV, EF1a, SV40, PGK1 (mouse or human), Ubc, CAG, CaMKIIa, and beta-Act, and others known in the art (Khan, K. H. Advanced Pharmaceutical Bulletin (2013) 3:257-263). Furthermore, mammalian RNA polymerase III promoters, including H1 and U6, can be used.

Numerous mammalian cell lines have been utilized for expression of gene products including HEK 293 (human embryonic kidney) and CHO (Chinese hamster ovary). These cell lines can be transfected by standard methods (*e.g*., using calcium phosphate or polyethyleneimine (PEI), or electroporation). Other typical mammalian cell lines include, but are not limited to HeLa, U2OS, 549, HT1080, CAD, P19, NIH 3T3, L929, N2a, human embryonic kidney 293 cells, MCF-7, Y79, SO-Rb50, Hep G2, DUKX-X11, J558L, and baby hamster kidney (BHK) cells.

Vectors can be introduced into and propagated in a prokaryote. Prokaryotic vectors are well known in the art. Typically, a prokaryotic vector comprises an origin of replication suitable for the target host cell (*e.g.,* oriC derived from *E. coli,* pUC derived from pBR322, pSC101 derived from *Salmonella*), 15A origin (derived from p15A) and bacterial artificial chromosomes). Vectors can include a selectable marker (*e.g*., genes encoding resistance for ampicillin, chloramphenicol, gentamicin, and kanamycin). Zeocin.TM. (Life Technologies, Grand Island, NY) can be used as a selection in bacteria, fungi (including yeast), plants and mammalian cell lines. Accordingly, vectors can be designed that carry only one drug resistance gene for Zeocin for selection work in a number of organisms. Useful promoters are known for expression of proteins in prokaryotes, for example, T5, T7, Rhamnose (inducible), Arabinose (inducible), and PhoA (inducible). Furthermore, T7 promoters are widely used in vectors that also encode the T7 RNA polymerase. Prokaryotic vectors can also include ribosome binding sites of varying strength, and secretion signals (*e.g*., mal, sec, tat, ompC, and pelB). In addition, vectors can comprise RNA polymerase promoters for the expression of NATNAs. Prokaryotic RNA polymerase transcription termination sequences are also well known (*e.g*., transcription termination sequences from *S. pyogenes*).

Expression of proteins in prokaryotes is typically carried out in *E. coli* with vectors containing constitutive or inducible promoters directing the expression of either fusion or non-fusion proteins.

In some embodiments, a vector is a yeast expression vector. Examples of vectors for expression in *Saccharomyces cerivisiae* include, but are not limited to, the following: pYepSec1, pMFa, pJRY88, pYES2, and picZ. Methods for gene expression in yeast cells are known in the art (*see, e.g.,* Methods in Enzymology, Volume 194, "Guide to Yeast Genetics and Molecular and Cell Biology, Part A," (2004) Christine Guthrie and Gerald R. Fink (eds.), Elsevier Academic Press, San Diego, CA). Typically, expression of protein-encoding genes in yeast requires a promoter operably linked to a coding region of interest plus a transcriptional terminator. Various yeast promoters can be used to construct expression cassettes for expression of genes in yeast.

### Genomic Editing of Cells using CABRNT, CAVA, and CABRNT-CAVA Guide/Nucleoprotein Complexes

Delivery of CABRNT, CAVA, and CABRNT-CAVA guides and CABRNT, CAVA, or CABRNT-CAVA/Cas nucleoprotein complexes of the present disclosure to cells, *in vitro* or *in vivo,* may be achieved by a number of methods known to one of ordinary skill in the art. In some embodiments, CABRNT, CAVA, or CABRNT-CAVA/Cas nucleoprotein complexes can be directly introduced into cells. Non-limiting methods to introduce these components into a cell include microinjection, electroporation, nucleofection, lipofection, particle gun technology, and microprojectile bombardment.

In some embodiments, electroporation can be used to deliver the CABRNT, CAVA, and CABRNT-CAVA guides of the present disclosure. Electroporation may also be used to deliver complexes of a Cas protein and CABRNT, CAVA, or CABRNT-CAVA guides of the present disclosure. In these methods, the CABRNT, CAVA, and CABRNT-CAVA guides, or the complexes of Cas protein and CABRNT, CAVA, or CABRNT-CAVA guides are mixed in an electroporation buffer with the target cells to form a suspension. This suspension is then subjected to an electrical pulse at an optimized voltage, which creates temporary pores in the phospholipid bilayer of the cell membrane, permitting charged molecules like nucleic acids and proteins to be driven through the pores and into the cell. Reagents and equipment to perform electroporation are sold commercially.

Example 3 illustrates nucleofection of activated T cells with guide/Cas9 protein nucleoprotein complexes.

In another embodiment, the present disclosure provides kits for carrying out the methods of the disclosure. In some embodiments, a kit can include one or more of a CABRNT, CAVA, and/or CABRNT-CAVA guide of the disclosure, a Cas protein, a polynucleotide encoding a Cas protein, an effector protein, a polynucleotide encoding an effector protein, a multiplexed genetic targeting agent, a polynucleotide encoding a multiplexed genetic targeting agent, a donor polynucleotide, a tandem fusion protein, a polynucleotide encoding a tandem fusion protein, a reporter element, a genetic element of interest, a component of a split system and/or any nucleic acid or proteinaceous molecule necessary to carry out the embodiments of the methods of the disclosure, or any combination thereof.

In some embodiments of the kits, the kit can comprise a single-guide nucleic acid-targeting nucleic acid. In some embodiments of the kits, the kit can comprise a dual-guide nucleic acid-targeting nucleic acid. In some embodiments of the kits, the kit can comprise two or more dual guide or single-guide nucleic acid-targeting nucleic acids. In some embodiments, a vector may encode for a nucleic acid targeting nucleic acid that is used in combination with a synthesized CABRNT, CAVA, or CABRNT-CAVA polynucleotide or guide (*e.g.,* a tracrRNA, a crRNA, a sgRNA, or a crRNA/tracrRNA).

In some embodiments of the kits, the kit can further comprise a donor polynucleotide, or a polynucleotide sequence encoding the donor polynucleotide, or a viral vector comprising a donor polynucleotide, to achieve the desired genetic modification. Components of a kit can be in separate containers or can be combined in a single container.

A kit can further comprise one or more additional reagents, where such additional reagents can be selected from a buffer, a buffer for introducing a polypeptide or polynucleotide item of the kit into a cell, a wash buffer, a control reagent, a control vector, a control RNA polynucleotide, a reagent for *in vitro* production of the polypeptide from DNA, adaptors for sequencing and the like. A buffer can be a stabilization buffer, a reconstituting buffer, or a diluting buffer.

In some instances, a kit can comprise one or more additional reagents specific for plants and/or fungi. One or more additional reagents for plants and/or fungi can include, for example, soil, nutrients, plants, seeds, spores, Agrobacterium, T-DNA vector, and a pBINAR vector.

A kit can also include instructions for using the components of the kit to practice the methods. The instructions for practicing the methods are generally recorded on a suitable recording medium. For example, the instructions may be printed on a substrate, such as paper or plastic, etc. The instructions may be present in the kits as a package insert or in the labeling of the container of the kit or components thereof (*e.g*., associated with the packaging or subpackaging).

Cells comprising CABRNT/Cas, CAVA/Cas, and/or CABRNT-CAVA/Cas protein complexes, cells modified through the use of the complexes, or progeny of such cells can be used as pharmaceutical compositions formulated, for example, with a pharmaceutically acceptable excipient. Illustrative excipients include carriers, stabilizers, diluents, dispersing agents, suspending agents, thickening agents, and the like. The pharmaceutical compositions can facilitate administration of CABRNT/Cas, CAVA/Cas, and/or CABRNT-CAVA/Cas protein complexes to a subject. Pharmaceutical compositions can be administered in therapeutically effective amounts by various forms and routes including, for example, intravenous, subcutaneous, intramuscular, oral, aerosol, parenteral, ophthalmic, and pulmonary administration.

CABRNT/Cas, CAVA/Cas, and/or CABRNT-CAVA/Cas nucleoprotein complexes can be used to cleave or bind to a target nucleic acid. A CABRNT-, CAVA-, and/or CABRNT-CAVA-containing guide can be introduced into cells with a Cas protein, thereby forming a complex. The complex can hybridize to a target nucleic acid, wherein the target nucleic acid comprises a PAM. In one embodiment, the present invention includes a method of binding a nucleic acid target sequence in a polynucleotide (*e.g*., double-stranded DNA (dsDNA)) comprising providing one or more CABRNT, CAVA, and/or CABRNT-CAVA nucleoprotein complexes for introduction into a cell or a biochemical reaction and delivering the CABRNT, CAVA, and/or CABRNT-CAVA nucleoprotein complex into the cell or biochemical reaction, such as by methods described herein, thereby facilitating contact of the CABRNT, CAVA, and/or CABRNT-CAVA nucleoprotein complex with the target polynucleotide sequence. In one embodiment, a first CABRNT nucleoprotein complex comprises a CABRNT guide having a first spacer element complementary to a first nucleic acid target sequence in the polynucleotide and a second CABRNT nucleoprotein complex comprises a CABRNT guide having a second spacer element complementary to a second nucleic acid target sequence in the polynucleotide. Contact of the complex with the polynucleotide results in binding of the CABRNT nucleoprotein complex to the nucleic acid target sequence in the polynucleotide. In one embodiment, a first CABRNT nucleoprotein complex binds to a first nucleic acid target sequence and a second CABRNT nucleoprotein complex binds to a second nucleic acid target sequence in the polynucleotide.

In another embodiment, a first CAVA nucleoprotein complex comprises a CAVA guide having a first spacer element complementary to a first nucleic acid target sequence in the polynucleotide, and a second CAVA nucleoprotein complex comprises a CAVA guide having a second spacer element complementary to a second nucleic acid target sequence in the polynucleotide. Contact of the complex with the polynucleotide results in binding of the CAVA nucleoprotein complex to the nucleic acid target sequence in the polynucleotide. In one embodiment, a first CAVA nucleoprotein complex binds to a first nucleic acid target sequence and a second CAVA nucleoprotein complex binds to a second nucleic acid target sequence in the polynucleotide.

In another embodiment, a first CABRNT-CAVA nucleoprotein complex comprises a CABRNT-CAVA guide having a first spacer element complementary to a first nucleic acid target sequence in the polynucleotide, and a second CABRNT-CAVA nucleoprotein complex comprises a CABRNT-CAVA guide having a second spacer element complementary to a second nucleic acid target sequence in the polynucleotide. Contact of the complex with the polynucleotide results in binding of the CABRNT-CAVA nucleoprotein complex to the nucleic acid target sequence in the polynucleotide. In one embodiment, a first CABRNT-CAVA nucleoprotein complex binds to a first nucleic acid target sequence and a second CABRNT-CAVA nucleoprotein complex binds to a second nucleic acid target sequence in the polynucleotide.

Such methods of binding a nucleic acid target sequence can be carried out *in vitro (e.g.,* in a biochemical reaction or in cultured cells; in some embodiments, the cultured cells are human cultured cells that remain in culture and are not introduced into a human); *in vivo* (*e.g.,* in cells of a living organism, with the proviso that, in some embodiments, the organism is a non-human organism); or *ex vivo* (*e.g.,* cells removed from a subject, with the proviso that, in some embodiments, the subject is a non-human subject).

A variety of methods are known in the art to evaluate and/or quantitate interactions between nucleic acid sequences and polypeptides including, but not limited to, the following: immunoprecipitation (ChIP) assays, DNA electrophoretic mobility shift assays (EMSA), DNA pull-down assays, and microplate capture and detection assays. Commercial kits, materials, and reagents are available to practice many of these methods and, for example, can be obtained from the following suppliers: Thermo Scientific (Wilmington, DE), Signosis (Santa Clara, CA), Bio-Rad (Hercules, CA), and Promega (Madison, WI). A common approach to detect interactions between a polypeptide and a nucleic acid sequence is EMSA. *See, e.g.,* Hellman et al. (Nature Protocols, 2007, 2(8):1849-1861).

In another embodiment, the present invention includes a method of cutting a nucleic acid target sequence in a polynucleotide (*e.g*., a single-strand cut in dsDNA or double-strand cut in dsDNA) comprising providing one or more CABRNT, CAVA, and/or CABRNT-CAVA nucleoprotein complexes for introduction into a cell or biochemical reaction and delivering the CABRNT, CAVA, and/or CABRNT-CAVA nucleoprotein complex into the cell or biochemical reaction, thereby facilitating contact of the CABRNT, CAVA, and/or CABRNT-CAVA nucleoprotein complex with the polynucleotide. In one embodiment, a first CABRNT nucleoprotein complex comprising a CABRNT guide having a first spacer sequence complementary to a first nucleic acid target sequence in the polynucleotide, and a second CABRNT nucleoprotein complex comprising a CABRNT guide having a second spacer sequence complementary to a second nucleic acid target sequence in the polynucleotide are introduced into the cell or biochemical reaction. The contacting results in cutting of the nucleic acid target sequence in the polynucleotide (*e.g*., a dsDNA) by the CABRNT nucleoprotein complex. In one embodiment, the first CABRNT nucleoprotein complex binds to the first nucleic acid target sequence in dsDNA and cleaves the first strand of a dsDNA, and the second CABRNT nucleoprotein complex binds to the second nucleic acid target sequence in dsDNA and cleaves the second strand of a dsDNA. In some embodiments the nucleic acid target sequence is DNA or genomic DNA. Such methods of binding a nucleic acid target sequence are carried out *in vitro,* in cell (*e.g.,* in cultured cells), *ex vivo* (*e.g.,* stem cells removed from a subject), and *in vivo.*

In one embodiment, a first CAVA nucleoprotein complex comprising a CAVA guide having a first spacer sequence complementary to a first nucleic acid target sequence in the polynucleotide, and a second CAVA nucleoprotein complex comprising a CAVA guide having a second spacer sequence complementary to a second nucleic acid target sequence in the polynucleotide are introduced into the cell or biochemical reaction. The contacting results in cutting of the nucleic acid target sequence in the polynucleotide (*e.g*., a dsDNA) by the CAVA nucleoprotein complex. In one embodiment, the first CAVA nucleoprotein complex binds to the first nucleic acid target sequence in dsDNA and cleaves the first strand of a dsDNA, and the second CAVA nucleoprotein complex binds to the second nucleic acid target sequence in dsDNA and cleaves the second strand of a dsDNA. In some embodiments the nucleic acid target sequence is DNA or genomic DNA. Such methods of binding a nucleic acid target sequence are carried out *in vitro,* in cell (*e.g.,* in cultured cells), *ex vivo* (*e.g.,* stem cells removed from a subject), and *in vivo.*

In one embodiment, a first CABRNT-CAVA nucleoprotein complex comprising a CABRNT-CAVA guide having a first spacer sequence complementary to a first nucleic acid target sequence in the polynucleotide, and a second CABRNT-CAVA nucleoprotein complex comprising a CABRNT-CAVA guide having a second spacer sequence complementary to a second nucleic acid target sequence in the polynucleotide are introduced into the cell or biochemical reaction. The contacting results in cutting of the nucleic acid target sequence in the polynucleotide (*e.g*., a dsDNA) by the CABRNT-CAVA nucleoprotein complex. In one embodiment, the first CABRNT-CAVA nucleoprotein complex binds to the first nucleic acid target sequence in dsDNA and cleaves the first strand of a dsDNA, and the second CABRNT-CAVA nucleoprotein complex binds to the second nucleic acid target sequence in dsDNA and cleaves the second strand of a dsDNA. In some embodiments the nucleic acid target sequence is DNA or genomic DNA. Such methods of binding a nucleic acid target sequence are carried out *in vitro,* in cell (*e.g.,* in cultured cells), *ex vivo* (*e.g.,* stem cells removed from a subject), and *in vivo.*

In an additional embodiment of the method of cutting a nucleic acid target sequence in a polynucleotide, a donor polynucleotide can also be introduced into a cell to facilitate incorporation of at least a portion of the donor polynucleotide into genomic DNA of the cell. This disclosure describes methods for bringing a donor polynucleotide into close proximity to a site-directed target nucleic acid break to enhance insertion (*e.g*., homologous recombination) of the donor polynucleotide into the site of the double-strand break. The method can be performed using any of the Cas proteins, CABRNT, CAVA, or CABRNT-CAVA guides, and nucleoprotein complexes as described herein.

In some instances, the methods of the disclosure provide for bringing a donor polynucleotide in close proximity to the site of a double-strand break in a target nucleic acid, by binding it to the nuclease that generates the double-strand break (*e.g*., Cas9).

The donor polynucleotide sequence(s) can be appropriately selected, based upon, for example, the desired modification being pursued. For instance, a donor polynucleotide may encode all or part of a protein of interest. In some embodiments, the donor polynucleotide may encode a CAR.

The present disclosure further encompasses the delivery of a donor polynucleotide to a cell via a virus, wherein the donor polynucleotide encodes a CAR.

In some embodiments, the donor polynucleotide can be single stranded. In some embodiments, the donor polynucleotide can be double stranded. In some embodiments, the donor DNA can be a minicircle. In some embodiments, the donor polynucleotide can be a plasmid. In some embodiments, the plasmid can be supercoiled. In some embodiments, the donor polynucleotide can be methylated. In some embodiments, the donor polynucleotide can be unmethylated. The donor polynucleotide can comprise a modification. Modifications can include those described here including, but not limited to, biotinylation, chemical conjugate, and synthetic nucleotides.

### Therapeutic Compositions, Applications, and Methods

CABRNT, CAVA, and CABRNT-CAVA guides and CABRNT, CAVA, or CABRNT-CAVA/Cas nucleoprotein complexes of the present disclosure can be used in the production of modified cells (such as CAR-expressing cells). Such modified cells can be used, for example, in the field of cell therapy (*e.g*., the treatment or prevention of a disease via the administration of cells), and especially for adoptive cell therapy. Such administered cells may be, for example, genetically modified adoptive cells. Genetic modifications may be introduced into adoptive cells by way of the CABRNT, CAVA, and CABRNT-CAVA guides and CABRNT, CAVA, or CABRNT-CAVA/Cas nucleoprotein complexes disclosed herein, using one or more delivery techniques. The present disclosure encompasses, for example, the modification and administration of cells that are autologous or allogeneic with respect to the recipient to which they are to be administered. As used herein, the term "allogeneic" refers to a different, genetically non-identical, individual of the same species. For instance, an allogeneic cell refers to a cell derived from a different, genetically non-identical, individual of the same species (with respect to the recipient to be administered the cell). By contrast, the term "autologous" refers to the same individual. For instance, an autologous cell administered to an individual refers to a cell (modified or unmodified, or modified or unmodified progeny thereof) that is derived from that same individual.

An "adoptive cell" refers to a cell that can be genetically modified for use in a cell therapy treatment. Adoptive cells include, but are not limited to, stem cells, induced pluripotent stem cells, embryonic stem cells, cord blood stem cells, lymphocytes, natural killer cell, fibroblasts, endothelial cells, epithelial cells, pancreatic precursor cells, and the like.

A "stem cell" refers to a cell that has the capacity for self-renewal, *i.e.,* the ability to go through numerous cycles of cell division while maintaining the undifferentiated state. Stem cells can be totipotent, pluripotent, multipotent, oligopotent, or unipotent. Stem cells are embryonic, fetal, amniotic, adult, or induced pluripotent stem cells.

An "induced pluripotent stem cell" (iPSCs) refers to a type of pluripotent stem cell that is artificially derived from a non-pluripotent cell, typically a somatic cell. In some embodiments, the somatic cell is a human somatic cell. Examples of somatic cells include, but are not limited to, dermal fibroblasts, bone marrow-derived mesencyhmal cells, cardiac muscle cells, keratinocytes, liver cells, stomach cells, neural stem cells, lung cells, kidney cells, spleen cells, and pancreatic cells. Additional examples of somatic cells include cells of the immune system, including, but not limited to, B cells, dendritic cells, granulocytes, innate lymphoid cells, megakaryocytes, monocytes/macrophages, myeloid-derived suppressor cells, NK cells, T cells, thymocytes, and hematopoietic stem cells. Pluripotent stem cells can be differentiated into a plurality of cell types including somatic cells, NK cells, NK-like cells, T cells, T cell-like cells, NK-T cells, NK-T cell-like cells, dendritic cells, dendritic-like cells, macrophages, and macrophage-like cells. Pluripotent stem cells can be edited, before or after differentiation, with a CABRNT, CAVA, or CABRNT-CAVA guide and/or a CABRNT, CAVA, or CABRNT-CAVA/Cas nucleoprotein complex. An iPSC can be further modified, before or after differentiation, through the introduction of an exogenous gene or sequence into the genome, such as sequence encoding a CAR.

A "hematopoietic stem cell" refers to an undifferentiated cell that has the ability to differentiate into a hematopoietic cell, such as a lymphocyte.

A "lymphocyte" refers to a leukocyte (white blood cell) that is part of the vertebrate immune system. Also encompassed by the term "lymphocyte" is a hematopoietic stem cell that gives rise to lymphoid cells. Lymphocytes include T cells for cell-mediated, cytotoxic adaptive immunity, such as CD4+ and/or CD8+ cytotoxic T cells; alpha/beta T cells and gamma/delta T cells; regulatory T cells such as Treg cells; natural killer (NK) cells that function in cell-mediated, cytotoxic innate immunity; and B cells, for humoral, antibody-driven adaptive immunity; NK/T cells; cytokine induced killer cells (CIK cells); and antigen presenting cells (APCs), such as dendritic cells. The lymphocyte can be a mammalian cell, such as a human cell.

Also encompassed by the term "lymphocyte" as used herein are T cell receptor engineered T cells (TCRs), genetically engineered to express one or more specific, naturally occurring or engineered, T-cell receptor(s) that can recognize protein or (glyco)lipid antigens of target cells. Small pieces of these antigens, such as peptides or fatty acids, are shuttled to the target cell surface and presented to the T cell receptors as part of the major histocompatibility complex (MHC). T cell receptor binding to antigen-loaded MHCs activates the lymphocyte.

Tumor infiltrating lymphocytes (TILs) are also encompassed by the term "lymphocyte" as used herein. TILs are immune cells that have penetrated the environment in and around a tumor (the "tumor microenvironment"). TILs are typically isolated from tumor cells and the tumor microenvironment, and are selected *in vitro* for high reactivity against tumor antigens. TILs are grown *in vitro* under conditions that overcome the tolerizing influences that exist *in vivo,* and are then introduced into a subject for treatment.

The term "lymphocyte" also encompasses genetically modified T cells and NK cells, such as those modified to produce chimeric antigen receptors (CARs) on the T or NK cell surface (CAR-T cells and CAR-NK cells).

Lymphocytes can be isolated from a subject, such as a human subject, for example from blood or from solid tumors, such as in the case of TILs, or from lymphoid organs such as the thymus, bone marrow, lymph nodes, and mucosal-associated lymphoid tissues. Techniques for isolating lymphocytes are well known in the art. For example, lymphocytes can be isolated from peripheral blood mononuclear cells (PBMCs), which are separated from whole blood using, *e.g.,* ficoll, a hydrophilic polysaccharide that separates layers of blood, and density gradient centrifugation. Generally, anticoagulant or defibrinated blood specimens are layered on top of a ficoll solution and centrifuged to form different layers of cells. The bottom layer includes red blood cells (erythrocytes), which are collected or aggregated by the ficoll medium and sink completely through to the bottom. The next layer contains primarily granulocytes, which also migrate down through the ficoll-paque solution. The next layer includes lymphocytes, which are typically at the interface between the plasma and the ficoll solution, along with monocytes and platelets. To isolate the lymphocytes, this layer is recovered, washed with a salt solution to remove platelets, ficoll and plasma, then centrifuged again.

Other techniques for isolating lymphocytes include biopanning, which isolates cell populations from solution by binding cells of interest to antibody-coated plastic surfaces. Unwanted cells are then removed by treatment with specific antibody and complement. Additionally, fluorescence activated cell sorter (FACS) analysis can be used to detect and count lymphocytes. FACS analysis uses a flow cytometer that separates labelled cells based on differences in light scattering and fluorescence.

For TILs, lymphocytes are isolated from a tumor and grown, for example, in high-dose IL-2 and selected using cytokine release co-culture assays against either autologous tumor or HLA-matched tumor cell lines. Cultures with evidence of increased specific reactivity compared to allogeneic non-MHC matched controls can be selected for rapid expansion and then introduced into a subject in order to treat cancer. *See, e.g.,* Rosenberg et al. (Clin. Cancer Res., 2011, 17:4550-4557); Dudly et al. (Science, 2002, 298:850-854); Dudly et al. (J. Clin. Oncol., 2008, 26:5233-5239); and Dudley et al. (J. Immunother., 2003, 26:332-342).

Upon isolation, lymphocytes can be characterized in terms of specificity, frequency, and function. Frequently used assays include an ELISPOT assay, which measures the frequency of T cell response.

After isolation, lymphocytes can be activated using techniques well known in the art in order to promote proliferation and differentiation into specialized effector lymphocytes. Surface markers for activated T cells include, for example, CD3, CD4, CD8, PD1, IL2R, and others. Activated cytotoxic lymphocytes can kill target cells after binding cognate receptors on the surface of target cells. Surface markers for NK cells include, for example, CD16, CD56, and others.

Following isolation and optionally activation, lymphocytes can be modified using CABRNT, CAVA, or CABRNT-CAVA guides and/or CABRNT, CAVA, or CABRNT-CAVA/Cas nucleoprotein complexes of the present disclosure, for use in adoptive T cell immunotherapies. Adoptive immunotherapy typically utilizes a patient's immune cells (autologous cells) to treat cancer. However, the present methods for the generation of adoptive immunotherapies also allow for the use of third-party donor cells (allogeneic cells), resulting in "off the shelf' therapies.

Thus, in some embodiments, lymphocytes for use in adoptive immunotherapies are isolated from a subject, modified *ex vivo,* and then reintroduced into the same subject. This technique is known as "autologous lymphocyte therapy."

Alternatively, lymphocytes can be isolated, modified *ex vivo,* and introduced into a different subject. This technique is known as "allogeneic lymphocyte therapy."

In certain embodiments, CABRNT, CAVA, or CABRNT-CAVA guides and/or CABRNT, CAVA, or CABRNT-CAVA/Cas nucleoprotein complexes are used for the production of therapeutic compositions comprising allogeneic cells. In a preferred embodiment, the allogeneic cells are T cells. In a more preferred embodiment, the T cells express a CAR. In an even more preferred embodiment, the CAR targets an antigen associated with a cancer.

In some embodiments, T cells can be modified to allow for safer and more efficient allogeneic therapies. For example, the T cell receptor α constant (TRAC) is a protein-coding gene that forms part of the αβ TCR. Selected mutations in TRAC, as well as knocking out expression of TRAC, can therefore help eliminate GvHD during allogeneic cell therapies. *See, e.g.,* Poirot et al. (Cancer Res., 2015, 75:3853-3864). It has been shown that directing a CD19-specific CAR to the TRAC locus using a CRISPR-Cas9 system can result in tumor rejection. *See, e.g.,* Eyquem et al. (Nature, 2017, 543:113). Similarly, T cell receptor β constant (TRBC) can also be targeted in order to prevent expression of the αβ TCR. *See, e.g.,* Ren et al. (Clin. Cancer Res., 2017, 23:2255-2266).

Programmed cell death protein 1, also known as PD1, PDCD1, and CD279, is a cell surface receptor that plays an important role in down-regulating the immune system, and promoting self-tolerance by suppressing T cell inflammatory activity. PDCD1 binds to its cognate ligand, "programmed death-ligand 1," also known as PD-L1, CD274, and B7 homolog 1 (B7-H1). PD1 guards against autoimmunity through a dual mechanism of promoting programmed cell death (apoptosis) in antigen-specific T cells in lymph nodes, while simultaneously reducing apoptosis in anti-inflammatory, suppressive T cells (regulatory T cells). Through these mechanisms, PD1 binding of PD-L1 inhibits the immune system, thus preventing autoimmune disorders, but also prevents the immune system from killing cancer cells. Accordingly, mutating or knocking out production of PD1 can be beneficial in T cell therapies.

PD1 is an example of an "immune checkpoint" molecule. Immune checkpoint molecules serve to down-modulate or inhibit an immune response. Immune checkpoint molecules include, but are not limited to, PD1, Cytotoxic T-Lymphocyte Antigen 4 (CTLA-4, also known as CD152), LAG3 (also known as CD223), Tim3 (also known as HAVCR2), BTLA (also known as CD272), BY55 (also known as CD160), TIGIT (also known as IVSTM3), LAIR1 (also known as CD305), SIGLEC10, 2B4 (also known as CD244), PPP2CA, PPP2CB, PTPN6, PTPN22, CD96, CRTAM, SIGLEC7, SIGLEC9, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT1, FOXP3, PRDM1, BATF, GUCY1A2, GUCY1A3, GUCY1B2, and GUCY1B3. In some embodiments, one or more immune checkpoint molecules are inactivated using CABRNT, CAVA, or CABRNT-CAVA guides and/or CABRNT, CAVA, or CABRNT-CAVA/Cas nucleoprotein complexes of the present disclosure. In some embodiments, the inactivation of one or more immune checkpoint molecules is combined with the inactivation of one or more TCR components, as described above.

Beta-2 microglobulin (B2M) is a component of MHC class I molecules present on nucleated cells. Beta-2 microglobulin is shed by cells, including tumor cells, into the blood and is essential for the assembly and expression of the HLA I complex. However, expression of HLA on the surface of allogeneic T cells causes rapid rejection by T cells of the host immune system. Thus, disrupting expression of beta-2 microglobulin is also desirable for increasing allogeneic T cell therapy efficiency. Additionally, lack of expression of MHC class I molecules on allogeneic T cells causes clearance by the host immune system. Thus, presentation of only a subset of the HLA molecules, preferably HLA-E, on the suface of cells is desirable.

Additional genes can be similarly targeted with the CABRNT, CAVA, and CABRNT-CAVA guides and CABRNT, CAVA, or CABRNT-CAVA/Cas nucleoprotein complexes disclosed herein to enhance the efficacy of an adoptive immune cell therapy. Non-limiting examples of preferred genes and chromosomal locations (hg38 genome assembly) are provided in Table 2.

| **Table 2** | |
|---|---|
| **Adoptive Cell Therapy Targets** | |
| **Encoded Protein/Alternative name** | **Locus coordinates** |
| TRAC | chr14:22,509,239-22,552,153 |
| TRBV proteins | TRBV locus chr7 |
| Beta-2 microglobulin (B2M) | chr15:44,711,477-44,718,877 |
| PDCD1/PD1/CD279 | chr2:241,849,881-241,858,908 |
| PD-L1 | chr9:5,450,525-5,470,547 |
| CTLA-4/CD 152 | chr2:203,867,786-203,873,960 |
| LAG-3/CD223 | chr12:6,772,520-6,778,453 |
| TIGIT/IVSTM3 | chr3:114,291,059-114,308,290 |
| TIM3/HAVCR2 | chr5:157,085,832-157,109,714 |
| HLA-E | HLA-E locus chr6 |
| HLA-A | HLA-A locus chr6 |
| HLA-B | HLA-B locus chr6 |
| HLA-C | HLA-C locus chr6 |
| HLA-DRA | HLA-DRA locus chr6 |
| ADAM17 | chr2:9,488,486-9,555,788 |
| BTLA/CD272 | chr3:112,463,968-112,499,561 |
| CD160 | chr1:145,719,520-145,739,153 |
| SIGLEC10 | chr19:51,411,054-51,417,698 |
| 2B4/CD244 | chr1:160,830,160-160,862,855 |
| LAIR1/CD305 | chr19_KI270938v1_alt:337,398-347,586 |
| CD52 | chr1:26,317,957-26,320,523 |
| CD96 | chr3:111,542,118-111,652,241 |
| VSIR or C10orf54/VISTA | chr10:71,747,559-71,773,498 |
| KIR2DL1 | chr19_KI270933v1_alt:71,449-85,961 |
| KIR2DL2 | chr19_KI270932v1_alt:56,346-70,673 |
| KIR2DL3 | chr19:54,738,515-54,753,052 |
| CEACAM1 | chr19:42, 507,306-42, 528, 509 |
| CBLB | chr3:105,655,461-105,869,043 |
| CISH | chr3:50,606,522-50,611,831 |
| IL-1R8/TIR8/SIGIRR | chr11:405,716-417,397 |
| AHR | chr7:17,298,622-17,346,150 |
| Adenosine 2A receptor/ADORA2A | chr22:24,423,597-24,442,356 |
| GMCSF/CSF2 | chr5:132,073,789-132,076,170 |
| VISTA/VSIR | chr10:71,747,556-71,773,520 |
| CII2A | chr16:10,877,198-10,936,388 |
| NKG2A | chr12:10,446,041-10,454,616 |

In some embodiments, a gene encoding TRAC is targeted within a cell using a CABRNT, CAVA, or CABRNT-CAVA guide and/or a CABRNT, CAVA, or CABRNT-CAVA/Cas nucleoprotein complex as disclosed herein. In some embodiments, a gene encoding PD1 is targeted within a cell using a CABRNT, CAVA, or CABRNT-CAVA guide and/or a CABRNT, CAVA, or CABRNT-CAVA/Cas nucleoprotein complex as disclosed herein. In some embodiments, a gene encoding B2M is targeted within a cell using a CABRNT, CAVA, or CABRNT-CAVA guide and/or a CABRNT, CAVA, or CABRNT-CAVA/Cas nucleoprotein complex as disclosed herein. In some embodiments, a gene encoding TRAC and a gene encoding B2M are targeted within a cell, using CABRNT, CAVA, and/or CABRNT-CAVA guides and/or CABRNT, CAVA, and/or CABRNT-CAVA/Cas nucleoprotein complexes as disclosed herein.

Cells modified using a CABRNT, CAVA, or CABRNT-CAVA guide and/or a CABRNT, CAVA, or CABRNT-CAVA/Cas nucleoprotein complex of the present disclosure can be used, for example, in adoptive cell therapy for the treatment of cancer. In some embodiments thereof, the modified cell is a genetically modified lymphocyte. Such genetically modified lymphocytes, such as CAR-T cells, can be used to treat various types of cancers in a subject, including, but not limited to, prostate cancers; ovarian cancers; cervical cancers; colorectal cancers; intestinal cancers; testicular cancers; skin cancers; lung cancers; thyroid cancers; bone cancers; breast cancers; bladder cancers; uterine cancers; vaginal cancers; pancreatic cancers; liver cancers; kidney cancers; brain cancers; spinal cord cancers; oral cancers; parotid tumors; blood cancers; lymphomas, such as B cell lymphomas; and leukemias, *etc.* Preferably, an effective amount of modified cells is used for such treatment.

Table 3 lists representative B cell leukemias and lymphomas treatable using adoptive cells (such as CAR-T cells) produced using CABRNT, CAVA, and CABRNT-CAVA guides and/or CABRNT, CAVA, and CABRNT-CAVA/Cas nucleoprotein complexes of the present disclosure. It is to be understood that the lymphocytes modified by the CABRNT, CAVA, and CABRNT-CAVA guides and/or CABRNT, CAVA, and CABRNT-CAVA/Cas nucleoprotein complexes disclosed herein are not limited for treatment of the diseases listed in Table 3 herein.

| **Table 3** | |
|---|---|
| **B-Cell Leukemias/Lymphomas and Acronyms Thereof** | |
| **B-Cell leukemia/lymphoma** | **Acronym** |
| Acute lymphoblastic leukemia | B-ALL |
| Precursor B lymphoblastic leukemia | LBL |
| Hairy cell leukemia | HCL |
| B-cell prolymphocytic leukemia | B-PLL |
| Chronic lymphocytic leukemia/small lymphocytic leukemia | CLL/SLL |
| Diffuse large B-cell lymphoma | DLBCL |
| ALK-positive large B-cell lymphoma | ALK+LBCL |
| T-cell/histiocyte-rich large B-cell lymphoma | T/HRBCL |
| Follicular lymphoma | FL |
| Mantle cell lymphoma | MCL |
| Burkitt lymphoma (EBV) | BL |
| Multiple myeloma | MM |
| Lymphoplasmacytic lymphoma/Waldenstrom macroglobulinemia | MG |
| Mucosa-associated lymphoid tissue lymphoma | MALT |
| Nodal marginal zone B-cell lymphoma | NMZL |
| Splenic marginal zone B-cell lymphoma | SMZL |
| Primary central nervous system lymphoma | PCNSL |
| Primary cutaneous follicle center lymphoma | PCFCL |
| Primary mediastinal large B-cell lymphoma | PMLBCL |
| Intravascular large B-cell lymphoma | ILBCL |
| Lymphomatoid granulomatosis (EBV) | LYG |
| Plasmablastic lymphoma (ARL) | PBL |
| Primary effusion lymphoma | PEL |

In other embodiments, other cell proliferative disorders can be treated using adoptive cells (such as CAR-T cells) produced using CABRNT, CAVA, and CABRNT-CAVA guides and/or CABRNT, CAVA, and CABRNT-CAVA/Cas nucleoprotein complexes of the present disclosure, including precancerous conditions; hematologic disorders; and immune disorders, such as autoimmune disorders including, without limitation, Addison's disease, celiac disease, diabetes mellitus type 1, Grave's disease, Hashimoto's disease, inflammatory bowel disease, multiple sclerosis, psoriasis, rheumatoid arthritis, scleroderma, and systemic lupus erythematosus.

The adoptive cell therapy treatments described herein can be combined, at the same or different times, with one or more additional therapies selected from the group consisting of antibody therapy, chemotherapy, cytokine therapy, dendritic cell therapy, gene therapy, hormone therapy, laser light therapy, and radiation therapy.

The administration of modified cells of the present disclosure to subjects may be carried out in any convenient manner, including by aerosol inhalation, injection, ingestion, transfusion, implantation or transplantation. The compositions described herein may be administered to a patient subcutaneously, intradermally, intratumorally, intranodally, intramedullary, intramuscularly, by intravenous or intralymphatic injection, or intraperitoneally.

In one embodiment, the modified cell compositions of the present disclosure are preferably administered by intravenous injection. The administration may comprise the administration of 10⁴-10⁹ cells per kg body weight, preferably 10⁵ to 10⁶ cells/kg body weight. The cells can be administrated in one or more doses. In some embodiments, an effective amount of modified cells is administrated as a single dose. In other embodiments, an effective amount of cells is administrated as more than one dose over a period time. The determination of optimal ranges of effective amounts of a given cell type for a particular disease or condition is within the skill of those in the art.

### Chimeric Antigen Receptor (CAR) Cells

In some embodiments, an adoptive cell is a CAR-expressing cell. A CAR is a receptor engineered to recognize and bind to a specific antigen or epitope. The receptor is chimeric because it combines both antigen-binding and T cell activating functions into a single receptor. A CAR is typically a fusion protein comprising an extracellular ligand-binding domain capable of binding to an antigen, a transmembrane domain, and at least one intracellular signaling domain. An extracellular ligand-binding domain may comprise a single-chain variable fragment (scFv) comprising a fusion of two or more variable regions connected by one or more linkers. A CAR may further comprise a hinge region. A CAR is sometimes called a "chimeric receptor," a "T-body," or a "chimeric immune receptor (CIR)."

In some embodiments, the CAR can be a TRUCK, Universal CAR, Self-driving CAR, TanCAR, Armored CAR, Self-destruct CAR, Conditional CAR, Marked CAR, TenCAR, Dual CAR, or sCAR.

TRUCKs (T cells redirected for universal cytokine killing) co-express a chimeric antigen receptor (CAR) and an antitumor cytokine. Cytokine expression may be constitutive or induced by T cell activation. Targeted by CAR specificity, localized production of pro-inflammatory cytokines recruits endogenous immune cells to tumor sites and may potentiate an antitumor response.

Universal, allogeneic CAR-T cells are engineered to no longer express endogenous T cell receptor (TCR) and/or major histocompatibility complex (MHC) molecules, thereby preventing graft-versus-host disease (GVHD) or rejection, respectively.

Self-driving CARs co-express a CAR and a chemokine receptor, which binds to a tumor ligand, thereby enhancing tumor homing.

CAR-T cells engineered to be resistant to immunosuppression (Armored CARs) may be genetically modified to no longer express various immune checkpoint molecules (for example, cytotoxic T lymphocyte-associated antigen 4 (CTLA4) or programmed cell death protein 1 (PD1)), with an immune checkpoint switch receptor, or may be administered with a monoclonal antibody that blocks immune checkpoint signaling.

A self-destruct CAR may be designed using RNA delivered by electroporation to encode the CAR. Alternatively, inducible apoptosis of the T cell may be achieved based on ganciclovir binding to thymidine kinase in gene-modified lymphocytes or the more recently described system of activation of human caspase 9 by a small-molecule dimerizer.

A conditional CAR-T cell is by default unresponsive, or switched `off , until the addition of a small molecule to complete the circuit, enabling full transduction of both signal 1 and signal 2, thereby activating the CAR-T cell. Alternatively, T cells may be engineered to express an adaptor-specific receptor with affinity for subsequently administered secondary antibodies directed at target antigen.

Marked CAR-T cells express a CAR plus a tumor epitope to which an existing monoclonal antibody agent binds. In the setting of intolerable adverse effects, administration of the monoclonal antibody clears the CAR-T cells and alleviates symptoms with no additional off-tumor effects.

A tandem CAR (TanCAR) T cell expresses a single CAR comprising two linked scFvs that have different affinities and are fused to one or more intracellular co-stimulatory domain(s) and a CD3ζ signaling domain. TanCAR-T cell activation requires only one antigen to be present on target cells; however, the presence of both antigens facilitates a synergistic activation. In certain embodiments, an scFv of the TanCAR comprises a heavy chain variable region (VH) and light chain variable region (VL), a pair of two heavy chain variable regions (VH), or a pair of two light chain variable regions (VL). In another embodiment, the two scFvs of the TanCAR can occur in a stacked configuration. In yet another embodiment, the two scFvs of the TanCAR can occur in series, or in a looped configuration. In specific embodiments, at least one of the scFvs of the tandem CAR is an anti-CD20 scFv, and the second scFv is selected to target a specific antigen on cancer cells, such as an anti-BCMA scFv, an anti-CD19 scFv, an anti-CD30 scFv, an anti-CD22 scFv, an anti-CD70 scFv, an anti-ROR1 scFv, or an anti-kappa light chain scFv.

A dual CAR-T cell expresses two separate CARs with different ligand binding targets; one CAR includes only the CD3ζ domain and the other CAR includes only the co-stimulatory domain(s). Dual CAR-T cell activation requires co-expression of both targets on the tumor.

A safety CAR (sCAR) consists of an extracellular scFv fused to an intracellular inhibitory domain, sCAR-T cells co-expressing a standard CAR become activated only when encountering target cells that possess the standard CAR target but lack the sCAR target.

The extracellular (antigen recognition) domain of a CAR is preferably a single chain antibody, and more preferably an scFv. In one embodiment, the antigen-binding domain comprises an scFv. However, any suitable moiety that binds a given target with high affinity can be used as an antigen recognition region. The extracellular domain of a CAR capable of binding to an antigen may be, for example, any oligopeptide or polypeptide that can bind to a certain antigen.

Depending on the desired antigen to be targeted, a CAR of the present disclosure can be engineered to include the appropriate antigen-binding moiety that is specific to the desired antigen target. For example, if BCMA is the desired antigen that is to be targeted, an antibody or antibody fragment (for example an scFv) targeting BCMA can be used as the antigen-binding moiety for incorporation into the CAR of the present disclosure.

Preferable cellular targets and the CAR scFvs/binding proteins that target them are set forth in Table 4.

| **Table 4** | |
|---|---|
| **List of Exemplary Cellular Targets and CAR scFvs** | |
| **Cellular Target** | **CAR scFv/binding portion** |
| B7-H3 | anti-B7-H3 |
| B7-H6 | anti-B7-H6 |
| B-cell maturation antigen (BCMA) | anti-BCMA |
| CD123 | anti-CD123 |
| CD138 | anti-CD138 |
| CD171/L1CAM | anti-CD171 |
| CD19 | anti-CD19 |
| CD20 | anti-CD20 |
| CD22 | anti-CD22 |
| CD30 | anti-CD30 |
| CD33 | anti-CD33 |
| CD37 | anti-CD37 |
| CD38 | anti-CD38 |
| CD4 | anti-CD4 |
| CD47 | anti-CD47 |
| CD70 | anti-CD70 |
| CD73 | anti-CD73 |
| CD79b | anti-CD79b |
| CD371 | anti-CD371 |
| CEA | anti-CEA |
| Claudin 18.1 | anti-CLDN 18.1 |
| Claudin 18.2 | anti-CLDN 18.2 |
| CS-1 | anti-CS1 |
| CSPG4 | anti-CSPG4 |
| EFGRvIII | anti-EFGRvIII |
| ENPP3 | anti-ENPP3 |
| EpCAM | anti-EpCAM |
| EphA2 | anti-EphA2 |
| Epidermal growth factor receptor | anti-epidermal growth factor receptor |
| ErbB | anti-ErbB |
| ErbB2 (HER2) | anti-ErbB2 (HER2) |
| FAP | anti-FAP |
| FRα | anti-FRα |
| GD2 | anti-GD2 |
| GD3 | anti-GD3 |
| Glypican 3 | anti-glypican 3 |
| Her2 | anti-Her2 |
| IL-11Rα | anti-IL-11Rα |
| IL-13Rα2 | anti-IL-13Rα2 |
| IL13 receptor alpha | IL13 |
| kappa light chain | anti-kappa light chain |
| LewisY/LeY | Anti-LeY |
| Mesothelin | anti-mesothelin |
| MUC1 | anti-MUC1 |
| MUC16 | anti-MUC16 |
| NGFR | anti-NGFR |
| NKG2D ligands | anti-NKG2D ligands |
| PD1 | anti-PD1 |
| PD-L1 | anti-PD-L1 |
| PSCA | anti-PSCA |
| PSMA | anti-PSMA |
| ROR-1 | anti-ROR-1 |
| SLAMF7 | anti-SLAMF7 |
| TACI | anti-TACI |
| TAG72 | anti-TAG72 |
| ULBP and MICA/B proteins | NKG2D |
| VEGF2 | anti-VEGF2 |
| VEGFR2 | anti-VEGFR2 |
| WT1 | anti-WT1 |

In certain embodiments, the cellular target to which the CAR binds is more preferably selected from BCMA, CD19, CD20, CD22, CD47, CD79b, CD371, ROR-1, EphA2, MUC16, Glypican 3, PSCA, and Claudin 18.2.

In an even more preferable embodiment, the cellular target to which the CAR binds is BCMA.

In an even more preferable embodiment, the cellular target to which the CAR binds is CD371.

The intracellular domain of a CAR may be an oligopeptide or polypeptide known to function as a domain that transmits a signal to cause activation or inhibition of a biological process in a cell. The intracellular domain may comprise an activation domain comprising all or a portion of the intracellular signaling domain of a T-cell receptor (TCR) and/or a co-receptor, as long as it transduces the effector function signal. Cytoplasmic signaling sequences that regulate primary activation of the TCR complex that act in a stimulatory manner may contain signaling motifs which are known as immunoreceptor tyrosine-based activation motifs (ITAMs). Examples of ITAM containing cytoplasmic signaling sequences include those derived from CD8, CD3ζ, CD3δ, CD3γ, CD3ε, CD32 (FcyRIIa), DAP10, DAP12, CD79a, CD79b, FcγRIγ, FcyRIIIy, FcεRIβ (FCERIB), and FcεRIγ (FCERIG).

In preferred embodiments, the activation domain of the intracellular signaling domain is derived from CD3ζ.

The intracellular signaling domain of a CAR of the present disclosure can be designed to comprise an activation domain, such as a CD3ζ signaling domain, either by itself or combined with any other desired cytoplasmic domain(s) useful in the context of a CAR of the present disclosure. For example, the intracellular signaling domain of the CAR may comprise an activation domain, such as a CD3ζ chain portion, in addition to a costimulatory domain. The costimulatory domain refers to a portion of the CAR comprising the intracellular domain of a costimulatory molecule.

A costimulatory molecule is a molecule other than an antigen receptor or its ligands that is required for an efficient response of lymphocytes to an antigen. Examples of such co-stimulatory molecules, in which all or a part thereof can be used in a costimulatory domain of a CAR of the present disclosure, include CD27, CD28, 4-1BB (CD137), OX40, CD30, CD40, ICOS-1, GITR, lymphocyte function-associated antigen-1 (LFA-1), CD2, CD7, LIGHT, NKG2C and B7-H3.

In preferred embodiments, the CAR contains a costimulatory domain derived from at least 4-1BB.

The transmembrane domain may be derived from either a natural or synthetic source. Where the source is natural, the domain may be derived from any membrane-bound or transmembrane protein. For example, the transmembrane region may be derived from (*i.e.,* comprise at least a part of) the transmembrane region(s) of the alpha, beta or zeta chain of the T-cell receptor, CD28, CD3ζ, CD3ε, CD45, CD4, CD5, CD8 (e.g., CD8α, CD8β), CD9, CD16, CD22, CD33, CD37, CD64, CD80, CD86, CD134, CD137, or CD154, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2Rβ, IE2Pγ, IL7Rα, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD 11b, ITGAX, CD 11c, ITGB1, CD29, ITGB2, CD 18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, and PAG/Cbp.

Alternatively, the transmembrane domain may be synthetic, in which case it will comprise predominantly hydrophobic residues such as leucine and valine. In some cases, a triplet of phenylalanine, tryptophan and valine will be found at each end of a synthetic transmembrane domain. A short oligo- or polypeptide linker, such as between 2 and 10 amino acids in length, may form the linkage between the transmembrane domain and the endoplasmic domain of the CAR.

In a preferred embodiment, the transmembrane domain is derived from CD8.

In some embodiments, the CAR has more than one transmembrane domain, which can be a repeat of the same transmembrane domain, or can be different transmembrane domains.

The hinge region may comprise a polypeptide hinge of variable length, such as one or more amino acids, a CD8 portion, or a IgG4 region, and combinations thereof.

In a preferred embodiment, the hinge region is derived from CD8.

CARs can also be incorporated into TILs, NK cells, macrophages, dendritic cells, induced pluripotent stem cells (iPSCs), or TCRs resulting in CAR-TILs, CAR-NK cells, CAR-M, CAR-DC, and TCR engineered CAR-T cells, respectively. For descriptions of CAR-T cells, methods of making CAR-T cells, and uses thereof, *see, e.g.,* Brudno et al. (Nature Rev. Clin. Oncol., 2018, 15:31-46); Maude et al. (N. Engl. J. Med., 2014, 371: 1507-1517); and Sadelain et al. (CancerDisc., 2013, 3:388-398).

In some embodiments, the CAR expression cassette is transduced into an adoptive cell, and the cassette is integrated into a Cas protein-mediated break site.

In some embodiments, the CAR expression cassette comprises a promoter to drive CAR expression. Commonly used promoters include the constitutive mammalian promoters CMV, MND, EF1a, SV40, PGK1 (mouse or human), Ubc, CAG, CaMKIIa, and beta-Act, and others are known in the art. *See, e.g.,* Khan et al. (Advanced Pharmaceutical Bulletin, 2013, 3:257-263). Alternatively, a CAR expression cassette can comprise a ribosomal skipping sequence (also called a self-cleaving peptide) and can be introduced in-frame of an endogenously expressed gene. Commonly used ribosomal skipping sequences include T2A, P2A, E2A, and F2A. For a description of ribosomal skipping sequences and uses thereof, *see, e.g.,* Chng et al. (MAbs, 2015, 7(2):403-412). Similarly, non-CAR expression cassettes can comprise similar promoters or ribosomal skilling sequences.

In certain embodiments, CABRNT, CAVA, and CABRNT-CAVA guides and/or CABRNT, CAVA, and CABRNT-CAVA/Cas nucleoprotein complexes are used to treat genetic disorders that are caused by pathogenic, autosomal "dominant negative" mutations that are present on a single allele in a patient. In some instances, the underlying genetic mutation may be a single nucleotide polymorphism (SNP) on one of the alleles. CABRNT, CAVA, and CABRNT-CAVA guides and/or CABRNT, CAVA, and CABRNT-CAVA/Cas nucleoprotein complexes may be engineered to target the SNP allele, but not target the wild type allele, thereby disrupting only the SNP allele.

In some embodiments, CABRNT, CAVA, and CABRNT-CAVA guides and/or CABRNT, CAVA, and CABRNT-CAVA/Cas nucleoprotein complexes can be used to selectively edit (e.g., knock-out, or revert back to wild type with homology directed repair) the SNP-containing allele, while not modifying the wild type allele. In some embodiments, such editing may lead to gene disruption. In other embodiments, such editing may restore the allele back to a "wild type" state, such as through homology directed repair. For instance, a number of genetic diseases that lead to progressive vision loss are due to pathogenic, autosomal "dominant-negative" mutations. Examples of SNP correction strategies of dominant negative disease include, but are not limited to, targeting of SNP mutations in Rhodopsin gene causing retinitis pigmentosa, *see, e.g.,* Li et al. (CRISPR J., 2018, 1(1):55-64); and targeting of SNP mutations in the transforming growth factor, beta-induced (*TGFBI)* gene causing corneal dystrophies, *see, e.g.,* Christie et al. (Scientific Reports, 2017, 7(1): 16174).

CABRNT, CAVA, and CABRNT-CAVA guides and/or CABRNT, CAVA, and CABRNT-CAVA/Cas nucleoprotein complexes of the present disclosure can be delivered, for example, to ocular tissues that are affected by autosomal, pathogenic "dominant-negative" genetic mutations. In some embodiments thereof, the CABRNT, CAVA, or CABRNT-CAVA guide and/or the CABRNT, CAVA, or CABRNT-CAVA/Cas nucleoprotein complex is designed to selectively disrupt the disease allele, while not targeting the wild type allele, to treat the underlying pathology. Such diseases may include, but are not limited to, macular dystrophies, rod-cone dystrophies, cone-rod dystrophies, or chorioretinopathies. It is understood that the CABRNT, CAVA, and CABRNT-CAVA guides and CABRNT, CAVA, and CABRNT-CAVA/Cas nucleoprotein complexes disclosed herein are not limited for the treatment of genetic diseases that cause progressive vision loss.

### Experimental

Non-limiting embodiments of the present invention are illustrated in the following Examples. Efforts have been made to ensure accuracy with respect to numbers used (*e.g.,* amounts, concentrations, percent changes, and the like), but some experimental errors and deviations should be accounted for. Unless indicated otherwise, temperature is in degrees Centigrade and pressure is at or near atmospheric. It should be understood that these Examples are given by way of illustration only and are not intended to limit the scope of what the inventor regards as various embodiments of the present invention. Not all of the following steps set forth in each Example are required nor must the order of the steps in each Example be as presented. As used herein, an "r" preceding a nucleotide indicates RNA; abasic ribose sites are represented as "rN"; abasic deoxyribose are represented a "dN"; deoxyuradine bases are shown as "U"; inosine base are shown as "rI"; deoxyinosine bases are shown as "I"; and all other nucleotides are DNA.

### Example 1

### Preparation of Cytotoxic T Cells (CD4+ and CD8+) from PBMCs and Culture of Primary Cells

This Example illustrates the preparation of CD4+ and CD8+ T cells from donor peripheral blood mononuclear cells (PBMCs).

CD4+ and CD8+ T cells were prepared from donor PBMCs essentially as follows. T cells were isolated from peripheral blood mononuclear cells (PBMCs) using RoboSep-S (STEMCELL Technologies, Cambridge, MA) and EasySep^{™} Human T cell Isolation Kit (STEMCELL Technologies 17951RF) and activated for 3 days in the presence of anti-CD3/CD28 beads (Dynabeads^{™}; Gibco 11132D) in ImmunoCult-XF complete medium (ImmunoCult-XF T Cell Expansion Medium (STEMCELL Technologies 10981), CTS Immune Cell SR (Gibco A2596102), Antibiotics-Antimycotics (100X, Corning 30-004-Cl)) supplemented with recombinant human (rh) IL-2 (100 units/mL). After 3 days, beads were removed via magnetic separation and cells were expanded for 1 day in ImmunoCult-XF complete medium supplemented with IL-2 (100 units/mL).

### Example 2

### Cloning. Expression, Production, and Assembly of

### Guide/Cas9 Protein Nucleoprotein Complexes

This Example describes a method for cloning, expressing, and purifying guide/Cas9 protein complexes, as well as methods of producing guide components.

### A. Cloning a Cas9 protein

The *S. pyogenes* (Spy) catalytically active Cas9 protein sequence (SEQ ID NO: 3) was codon optimized for expression in *E. coli* cells. At the C-terminus, one nuclear localization sequence (NLS) (SEQ ID NO: 4) was added. Oligonucleotide sequences coding for the Cas9-NLS protein (referred to as the Cas9 protein in the following Examples) were provided to commercial manufacturers for synthesis. DNA sequences were then cloned into suitable bacterial expression vectors using standard cloning methods.

### B. Expression and purification of a Cas9 protein

The SpyCas9 protein was expressed in *E. coli* using an expression vector and purified using affinity chromatography, ion exchange, and size exclusion chromatography essentially as described in Jinek et al. (Science, 2012, 337:816-821).

### C. Production of guide components

Guide sequences (such as crRNAs, tracrRNAs, sgRNAs, CABRNT, CAVA, and ch-acrs) were provided to a commercial manufacturer for synthesis.

Guide RNA components (such as crRNAs, tracrRNAs, and sgRNAs) can be produced by *in vitro* transcription (*e.g.,* T7 Quick High Yield RNA Synthesis Kit; New England Biolabs, Ipswich, MA) from double-stranded (ds) DNA templates by incorporating a T7 promoter at the 5' end of the dsDNA template sequences.

### D. Assembly of guide/Cas9 protein nucleoprotein complexes

*S. pyogenes* Cas9 was tagged at the C-terminus with a nuclear localization sequence (NLS) and was recombinantly expressed in *E. coli* and purified using chromatographic methods. Nucleoprotein complexes were formed at a concentration of 60 pmol Cas9 protein: 180 pmol guide (dual guide components were used at 180 pmol each). Prior to assembly with Cas9 protein, each of the guide components (*e.g*., crRNA, tracrRNA, CABRNT, CAVA, or ch-acr) was adjusted to the desired total concentration (180 pmol) in a final volume of 1.5 µl, incubated for 2 minutes at 95°C, removed from a thermocycler, and allowed to equilibrate to room temperature. The Cas9 protein was diluted to an appropriate concentration in binding buffer (20 mM HEPES, 150 mM KCl, 10 mM MgCl₂, and 5% glycerol at pH 7.4) to a final volume of 3 µl and mixed with the 1.5 µl of the guide components, followed by incubation at 37°C for 30 minutes.

### Example 3

### Nucleofection of T Cells (CD4+ and CD8+) from PBMCs with Guide/Cas9 Protein Nucleoprotein Complexes

This Example describes the nucleofection of activated T cells with guide/Cas9 protein nucleoprotein complexes.

The guide/Cas9 *protein* nucleoprotein complexes of Example 2 were transfected into primary activated T cells (CD4+ and CD8+) (prepared as described in Example 1) using the Nucleofector^{™} 96-well Shuttle System (Lonza, Allendale, NJ). The guide/Cas9 protein nucleoprotein complexes were dispensed in a 2.5 µl final volume into individual wells of a 96-well plate. The suspended T cells were pelleted by centrifugation for 10 minutes at 200 x g, washed with calcium and magnesium-free phosphate buffered saline (PBS), and the cell pellet was resuspended in 10 ml of calcium and magnesium-free PBS. The cells were counted using the Countess^{®} II Automated Cell Counter (Life Technologies; Grand Island, NY).

2.2e7 cells were transferred to a 15 ml conical tube and pelleted. The PBS was aspirated, and the cells resuspended in Nucleofector^{™} P4 (Lonza, Allendale, NJ) solution to a density of 1e7 cells/ml per sample. 20 µl of the cell suspension was then added to each well containing 2.5 µl of the Cas12a guide/nucleoprotein complexes, and the entire volume from each well was transferred to a well of a 96-well Nucleocuvette^{™} Plate (Lonza, Allendale, NJ). The plate was loaded onto the Nucleofector^{™} 96-well Shuttle (Lonza, Allendale, NJ) and cells nucleofected using the CA137 Nucleofector^{™} program (Lonza, Allendale, NJ). Post-nucleofection, 77.5 µl of ImmunoCult-XF complete medium supplemented with IL-2 (100 units/mL) was added to each well, and the entire volume of transfected cell suspension was transferred to a 96-well cell culture plate containing 100 µl pre-warmed ImmunoCult-XF complete medium supplemented with IL-2 (100 units/mL). The plate was transferred to a tissue culture incubator and maintained at 37°C in 5% CO₂ for 48 hours before downstream analysis.

### Example 4

### Targeting of PDCD1 with CABRNT/Cas9 Nucleoprotein Complexes

This Example describes the design and use of CABRNT guides to target the programmed cell death 1 (PDCD1) gene in human T cells.

### A. Designing the PDCD1 target CABRNT guides

A 20-nucleotide sequence upstream (in a 5' direction) of a 5'- NGG PAM motif in the human PDCD1 gene was selected for targeting (SEQ ID NO: 61). Target selection criteria included, but were not limited to, homology to other regions in the genome; percent G-C content; melting temperature; and presence of homopolymer within the spacer. The identified spacer and PAM (underline) sequence are shown in Table 5.

| **Table 5 PDCD1 Target Sequence** | | |
|---|---|---|
| **SEQ ID NO:** | **Motif Location** | **Spacer and PAM** |
| SEQ ID NO: 61 | chr2:241858786-241858808 | GTCTGGGCGGTGCTACAACTGGG |

Three CABRNTs were designed for SEQ ID NO: 61, wherein an abasic site with a deoxyribose was used at positions 4, 5, or 6 (counting from the 5' edge of the spacer). The abasic spacer sequences were appended to the 5' end of a CRISPR repeat sequence (SEQ ID NO: 65), and sequences were provided to a commercial manufacturer for synthesis. The PDCD1 CABRNT sequences are shown in Table 6. For the sequences provided in Table 6, RNA bases are proceeded by an "r," and the deoxyribose abasic sites are shown as "dN."

| **Table 6 PDCD1 CABRNTs** | |
|---|---|
| **SEQ ID NO:** | **Sequence** |
| SEQ ID NO: 7 | rGrUrCdNrGrGrGrCrGrGrUrGrCrUrArCrArArCrUrGrUrUrUrUrArGrArGr GrArUrUrGrCrU |
| SEQ ID NO: 8 | rGrUrCrUdNrGrGrCrGrGrUrGrCrUrArCrArArCrUrGrUrUrUrUrArGrArGr GrArUrUrGrCrU |
| SEQ ID NO: 9 | rGrUrCrUrGdNrGrCrGrGrUrGrCrUrArCrArArCrUrGrUrUrUrUrArGrArGr GrArUrUrGrCrU |

A crRNA (SEQ ID NO: 5) and a chRDNA (SEQ ID NO: 6) guide were also designed for targeting of SEQ ID NO: 61; a ch-acr (SEQ ID NO: 2) was also designed. Sequences were provided to commercial manufacturers for synthesis. Then, individual guide/Cas9 protein nucleoprotein complexes were prepared as described in Example 2 and transfected into primary T cells as described in Example 3.

### B. Determining genome editing efficiency

### (1) Target dsDNA sequence generation for deep sequencing

gDNA was isolated from the nucleofected primary T cells 48 hours after transfection using the guide/Cas9 nucleoprotein complexes and 50µL QuickExtract^{™} DNA extraction solution (Epicentre, Madison, WI) per well, followed by incubation at 37°C for 10 minutes, 65°C for 6 minutes, and 95°C for 3 minutes to stop the reaction. The isolated gDNA was diluted with 50µL sterile water and samples was stored at -80°C.

Using the isolated gDNA, a first PCR was performed using Q5 Hot Start High-Fidelity 2X Master Mix (New England Biolabs, Ipswich, MA) at 1x concentration, primers designed to amplify the region around the PDCD1 target were used at 0.5µM each (SEQ ID NO: 66 and SEQ ID NO: 67), 3.75µL of gDNA in a final volume of 10µL and amplification at 98°C for 1 minute, 35 cycles of 10s at 98°C, 20 seconds at 60°C, 30 seconds at 72°C, and a final extension at 72°C for 2 minutes. The PCR reaction were diluted 1: 100 in water.

A unique set of index primers for a "barcoding" PCR were used to facilitate multiplex sequencing for each sample. Barcoding PCRs were performed using a reaction mix comprising Q5 Hot Start High-Fidelity 2X Master Mix (New England Biolabs, Ipswich, MA) at 1x concentration, primers at 0.5 µM each, and 1µL of 1:100 diluted first PCR in a final volume of 10µL. The reaction mix were amplified as follows: 98°C for 1 minute; followed 12 cycles of 10s at 98°C, 20 seconds at 60°C, and 30 seconds at 72°C; with a final extension reaction at 72°C for 2 minutes.

### (2) SPRIselect clean-up

The PCR reactions were pooled and transferred into a single microfuge tube for SPRIselect (Beckman Coulter, Pasadena, CA) bead-based cleanup of amplicons for sequencing.

To the amplicon, 0.9x volumes of SPRIselect beads were added, mixed, and incubated at room temperature for 10 minutes. The microfuge tube was placed on magnetic tube stand (Beckman Coulter, Pasadena, CA) until the solution clears. Supernatant was removed and discarded, the residual beads were washed with 1 volume of 85% ethanol, and the beads were incubated at room temperature for 30 seconds. After incubation, ethanol was aspirated, and the beads air-dried at room temperature for 10 minutes. The microfuge tube was removed from the magnetic stand and 0.25x volumes of Qiagen EB buffer (Qiagen, Venlo, Netherlands) added to the beads, mixed vigorously, and incubated for 2 minutes at room temperature. The microfuge tube was returned to the magnet, incubated until the solution has cleared, and supernatant containing the purified amplicons dispensed into a clean microfuge tube. The purified amplicons were quantified using the Nanodrop^{™} 2000 System (Thermo Scientific, Wilmington DE) and library quality analyzed using the Fragment Analyzer^{™} System (Advanced Analytical Technologies, Ames, IA) and the DNF-910 dsDNA Reagent Kit (Advanced Analytical Technologies, Ames, IA).

### (3) Deep sequencing set-up

The pooled amplicons were normalized to a 4nM concentration as calculated from the Nanodrop 2000 System values and the average size of the amplicons. The library was analyzed on MiSeq Sequencer (Illumina, San Diego, CA) with MiSeq Reagent Kit v2 (Illumina, San Diego, CA) for 300 cycles with two 151-cycle paired-end runs plus two 8-cycle index reads.

### (4) Deep sequencing data analysis

The identities of products in the sequencing data were determined based on the index barcode sequences adapted onto the amplicons in the barcoding PCR. A computational script was used to process the MiSeq data that executes, for example, the following tasks:
a. Reads were aligned to the human genome (build GRCh38/38) using Bowtie (bowtie-bio.sourceforge.net/index.shtml) software;
b. Aligned reads were compared to the expected wild-type PDCD1 locus sequence, and reads not aligning to any part of the PDCD1 locus discarded;
c. Reads matching wild-type PDCD1 sequence were tallied;
d. Reads with indels (insertion or deletion of bases) were categorized by indel type and tallied; and
e. Total indel reads were divided by the sum of wild-type reads and indel reads to give percent-mutated reads.

Through the identification of indel sequences at regions targeted by the guide/Cas9 protein nucleoprotein complexes, the resulting genome editing efficiency of the crRNA/ch-acr/Cas9 protein nucleoprotein complexes, chRDNA/ch-acr/Cas9 nucleoprotein complex, and CABRNT/ch-acr/Cas9 nucleoprotein complex was determined. The results of the in-cell cleavage experiment are shown in Table 7.

| **Table 7 Percent Indels Detected with CABRNT Protein Nucleoprotein Complexes** | | |
|---|---|---|
| **SEQ ID NO:** | **Average** | **StDev** |
| SEQ ID NO: 5 | 95.000% | 0.100% |
| SEQ ID NO: 6 | 93.467% | 0.115% |
| SEQ ID NO: 7 | 94.400% | 0.265% |
| SEQ ID NO: 8 | 90.533% | 0.058% |
| SEQ ID NO: 9 | 30.067% | 0.666% |

| | | |
|---|---|---|
| StDev=standard deviation; n=3 | | |

The data presented in Table 7 demonstrate that CABRNTs are capable of on editing rates comparable to an all-RNA guide (*see* Table 7, SEQ ID NO: 5 compared to SEQ ID NO: 7; or SEQ ID NO: 5 compared to SEQ ID NO: 8) and also comparable to chRDNA guides (*see* Table 7, SEQ ID NO: 6 compared to SEQ ID NO: 7; or SEQ ID NO: 6 compared to SEQ ID NO: 8).

### Example 5

### SITE-Seq Assay Evaluation of CABRNT Reagents

The following Example describes the use of the biochemical SITE-Seq^{®} (Caribou Biosciences, Inc., Berkeley, CA) off-target assay and in cell validation to characterize the specificity of guide reagents. The method set forth herein was adapted from Cameron et al. (Nature Meth., 2017, 14:600-606).

### A. SITE-Seq assay

Human primary T cells were grown as described in Example 1. After expansion of cells, in 50 ml conical tubes, high molecular weight genomic DNA (gDNA) was extracted from the human primary T cells using the Blood and Cell Culture DNA Maxi Kit (Qiagen, Hilden, Germany) according to the manufacturer's protocol.

Nucleoprotein complexes for both crRNA (SEQ ID NO: 5) and three CABRNTs (SEQ ID NO: 7, SEQ ID NO: 8, and SEQ ID NO: 9) were mixed at an equimolar amounts with ch-acr (SEQ ID NO: 2), and nucleoprotein complexes was were prepared by incubating each guide component at 95°C for 2 minutes, then each sample was allowed to slowly come to room temperature over 5 minutes. Each guide was then combined with Cas9 protein in a 3:1 guide:Cas9 molar ratio and incubated at 37°C for 10 minutes in cleavage reaction buffer (20 mM HEPES, pH 7.4, 150 mMKCl, 10 mM MgCl2, 5% glycerol). In a 96-well plate format, 10 µg of gDNA was treated with 0.2 pmol, 0.8 pmol, 3.2 pmol, and 12.8 pmol of each nucleoprotein complex in 50 µL total volume in cleavage reaction buffer. Each cleavage reaction was performed in triplicate. Negative control reactions were assembled in parallel and did not include any nucleoprotein complex. gDNA was treated with nucleoprotein complexes for 4 hours at 37°C. Library preparation and sequencing were done essentially as described by Cameron et al. (Nature Meth., 2017, 14:600-606), using the Illumina NextSeq platform (Illumina, San Diego, CA), and ~3 million reads were obtained for each sample. Any SITE-Seq assay off-target sites without off-target motifs located within 1 nucleotide of the cut-site were considered false-positives and discarded.

The number of recovered targeted sites from the SITE-Seq assay off-target experiment is shown in Table 8.

| **Table 8 Number of SITE-Seq Assay Recovered Sites** | | | | |
|---|---|---|---|---|
| **SEQ ID NO:** | **Nucleoprotein Complex Concentration** | | | |
| | **4nM** | **16nM** | **64nM** | **256nM** |
| SEQ ID NO: 5 | 10 | 29 | 193 | 544 |
| SEQ ID NO: 7 | 2 | 2 | 9 | 23 |
| SEQ ID NO: 8 | 3 | 3 | 4 | 7 |
| SEQ ID NO: 9 | 2 | 2 | 3 | 4 |

The data presented in Table 8 demonstrate that CABRNT reagents recover fewer number of SITE-Seq assay off-target sites compared to crRNA reagents for the same target site *(compare* Table 8, SEQ ID NO: 5 to either SEQ ID NO: 7, SEQ ID NO: 8, or SEQ ID NO: 9).

### B. In-cell validation of the SITE-Seq assay off-target recovered sites

To measure indel frequencies at SITE-Seq assay off-target sites shown in Table 8, targeted deep sequencing analyses were performed on a subset of the sites recovered in the crRNA samples (SEQ ID NO: 5). Three off-target sites (SEQ ID NO: 62, SEQ ID NO: 63, and SEQ ID NO: 64) were selected for evaluation of in-cell off-target editing rates with crRNA and CABRNT reagents. Forward and reverse amplicon primers were designed for each off-target site and ordered from a commercial manufacturer.

Human primary T cells were cultured as described in Example 1. crRNA (SEQ ID NO: 5) and three CABRNTs (SEQ ID NO: 7, SEQ ID NO: 8, and SEQ ID NO: 9) nucleoprotein complexes were assembled and nucleofected as described in Example 3. 48 hours post-nucleofection cells were harvested. Sequencing of the three off-target sites was carried out essentially as described in Example 4. An un-transfected pool of cells was used as a wild-type reference. Mutant reads (% indels) were defined as any non-reference variant calls within 20 base pairs (bp) of the cut site. Sites were discarded that had low sequencing coverage (<1,000 reads in the combined, Cas9-treated samples or <200 reads in the reference samples) or >2% variant calls in the reference samples. Sites were tallied as cellular off-targets if they accumulated >0.1% mutant reads in the combined, Cas9-treated samples. The results of the targeted deep sequencing of recovered SITE-Seq assay off-target sites is presented in Table 9.

| **Table 9 In-Cell Validation of SITE-Seq Assay Off- Target Recovered Sites** | | | | | | |
|---|---|---|---|---|---|---|
| **SEQ ID NO:** | **Off-Target 1 SEQ ID NO: 62** | | **Off-Target 2 SEQ ID NO: 63** | | **Off-Target 3 SEQ ID NO: 64** | |
| | **Average** | **StDev** | **Average** | **StDev** | **Average** | **StDev** |
| SEQ ID NO: 5 | 28.000% | 0.608% | 1.407% | 0.125% | 0.375% | 0.049% |
| SEQ ID NO: 7 | 0.406% | 0.090% | <0.1% | 0.000% | <0.1% | 0.000% |
| SEQ ID NO: 8 | <0.1% | 0.000% | 0.335% | 0.081% | <0.1% | 0.000% |
| SEQ ID NO: 9 | <0.1% | 0.007% | 1.307% | 0.131% | <0.1% | 0.000% |

| | | | | | | |
|---|---|---|---|---|---|---|
| StDev=standard deviation; n=3 | | | | | | |

The data presented in Table 9 demonstrate that CABRNT/ch-acr/Cas9 protein nucleoprotein complexes have improved specificity (e.g., lower off-target editing rates) at SITE-Seq assay identified off-target sites when compared to the rate of off-target of crRNA/ch-acr/Cas9 *(compare* Table 9, SEQ ID NO: 5 to either SEQ ID NO: 7, SEQ ID NO: 8, or SEQ ID NO: 9).

### Example 6

### Editing of CABRNT with an Abasic Ribose Site or an Abasic Deoxyribose Site

This Example describes the comparison of CABRNT reagents with abasic sites comprising a ribose or deoxyribose sugar.

### A. In silico design of CABRNTs

CABRNTs for the PDCD1 target site (SEQ ID NO: 61) were designed with the abasic site at position 4, 5, or 6 (counting from the 5' edge of the spacer), where the abasic site comprised either a ribose or deoxyribose sugar. The abasic spacer sequences were appended to the 5' end of a CRISPR repeat sequence (SEQ ID NO: 65), and the sequences were provided to a commercial manufacturer for synthesis. Description of the ribose and deoxyribose CABRNTs is presented in Table 10.

| **Table 10 Number of SITE-Seq Assay Off-Site Assay Recovered Sites** | | |
|---|---|---|
| **SEQ ID NO:** | **Abasic Site Position** | **Abasic Sugar** |
| SEQ ID NO: 7 | 4 | DNA |
| SEQ ID NO: 10 | 4 | RNA |
| SEQ ID NO: 8 | 5 | DNA |
| SEQ ID NO: 11 | 5 | RNA |
| SEQ ID NO: 9 | 6 | DNA |
| SEQ ID NO: 12 | 6 | RNA |

### B. Cell transfection and analysis

Individual guide/Cas9 protein nucleoprotein complexes for screening were prepared essentially as described in Example 2. The nucleoprotein complexes were transfected into primary T cells as described in Example 3. The resulting genome editing efficiency of the crRNA/ch-acr/Cas9 protein nucleoprotein complexes and CABRNT/ch-acr/Cas9 protein nucleoprotein complexes was determined as described in Example 4. Percent indels detected from each CABRNT design are presented in Table 11.

| **Table 11 In Cell Editing of PDCD1 CABRNTs** | | |
|---|---|---|
| **SEQ ID NO:** | **On-Target** | |
| | **Average** | **StDev** |
| SEQ ID NO: 5 | 97.2% | 1.9% |
| SEQ ID NO: 7 | 96.6% | 2.0% |
| SEQ ID NO: 10 | 95.6% | 2.0% |
| SEQ ID NO: 8 | 94.3% | 0.5% |
| SEQ ID NO: 11 | 89.0% | 0.7% |
| SEQ ID NO: 9 | 55.2% | 17.7% |
| SEQ ID NO: 12 | 50.5% | 1.7% |

| | | |
|---|---|---|
| StDev=standard deviation; n=3 | | |

The data presented in Table 11 demonstrates that CABRNT comprising an abasic ribose sites or an abasic deoxyribose sites are capable of in-cell editing.

### Example 7

### CABRNT Abasic Position Screen

This Example describes the design and screening of CABRNTs with an individual abasic site at each position in the spacer of a T cell receptor alpha constant (TRAC) or beta-2-microglobulin (β2M) target sequence.

### A. In silico CABRNT design

A 20-nucleotide sequence upstream (in a 5' direction) of a 5'- NGG PAM motif was selected from the genomic regions of TRAC and β2M. The selected TRAC and β2M target sites, with the PAM underlined, are shown in Table 12.

| **Table 12 TRAC and β2M Targets** | | | |
|---|---|---|---|
| **SEQ ID NO:** | **Gene** | **Coordinates (hg38)** | **Sequence** |
| SEQ ID NO: 68 | TRAC | chr14:22550579-22550601 | TTCGGAACCCAATCACTGACAGG |
| SEQ ID NO: 69 | β2M | chr15:44711542-44711564 | GGCCACGGAGCGAGACATCTCGG |

CABRNTs for the TRAC target (SEQ ID NO: 14 - SEQ ID NO: 33) and the β2M target (SEQ ID NO: 35 - SEQ ID NO: 54) were designed where a single abasic deoxyribose site was used at each position in the 20-nucleotide spacer sequence; if the abasic site occurred at the 20^{th} position (i.e. SEQ ID NO: 14), a terminal guanine was added for synthesis considerations. A crRNA control was designed for the TRAC target (SEQ ID NO: 13) and the β2M target (SEQ ID NO: 34) with no abasic positions in the spacer. The spacer sequences were appended to the 5' end of a CRISPR repeat sequence (SEQ ID NO.65), and sequences were provided to a commercial manufacturer for synthesis. The position of each abasic deoxyribose sites in the spacer (counting from the 5' edge of the spacer) is presented in Table 13.

| **Table 13 TRAC and β2M Abasic Positions** | | | |
|---|---|---|---|
| **TRAC Target** | | **β2M Target** | |
| **SEQ ID NO:** | **Abasic Position** | **SEQ ID NO:** | **Abasic Position** |
| SEQ ID NO: 14 | 20 | SEQ ID NO: 35 | 20 |
| SEQ ID NO: 15 | 19 | SEQ ID NO: 36 | 19 |
| SEQ ID NO: 16 | 18 | SEQ ID NO: 37 | 18 |
| SEQ ID NO: 17 | 17 | SEQ ID NO: 38 | 17 |
| SEQ ID NO: 18 | 16 | SEQ ID NO: 39 | 16 |
| SEQ ID NO: 19 | 15 | SEQ ID NO: 40 | 15 |
| SEQ ID NO: 20 | 14 | SEQ ID NO: 41 | 14 |
| SEQ ID NO: 21 | 13 | SEQ ID NO: 42 | 13 |
| SEQ ID NO: 22 | 12 | SEQ ID NO: 43 | 12 |
| SEQ ID NO: 23 | 11 | SEQ ID NO: 44 | 11 |
| SEQ ID NO: 24 | 10 | SEQ ID NO: 45 | 10 |
| SEQ ID NO: 25 | 9 | SEQ ID NO: 46 | 9 |
| SEQ ID NO: 26 | 8 | SEQ ID NO: 47 | 8 |
| SEQ ID NO: 27 | 7 | SEQ ID NO: 48 | 7 |
| SEQ ID NO: 28 | 6 | SEQ ID NO: 49 | 6 |
| SEQ ID NO: 29 | 5 | SEQ ID NO: 50 | 5 |
| SEQ ID NO: 30 | 4 | SEQ ID NO: 51 | 4 |
| SEQ ID NO: 31 | 3 | SEQ ID NO: 52 | 3 |
| SEQ ID NO: 32 | 2 | SEQ ID NO: 53 | 2 |
| SEQ ID NO: 33 | 1 | SEQ ID NO: 54 | 1 |

### B. Cell transfection and analysis

Individual guide/Cas9 protein nucleoprotein complexes for screening were prepared essentially as described in Example 2. The nucleoprotein complexes were transfected into primary T cells as described in Example 3. The resulting genome editing efficiency of the crRNA/ch-acr/Cas9 protein nucleoprotein complexes and CABRNT/ch-acr/Cas9 protein nucleoprotein complexes was determined as described in Example 4. Percent indels detected from each CABRNT design are presented in Table 14.

| **Table 14** | | | | | |
|---|---|---|---|---|---|
| **In Cell Editing of TRAC and β2M CABRNTs** | | | | | |
| **TRAC** | | | **β2M** | | |
| SEQ ID NO: | Average | StDev | SEQ ID NO: | Average | StDev |
| SEQ ID NO: 13 | 97.63% | 0.12% | SEQ ID NO: 34 | 96.47% | 0.67% |
| SEQ ID NO: 14 | 10.77% | 0.40% | SEQ ID NO: 35 | 13.30% | 0.42% |
| SEQ ID NO: 15 | 15.17% | 1.48% | SEQ ID NO: 36 | 3.91% | 0.08% |
| SEQ ID NO: 16 | 1.85% | 0.08% | SEQ ID NO: 37 | 71.80% | 0.46% |
| SEQ ID NO: 17 | 0.06% | 0.02% | SEQ ID NO: 38 | <0.1% | 0.00% |
| SEQ ID NO: 18 | 0.27% | 0.13% | SEQ ID NO: 39 | 0.60% | 0.07% |
| SEQ ID NO: 19 | 0.14% | 0.02% | SEQ ID NO: 40 | 0.45% | 0.04% |
| SEQ ID NO: 20 | 2.47% | 0.54% | SEQ ID NO: 41 | 1.04% | 0.25% |
| SEQ ID NO: 21 | 65.50% | 1.39% | SEQ ID NO: 42 | 28.67% | 1.23% |
| SEQ ID NO: 22 | 10.01% | 0.08% | SEQ ID NO: 43 | 1.95% | 0.22% |
| SEQ ID NO: 23 | 20.43% | 0.50% | SEQ ID NO: 44 | 66.13% | 2.71% |
| SEQ ID NO: 24 | 65.90% | 0.82% | SEQ ID NO: 45 | 33.17% | 2.01% |
| SEQ ID NO: 25 | 61.43% | 0.23% | SEQ ID NO: 46 | 93.97% | 1.72% |
| SEQ ID NO: 26 | 37.17% | 0.67% | SEQ ID NO: 47 | 55.73% | 1.21% |
| SEQ ID NO: 27 | 57.43% | 1.00% | SEQ ID NO: 48 | 23.70% | 2.66% |
| SEQ ID NO: 28 | 65.83% | 4.34% | SEQ ID NO: 49 | 85.33% | 8.00% |
| SEQ ID NO: 29 | 26.37% | 1.63% | SEQ ID NO: 50 | 38.33% | 2.17% |
| SEQ ID NO: 30 | 20.70% | 1.01% | SEQ ID NO: 51 | 90.20% | 0.95% |
| SEQ ID NO: 31 | 95.70% | 0.26% | SEQ ID NO: 52 | 91.17% | 0.40% |
| SEQ ID NO: 32 | 96.87% | 0.15% | SEQ ID NO: 53 | 92.30% | 0.78% |
| SEQ ID NO: 33 | 96.87% | 0.29% | SEQ ID NO: 54 | 93.83% | 1.53% |

| | | | | | |
|---|---|---|---|---|---|
| StDev=standard deviation; n=3 | | | | | |

The data presented in Table 14 demonstrate that abasic sites can be utilized at many positions in the CABRNT sequences.

### C. Specificity Evaluation of Abasic CABRNTs

An off-target site (SEQ ID NO: 76) of the β2M target (SEQ ID NO: 69) was identified by the SITE-Seq assay and the method described in Example 5. Forward and reverse amplicon primers were designed for the off-target site and ordered from a commercial manufacturer. The off-target region was amplified from cell lysate from the β2M abasic position screen (SEQ ID NO: 34-54). The results of the targeted deep sequencing of β2M off-target site is presented in Table 15.

| **Table 15** | | |
|---|---|---|
| **β2M Off-Target Editing** | | |
| **SEQ ID NO:** | **β2M Off Target** | |
| | **Average** | **StDev** |
| SEQ ID NO: 13 | 2.080% | 0.136% |
| SEQ ID NO: 14 | <0.1% | 0.000% |
| SEQ ID NO: 15 | <0.1% | 0.000% |
| SEQ ID NO: 16 | <0.1% | 0.000% |
| SEQ ID NO: 17 | <0.1% | 0.000% |
| SEQ ID NO: 18 | <0.1% | 0.000% |
| SEQ ID NO: 19 | <0.1% | 0.000% |
| SEQ ID NO: 20 | <0.1% | 0.000% |
| SEQ ID NO: 21 | <0.1% | 0.000% |
| SEQ ID NO: 22 | <0.1% | 0.000% |
| SEQ ID NO: 23 | <0.1% | 0.000% |
| SEQ ID NO: 24 | <0.1% | 0.000% |
| SEQ ID NO: 25 | <0.1% | 0.000% |
| SEQ ID NO: 26 | <0.1% | 0.000% |
| SEQ ID NO: 27 | <0.1% | 0.000% |
| SEQ ID NO: 28 | <0.1% | 0.000% |
| SEQ ID NO: 29 | <0.1% | 0.021% |
| SEQ ID NO: 30 | 0.139% | 0.073% |
| SEQ ID NO: 31 | <0.1% | 0.000% |
| SEQ ID NO: 32 | <0.1% | 0.000% |
| SEQ ID NO: 33 | <0.1% | 0.017% |

| | | |
|---|---|---|
| StDev=standard deviation; n=3 | | |

The data presented in Table 15 demonstrate that abasic sites at multiple individual positions in the CABRNT sequences can reduce editing at an off-target sequence (Compare SEQ ID NO: 13 to SEQ ID NO: 14 - SEQ ID NO: 29 or SEQ ID NO: 31 - SEQ ID NO: 33).

### Example 8

### chRDNA-CABRNT Guide Reagents

This Example describes the design and use of guides comprising RNA and DNA (chRDNA) and an abasic site (CABRNT).

### A. In silico design of chRDNA-CABRNTs

A 20-nucleotide sequence upstream (in a 5' direction) of a 5'- NGG PAM motif was selected for the Adeno-Associated Virus Integration Site 1 (AAVS1) genomic region. The selected AAVS1 target sites, with the PAM underlined, are shown in Table 16.

| **Table 16** | | | |
|---|---|---|---|
| **AAVS1 Target Site** | | | |
| **SEQ ID NO:** | **Gene** | **Coordinates (hg38)** | **Sequence** |
| SEQ ID NO: 70 | AAVS1 | chr19:55115749-55115771 | GGGGCCACTAGGGACAGGATTGG |

For the selected AAVS1 target sites, guides were designed with varying amounts of DNA and RNA in the spacer sequence along with an abasic site. The abasic spacer sequences were appended to a CRISPR repeat sequence (SEQ ID NO.65), and sequences were provided to a commercial manufacturer for synthesis. For the sequences presented in Table 17, DNA bases are shown with standard IUPAC codes, RNA bases are proceeded by an "r," and the abasic sites are shown as "dN."

| **Table 17** | | |
|---|---|---|
| **chRDNA-CABRNT Guides** | | |
| **SEQ ID NO:** | **Reagent Name** | **Sequence** |
| SEQ ID NO: 55 | crRNA | rGrGrGrGrCrCrArCrUrArGrGrGrArCrArGrGrArUrGrUrU rUrUrArGrArGrGrArUrUrGrCrU |
| SEQ ID NO: 56 | chRDNA-CABRNT design - 1 | GGdNGCCACTArGrGrGrArCrArGrGrArUrGrUrUrUrUrA rGrArGrGrArUrUrGrCrU |
| SEQ ID NO: 57 | chRDNA-CABRNT design - 2 | GGdNGCrCrArCrUrArGrGrGrArCrArGrGrArUrGrUrUrUr UrArGrArGrGrArUrUrGrCrU |
| SEQ ID NO: 58 | chRDNA-CABRNT design - 3 | rGrGdNGCCACTArGrGrGrArCrArGrGrArUrGrUrUrUrUr ArGrArGrGrArUrUrGrCrU |
| SEQ ID NO: 59 | chRDNA-CABRNT design - 4 | GGdNrGCCACTAGGrGrArCrArGrGrArUrGrUrUrUrAr GrArGrGrArUrUrGrCrU |
| SEQ ID NO: 60 | chRDNA-CABRNT design - 5 | rGGdNrGCCACTrArGrGrGArCrAGGrArUrGrUrUrUrUrA rGrArGrGrArUrUrGrCrU |

### B. Off-target site identification

Off-target sites (SEQ ID NO: 71 - SEQ ID NO: 75) for the AAVS1 target (SEQ ID NO: 70) were identified by the SITE-Seq assay and the method described in Example 5. Forward and reverse amplicon primers were designed for the off-target site and ordered from a commercial manufacturer. The sequence and chromosomal location of the AAVS1 off-targets are presented in Table 18.

| **Table 18** | | | |
|---|---|---|---|
| **AAVS1 Off-Target Sites** | | | |
| **SEQ ID NO:** | **Gene** | **Coordinates (hg38)** | **Sequence** |
| SEQ ID NO: 71 | AAVS1 off- 1 | chr6:36797687-36797709 | GGGACCATCAGGGACAGGAT GGG |
| SEQ ID NO: 72 | AAVS1 off-2 | chr19:16064177-16064199 | GGGGCTTCTAAGGACAGGAT GGG |
| SEQ ID NO: 73 | AAVS1 off-3 | chr5:170084409-170084431 | GAGGCCACCAGGGACAGGCT GGG |
| SEQ ID NO: 74 | AAVS1 off-4 | chr13:105960563-105960585 | GGGGCCAATTAGGACAGGAT GGG |
| SEQ ID NO: 75 | AAVS1 off-5 | chr12:107092487-107092509 | ATGGCCACTAAGGACAGGAA AGG |

### C. Cell transfection and analysis

Individual guide/Cas9 protein nucleoprotein complexes for screening were prepared essentially as described in Example 2. The nucleoprotein complexes were transfected into primary T cells as described in Example 3. The resulting genome editing efficiency of the crRNA/ch-acr/Cas9 protein nucleoprotein complexes and chRDNA-CABRNT/ch-acr/Cas9 protein nucleoprotein complexes was determined as described in Example 4. Percent indels detected from each guide/Cas9 protein nucleoprotein complexes at the AAVS-1 on-target and off-target sites are presented in Table 19 and Table 20.

| **Table 19** | | | | | | |
|---|---|---|---|---|---|---|
| **chRDNA-CABRNT Editing** | | | | | | |
| **SEQ ID NO:** | **On-Target SEQ ID NO: 70** | | **Off_1 SEQ ID NO: 71** | | **Off_2 SEQ ID NO: 72** | |
| | Average | StDev | Average | StDev | Average | StDev |
| SEQ ID NO: 55 | 93.40% | 0.82% | 73.18% | 0.23% | 36.88% | 0.78% |
| SEQ ID NO: 56 | 54.25% | 0.42% | <0.1% | 0.01% | <0.1% | 0.02% |
| SEQ ID NO: 57 | 68.35% | 0.81% | <0.1% | 0.01% | <0.1% | 0.01% |
| SEQ ID NO: 58 | 69.30% | 0.45% | <0.1% | 0.01% | <0.1% | 0.02% |
| SEQ ID NO: 59 | 60.20% | 0.83% | <0.1% | 0.01% | <0.1% | 0.01% |
| SEQ ID NO: 60 | 41.33% | 0.39% | <0.1% | 0.01% | <0.1% | 0.01% |

| | | | | | | |
|---|---|---|---|---|---|---|
| StDev=standard deviation; n=3 | | | | | | |

| **Table 20** | | | | | | |
|---|---|---|---|---|---|---|
| **chRDNA-CABRNT Editing** | | | | | | |
| **SEQ ID NO:** | **Off_3 SEQ ID NO: 73** | | **Off_4 SEQ ID NO: 74** | | **Off_5 SEQ ID NO: 75** | |
| | Average | StDev | Average | StDev | Average | StDev |
| SEQ ID NO: 55 | 15.53% | 0.73% | 15.63% | 0.54% | 10.13% | 0.63% |
| SEQ ID NO: 56 | <0.1% | 0.00% | <0.1% | 0.01% | <0.1% | 0.01% |
| SEQ ID NO: 57 | 0.11% | 0.03% | <0.1% | 0.00% | <0.1% | 0.03% |
| SEQ ID NO: 58 | <0.1% | 0.00% | <0.1% | 0.00% | <0.1% | 0.01% |
| SEQ ID NO: 59 | <0.1% | 0.01% | <0.1% | 0.00% | <0.1% | 0.00% |
| SEQ ID NO: 60 | <0.1% | 0.01% | <0.1% | 0.00% | <0.1% | 0.00% |

| | | | | | | |
|---|---|---|---|---|---|---|
| StDev=standard deviation; n=3 | | | | | | |

The data presented in Table 19 and Table 20 demonstrate that chRDNA-CABRNT guides are capable of robust on-target editing and are also capable of reduced off-target editing compared to an all RNA guide (compare editing values of SEQ ID NO: 55 at target SEQ ID NO: 71 to editing values of SEQ ID NO: 56 - SEQ ID NO: 60 at SEQ ID NO: 71).

### Example 9

### Cloning of AAV Donor Cassette. AAV Production and AAV Transduction of Primary Cells

This Example describes the design and cloning of a DNA donor element cassette into an AAV vector, production of AAV, delivery of Cas9 CABRNT guide/nucleoprotein complexes into primary cells, and transduction of primary cells with AAV for site-specific integration of a CAR expression cassette into primary cells.

AAV can be engineered to deliver DNA donor elements to mammalian cells. If AAV delivery is combined with a genomic cleavage event, and the DNA donor element in the AAV is flanked by homology arms, the DNA donor element can be seamlessly inserted into the genomic cut site by HDR, *see, e.g.,* Eyquem et al. (Nature, 2017, 543: 113-117).

### A. In silico design of AAV donor element cassettes and rAAV production

The design of CAR receptors has been described. *See, e.g.,* Kochenderfer et al. (J. Immunotherapy, 2009, 32:689-702). The CAR construct is designed to contain an N-terminal secretion signal (CD8a signal peptide), an scFv portion specific for BCMA, followed by a CD8 hinge region and transmembrane, a 4-1BB effector region, a CD3ζ effector region, and a c-terminal BGH polyadenylation signal sequence. A mammalian promoter sequence is inserted upstream of the CAR element. In order to site-specifically insert CAR donor elements into the host cell genome after site-specific cleavage, a target site is chosen in the endogenous TRAC locus (SEQ ID NO: 68). Then, 500 bp long homology arms 5' and 3' of the cut site are identified. The 5' and 3' homology arms are appended to the end of the CAR donor elements, wherein the DNA donor elements are orientated in a reverse orientation *(i.e.,* 3' to 5') relative to the homology arms. The resulting sequence coding for CAR donor elements is provided to a commercial manufacturer for synthesis into a suitable recombinant AAV (rAAV) plasmid. The resulting rAAV plasmid containing CAR donor elements are provided to a commercial manufacturer for packaging into an AAV6 virus.

### B. Primary T cell transduction with rAAV

Primary activated T cells are obtained from PBMCs as described in Example 1. Cas9 CABRNT guide/nucleoprotein complexes targeting the genes encoding TRAC (SEQ ID NO: 31) are prepared as described in Example 2. T cells are transfected with TRAC (SEQ ID NO: 31)-targeting Cas9 CABRNT guide/nucleoprotein complexes, and between 1 minute and 4 hours after nucleofection, cells are infected with the AAV6 virus packaged with CAR donor sequence at an MOI of 1 × 10⁶. T cells are cultured in ImmunoCult-XF complete medium (STEMCELL Technologies, Cambridge, MA) supplemented with IL-2 (100 units/mL) for 24 hours after the transductions. The next day, the transduced T cells are transferred to 50 mL conical tubes and centrifuged at 300 x g for approximately 7-10 minutes to pellet cells. The supernatant is discarded, and the pellet is gently resuspended, and the T cells pooled in an appropriate volume of ImmunoCult-XF complete medium (STEMCELL Technologies, Cambridge, MA) supplemented with IL-2 (100 units/mL).

The enumerated T cells are resuspended at 1 × 10⁶ cells/mL in ImmunoCult-XF complete medium STEMCELL Technologies, Cambridge, MA) supplemented with IL-2 (100 units/mL), and plated into as many T-175 suspension flasks as required (max volume per flask is 250 mL).

### Example 10

### Editing and Specificity of CRISPR Accuracy via Analogs (CAVA) Guides

This Example describes the comparison of CAVA guides comprising deoxyuradine, inosine, or deoxyinosine analog bases in the spacer sequence with a guide comprising all RNA.

### A. In silico design of CAVA guides

CAVAs for the PDCD1 target site (SEQ ID NO: 61) were designed comprising deoxyuradine, inosine, or deoxyinosine analog bases at various positions in the spacer. The analog-containing spacer sequences were appended to the 5' end of a CRISPR repeat sequence (SEQ ID NO: 65), and the sequences were provided to a commercial manufacturer for synthesis. Sequences of the CAVA guides, analog type used, and location of the analogs in the spacer (counting from the 5' edge of the spacer) are presented in Table 21. For the sequences provided in Table 21, RNA bases are proceeded by an "r,"; the deoxyuradine bases are shown as "U"; inosine base are shown as "rI"; and deoxyinosine bases are shown as "I."

| **Table 21** | | | |
|---|---|---|---|
| **PDCD1 CAVA Guide** | | | |
| **SEQ ID NO:** | **Analog Type** | **Analog Location in Spacer** | **Sequence** |
| SEQ ID NO: 5 | n/a (crRNA) | - | rGrUrCrUrGrGrGrCrGrGrUrGrCrUr ArCrArArCrUrGrUrUrUrUrArGrAr GrGrArUrUrGrCrU |
| SEQ ID NO: 77 | deoxyuradine | 2 | rGUrCrUrGrGrGrCrGrGrUrGrCrUr ArCrArArCrUrGrUrUrUrUrArGrAr GrGrArUrUrGrCrU |
| SEQ ID NO: 78 | deoxyuradine | 4 | rGrUrCUrGrGrGrCrGrGrUrGrCrUr ArCrArArCrUrGrUrUrUrArGrAr GrGrArUrUrGrCrU |
| SEQ ID NO: 79 | deoxyuradine | 11 | rGrUrCrUrGrGrGrCrGrGUrGrCrUr ArCrArArCrUrGrUrUrUrArGrAr GrGrArUrUrGrCrU |
| SEQ ID NO: 80 | deoxyuradine | 14 | rGrUrCrUrGrGrGrCrGrGrUrGrCUr ArCrArArCrUrGrUrUrUrUrArGrAr GrGrArUrUrGrCrU |
| SEQ ID NO: 81 | deoxyuradine | 20 | rGrUrCrUrGrGrGrCrGrGrUrGrCrUr ArCrArArCUrGrUrUrUrUrArGrAr GrGrArUrUrGrCrU |
| SEQ ID NO: 82 | deoxyuradine | 2 and 11 | rGUrCrUrGrGrGrCrGrGUrGrCrUrA rCrArArCrUrGrUrUrUrUrArGrArGr GrArUrUrGrCrU |
| SEQ ID NO: 83 | deoxyuradine | 2, 4, 11, and 14 | rGUrCUrGrGrGrCrGrGUrGrCUrAr CrArArCrUrGrUrUrUrArGrArGr GrArUrUrGrCrU |
| SEQ ID NO: 84 | inosine | 3 and 4 | rGrUrIrIrGrGrGrCrGrGrUrGrCrUrA rCrArArCrUrGrUrUrUrUrArGrArGr GrArUrUrGrCrU |
| SEQ ID NO: 85 | inosine | 5 and 6 | rGrUrCrUrIrIrGrCrGrGrUrGrCrUrA rCrArArCrUrGrUrUrUrUrArGrArGr GrArUrUrGrCrU |
| SEQ ID NO: 86 | deoxyinosine | 2 and 3 | rGIIrUrGrGrGrCrGrGrUrGrCrUrAr CrArArCrUrGrUrUrUrArGrArGr GrArUrUrGrCrU |
| SEQ ID NO: 87 | deoxyinosine | 4 and 5 | rGrUrCIIrGrGrCrGrGrUrGrCrUrAr CrArArCrUrGrUrUrUrArGrArGr GrArUrUrGrCrU |
| SEQ ID NO: 88 | deoxyinosine | 6 and 7 | rGrUrCrUrGIIrCrGrGrUrGrCrUrAr CrArArCrUrGrUrUrUrUrArGrArGr GrArUrUrGrCrU |
| SEQ ID NO: 89 | deoxyinosine | 8 and 9 | rGrUrCrUrGrGrGIIrGrUrGrCrUrAr CrArArCrUrGrUrUrUrArGrArGr GrArUrUrGrCrU |
| SEQ ID NO: 90 | deoxyinosine | 10 and 11 | rGrUrCrUrGrGrGrCrGlIrGrCrUrAr CrArArCrUrGrUrUrUrUrArGrArGr GrArUrUrGrCrU |

### B. Cell transfection and analysis

Individual guide/Cas9 protein nucleoprotein complexes were prepared essentially as described in Example 2. The nucleoprotein complexes were transfected into primary T cells as described in Example 3. The resulting genome editing efficiency of the crRNA/ch-acr/Cas9 protein nucleoprotein complexes and CAVA/ch-acr/Cas9 protein nucleoprotein complexes was determined as described in Example 4 for the PDCD1 on-target (SEQ ID NO: 61) and off-target sites (SEQ ID NO: 62). Percent indels detected at the on and off-target sites from each CAVA design are presented in Table 22.

| **Table 22 CAVA Editing at PDCD1 On-Target and Off-Target Site** | | | | | |
|---|---|---|---|---|---|
| **SEQ ID NO:** | **Analog** | **On-Target SEQ ID NO: 61** | | **Off-Target SEQ ID NO: 62** | |
| | | **Average** | **StDev** | **Average** | **StDev** |
| SEQ ID NO: 5 | n/a | 92.95% | 0.92% | 23.15% | 1.34% |
| SEQ ID NO: 77 | deoxyuradine | 92.40% | 0.14% | 25.40% | 3.11% |
| SEQ ID NO: 78 | deoxyuradine | 92.20% | 0.42% | 7.13% | 0.53% |
| SEQ ID NO: 79 | deoxyuradine | 91.95% | 0.64% | 4.48% | 0.18% |
| SEQ ID NO: 80 | deoxyuradine | 93.80% | 0.42% | 12.70% | 0.14% |
| SEQ ID NO: 81 | deoxyuradine | 94.20% | 1.84% | 19.90% | 0.42% |
| SEQ ID NO: 82 | deoxyuradine | 94.45% | 1.06% | 2.45% | 0.06% |
| SEQ ID NO: 83 | deoxyuradine | 93.15% | 0.21% | 0.10% | 0.02% |
| SEQ ID NO: 84 | inosine | 55.35% | 1.20% | 7.79% | 0.06% |
| SEQ ID NO: 85 | inosine | 90.10% | 0.14% | <0.1% | - |
| SEQ ID NO: 86 | deoxyinosine | 74.45% | 1.06% | 0.35% | 0.02% |
| SEQ ID NO: 87 | deoxyinosine | 84.65% | 1.34% | 2.30% | 0.11% |
| SEQ ID NO: 88 | deoxyinosine | 90.70% | 0.00% | <0.1% | - |
| SEQ ID NO: 89 | deoxyinosine | 90.70% | 0.14% | <0.1% | - |
| SEQ ID NO: 90 | deoxyinosine | 85.50% | 3.11% | <0.1% | - |

| | | | | | |
|---|---|---|---|---|---|
| StDev=standard deviation; n=2 | | | | | |

The data presented in Table 22 demonstrate that CAVA guides are capable of robust on-target editing and are also capable of reduced off-target editing compared to an all RNA guide. CAVAs are capable of reduced off-target editing for guides comprising deoxyuradine *(compare* editing values of SEQ ID NO: 5 at target SEQ ID NO: 62 to SEQ ID NO: 82 at target SEQ ID NO: 62), inosine *(compare* editing values of SEQ ID NO: 5 at target SEQ ID NO: 62 to SEQ ID NO: 85 at target SEQ ID NO: 62), and deoxyinosine *(compare* editing values of SEQ ID NO: 5 at target SEQ ID NO: 62 to SEQ ID NO: 88-90 at target SEQ ID NO: 62).

As is apparent to one of skill in the art, modifications and variations of the above embodiments can be made without departing from the spirit and scope of this invention. Additional embodiments include the use of CABRNT, CAVA, and/or CABRNT-CAVA guides with other CRISPR-Cas systems such as, but are not limited to, Class 2 Type V and Class 1 Type I. Such modifications and variations are within the scope of this invention.

### Disclosed Items

**1.** A clustered regularly interspaced short palindromic repeats (CRISPR) polynucleotide comprising a spacer sequence comprising at least one nucleotide selected from the group consisting of a CRISPR abasic restricted nucleotide (CABRNT) and a CRISPR accuracy via analogs (CAVA) nucleotide.
**2.** The CRISPR polynucleotide of item 1, wherein the CRISPR polynucleotide comprises a CABRNT selected from an apurinic site or an apyrimidinic site.
**3.** The CRISPR polynucleotide of item 1, wherein the CRISPR polynucleotide is capable of forming a nucleoprotein complex with a CRISPR-associated (Cas) protein.
**4.** A guide, comprising:
   a CRISPR polynucleotide comprising a spacer sequence comprising at least one nucleotide selected from the group consisting of a CABRNT and a CAVA;
   wherein the spacer sequence is capable of binding a target nucleic acid sequence; and wherein the guide is capable of forming a nucleoprotein complex with a Cas protein.
**5.** The guide of item 4, wherein, upon formation of the nucleoprotein complex, the nucleoprotein complex is capable of binding a target nucleic acid sequence complementary to the spacer sequence.
**6.** The guide of item 4, wherein the guide comprises ribonucleotide bases, deoxyribonucleotide bases, or ribonucleotide and deoxyribonucleotide bases.
**7.** The guide of item 4, wherein the guide comprises one or more nucleic acid analogs selected from the group consisting of inosine, deoxyinosine, deoxyuradine, xanthosine, C3 spacer, 5-methyl dC, 5-hydroxybutynl-2'-deoxyuridine, 5-nitroindole, 5-methyl iso-deoxycytosine, iso deoxyguanosine, deoxyuradine, and iso deoxycytidine.
**8.** The guide of item 6, wherein the guide comprises a CABRNT selected from an apurinic site or an apyrimidinic site.
**9.** A nucleic acid/protein composition, comprising:
   the guide of item 4; and
   a Cas protein.
**10.** The nucleic acid/protein composition of item 9, wherein the guide is in a complex with the Cas protein.
**11.** The nucleic acid/protein composition of item 9, wherein the Cas protein comprises Cas9.
**12.** The nucleic acid/protein composition of item 11, wherein the CRISPR polynucleotide is hybridized to a tracr polynucleotide.
**13.** The nucleic acid/protein composition of item 12, wherein the CRISPR polynucleotide and the tracr polynucleotide are located on separate molecules.
**14.** The nucleic acid/protein composition of item 12, wherein the CRISPR polynucleotide and the tracr polynucleotide are covalently linked to form a single molecule.
**15.** The nucleic acid/protein composition of item 9, wherein the Cas protein comprises Cas 12.
**16.** The nucleic acid/protein composition of item 15, wherein the CRISPR polynucleotide comprises a stem-loop duplex.
**17.** A cell, comprising the guide of item 4.
**18.** The cell of item 17, further comprising a Cas protein.
**19.** The cell of item 18, wherein the guide is in a complex with the Cas protein.
**20.** The cell of item 18, further comprising a donor polynucleotide.
**21.** A method of cleaving a target nucleic acid sequence, the method comprising:
   contacting a target nucleic acid with a nucleoprotein complex comprising a catalytically active Cas protein and the guide of item 4;
   wherein the spacer is capable of binding to the target nucleic acid, the guide is capable of hybridizing to the target nucleic acid sequence, and the nucleoprotein complex is capable of cleaving the target nucleic acid sequence.
**22.** The method of item 21, further comprising providing a donor polynucleotide.
**23.** The method of item 21, wherein the target nucleic acid is cleaved to provide a cleavage site, and the method further comprises modifying the target nucleic acid.
**24.** The method of item 23, wherein the modifying comprises inserting at least a portion of a donor polynucleotide at the cleavage site.
**25.** The method of item 23, wherein the modifying comprises deleting one or more nucleotides at the cleavage site.
**26.** The method of item 24, wherein the target nucleic acid sequence is in a eukaryotic cell, and the donor polynucleotide comprises a CAR expression vector.
**27.** The cell of item 17, wherein the cell comprises a lymphocyte, a chimeric antigen receptor (CAR) T cell, a T cell receptor (TCR) cell, a TCR-engineered CAR-T cell, a tumor infiltrating lymphocyte (TIL), a CAR TIL, a dendritic cell (DC), a CAR-DC, a macrophage, a CAR-macrophage (CAR-M), a natural killer (NK) cell, or a CAR-NK cell.
**28.** The cell of item 27, wherein the cell is a CAR-T cell.
**29.** The method of item 26, wherein the resulting cell comprises a lymphocyte, a chimeric antigen receptor (CAR) T cell, a T cell receptor (TCR) cell, a TCR-engineered CAR-T cell, a tumor infiltrating lymphocyte (TIL), a CAR TIL, a dendritic cell (DC), a CAR-DC, a macrophage, a CAR-macrophage (CAR-M), a natural killer (NK) cell, or a CAR-NK cell.
**30.** The method of item 26, wherein the target nucleic acid sequence is within a target gene encoding a protein selected from the group consisting of a TRAC; a TRBV; a beta-2 microglobulin (B2M); a PD1; a PD-L1; a CTLA-4; a LAG-3; a TIGIT; a TIM3; a HLA-E; a HLA-A; a HLA-B; a HLA-C; a HLA-DRA; a ADAM17; a BTLA; a CD160; a SIGLEC10; a 2B4; a LAIR1; a CD52; a CD96; a VSIR; a VISTA; a KIR2DL1; a KIR2DL2; a KIR2DL3; a CEACAM1; a CBLB; a CISH; a IL-1R8; a AHR; a Adenosine 2A receptor; a GMCSF; a VISTA; a CII2A; and a NKG2A.
**31.** The cell of item 27, wherein the target nucleic acid sequence is within a target gene encoding a protein selected from the group consisting of a TRAC; a TRBV; a beta-2 microglobulin (B2M); a PD1; a PD-L1; a CTLA-4; a LAG-3; a TIGIT; a TIM3; a HLA-E; a HLA-A; a HLA-B; a HLA-C; a HLA-DRA; a ADAM17; a BTLA; a CD160; a SIGLEC10; a 2B4; a LAIR1; a CD52; a CD96; a VSIR; a VISTA; a KIR2DL1; a KIR2DL2; a KIR2DL3; a CEACAM1; a CBLB; a CISH; a IL-1R8; a AHR; a Adenosine 2A receptor; a GMCSF; a VISTA; a CII2A; and a NKG2A.
**32.** The CRISPR polynucleotide of item 1, wherein the CRISPR polynucleotide comprises a CAVA.
**33.** The CRISPR polynucleotide of item 32, wherein the CAVA is selected from the group consisting of inosine, deoxyinosine, and deoxyuradine.
**34.** The guide of item 4, wherein the guide comprises a CAVA.
**35.** A CRISPR polynucleotide comprising a spacer sequence comprising a CABRNT.
**36.** A CABRNT-containing guide, comprising:
   a CRISPR polynucleotide comprising a spacer sequence comprising at least one CABRNT;
   wherein the spacer sequence is capable of binding a target nucleic acid sequence, and the guide is capable of forming a nucleoprotein complex with a Cas protein.
**37.** A nucleic acid/protein composition, comprising:
   the CABRNT-containing guide of item 36; and
   a Cas protein.
**38.** A cell, comprising the CABRNT-containing guide of item 36.
**39.** A method of cleaving a target nucleic acid sequence, the method comprising:
   contacting a target nucleic acid with a nucleoprotein complex comprising a catalytically active Cas protein and the CABRNT-containing guide of item 36;
   wherein the spacer is capable of binding to the target nucleic acid, the guide is capable of hybridizing to the target nucleic acid sequence, and the nucleoprotein complex is capable of cleaving the target nucleic acid sequence.
**40.** A CRISPR polynucleotide comprising a spacer sequence comprising a CAVA nucleotide.
**41.** A CAVA-containing guide, comprising:
   a CRISPR polynucleotide comprising a spacer sequence comprising at least one CAVA;
   wherein the spacer sequence is capable of binding a target nucleic acid sequence; and wherein the guide is capable of forming a nucleoprotein complex with a Cas protein.
**42.** A nucleic acid/protein composition, comprising:
   the CAVA-containing guide of item 41; and
   a Cas protein.
**43.** A cell, comprising the CAVA-containing guide of item 41.
**44.** A method of cleaving a target nucleic acid sequence, the method comprising:
   contacting a target nucleic acid with a nucleoprotein complex comprising a catalytically active Cas protein and the CAVA-containing guide of item 41;
   wherein the spacer is capable of binding to the target nucleic acid, the guide is capable of hybridizing to the target nucleic acid sequence, and the nucleoprotein complex is capable of cleaving the target nucleic acid sequence.
**45.** A cell produced by the method of item 26.
**46.** A CAR-T cell produced by the method of item 26.
**47.** A method of producing a CAR-T cell, comprising performing the method of item 26 using a T-lymphocyte as the cell.
**48.** A method of adoptive cell therapy, comprising administering to a subject in need thereof a cell produced by the method of item 26.
**49.** A method for producing a CAR-expressing cell, said method comprising: contacting a first target nucleic acid sequence in a cell with a nucleoprotein complex comprising a catalytically active Cas protein and a first guide, wherein the first guide comprises a guide of any one of items 4, 36 and 41, wherein the targeting region of the first guide is capable of hybridizing to the first target nucleic acid sequence, and the nucleoprotein complex is capable of cleaving the first target nucleic acid sequence; contacting a second target nucleic acid sequence in the cell with a nucleoprotein complex comprising a catalytically active Cas protein and a second guide, wherein the second guide comprises a guide of any one of items 4, 36 and 41 that is capable of binding to a different target nucleic acid sequence than the first guide, wherein the targeting region of the second guide is capable of hybridizing to the second target nucleic acid sequence, and the nucleoprotein complex is capable of cleaving the second target nucleic acid sequence; and providing a donor polynucleotide comprising a CAR expression vector to said cell, wherein at least a portion of the donor polynucleotide containing said CAR expression vector is capable of being inserted at the cleavage site in said first target nucleic acid sequence, and wherein the CAR comprises an extracellular ligand-binding domain.

## Claims

1. A clustered regularly interspaced short palindromic repeats (CRISPR) polynucleotide comprising a spacer sequence comprising SEQ ID NO: 5 or SEQ ID NO: 13 comprising ribonucleotide and deoxyribonucleotide bases and capable of binding a target nucleic acid sequence complementary to the spacer sequence; and wherein the polynucleotide is capable of forming a nucleoprotein complex with a Cas protein.

2. A nucleic acid/protein composition, comprising:
the CRISPR polynucleotide of claim 1; and
a Cas9 protein.

3. The nucleic acid/protein composition of claim 2, wherein the CRISPR polynucleotide is hybridized to a tracr polynucleotide.

4. The nucleic acid/protein composition of claim 3, wherein the CRISPR polynucleotide and the tracr polynucleotide are located on separate molecules.

5. The nucleic acid/protein composition of claim 3, wherein the CRISPR polynucleotide and the tracr polynucleotide are covalently linked to form a single molecule.

6. A cell, comprising the nucleic acid/protein composition of claim 2.

7. A method of cleaving a target nucleic acid sequence, the method comprising:
contacting a target nucleic acid with a nucleic acid/protein composition of claim 2;
wherein the spacer is capable of binding to the target nucleic acid, the CRISPR polynucleotide is capable of hybridizing to the target nucleic acid sequence, and the nucleoprotein complex is capable of cleaving the target nucleic acid sequence.

8. The method of claim 7, further comprising providing a donor polynucleotide.

9. The method of claim 7, wherein the target nucleic acid is cleaved to provide a cleavage site, and the method further comprises modifying the target nucleic acid.

10. The method of claim 9, wherein the modifying comprises inserting at least a portion of a donor polynucleotide at the cleavage site.

11. The method of claim 9, wherein the modifying comprises deleting one or more nucleotides at the cleavage site.

12. The method of claim 10, wherein the target nucleic acid sequence is in a eukaryotic cell, and the donor polynucleotide comprises a CAR expression vector.

13. The cell of claim 6, wherein the cell comprises a lymphocyte, a chimeric antigen receptor (CAR) T cell, a T cell receptor (TCR) cell, a TCR-engineered CAR-T cell, a tumor infiltrating lymphocyte (TIL), a CAR TIL, a dendritic cell (DC), a CAR-DC, a macrophage, a CAR-macrophage (CAR-M), a natural killer (NK) cell, or a CAR-NK cell.

14. The cell of claim 13, wherein the cell is a CAR-T cell.

15. The method of claim 12, wherein the resulting cell comprises a chimeric antigen receptor (CAR) T cell, a TCR-engineered CAR-T cell, a CAR tumor infiltrating lymphocyte (CAR TIL), a CAR-dendritic cell (CAR-DC), a CAR-macrophage (CAR-M), or a CAR-NK cell.

16. The method of claim 12, wherein the target nucleic acid sequence is within a target gene encoding a protein selected from the group consisting of a TRAC, and a PD1.

17. A cell produced by the method of claim 12.

18. A CAR-T cell produced by the method of claim 12.

19. A method of adoptive cell therapy, comprising administering to a subject in need thereof a cell produced by the method of claim 12.
